# EUROPEAN PATENT APPLICATION

(11) **EP 4 588 915 A1**
(43) Date of publication of application: **23.07.2025**
(21) Application number: 23864807.5
(22) Date of filing: 15.09.2023
(51) Int. Cl.: C07D 233/84, A61K 31/417, A61P 29/00, A61P 37/00, A61P 35/00, A61P 25/18, A61P 9/04, A61P 9/12

(54) **POLYMORPHIC FORM OF NEPICASTAT ACID ADDITION SALT, PREPARATION METHOD THEREFOR AND USE THEREOF**

(30) Priority: 16.09.2022 CN 202211129279
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: GUO, Yushen, Taizhou, Jiangsu 225316 (CN); HE, Xiaoqing, Taizhou, Jiangsu 225316 (CN); XU, Miaohuan, Taizhou, Jiangsu 225316 (CN); ZHOU, Lanping, Taizhou, Jiangsu 225316 (CN)
(74) Representative: Regimbeau
(86) International application number: PCT/CN2023/119143
(87) International publication number: WO 2024/056079

(57) **Abstract**

The present invention relates to a polymorphic form of a nepicastat acid addition salt, a preparation method therefor and a use thereof. The acid addition salt is selected from hydrochloride, sulfate, phosphate, mesylate, benzenesulfonate, p-toluenesulfonate, hydrobromate, maleate, tartrate, citrate, and fumarate, preferably hydrochloride. The nepicastat acid addition salt has high polymorphic form stability, high solubility, high plasma concentration, high bioavailability, and excellent pharmacological action, and therefore is suitable for pharmaceutical preparations.

## Description

### FIELD OF THE INVENTION

The present invention relates to polymorphs of an acid addition salt of nepicastat and preparation methods and uses thereof. The acid addition salt is selected from the group consisting of hydrochloride salt, sulfate salt, phosphate salt, methanesulfonate salt, benzenesulfonate salt, p-toluenesulfonate salt, hydrobromide salt, maleate salt, tartrate salt, citrate salt, and fumarate salt, preferably hydrochloride salt.

### BACKGROUND OF THE INVENTION

Anxiety disorders are the most common mental disease with significant economic burden. In addition to generalized anxiety disorder, they include post-traumatic stress disorder (PTSD), panic disorder, obsessive-compulsive disorder, social disorder, and other phobias.

Post-traumatic stress disorder may be severe and chronic, and some studies show that the incidence rate is 1.3% to 7.8% throughout the lifetime of the general population. Post-traumatic stress disorder usually accompanies psychologically painful traumatic events. These events may include, for example, aggressions, terrorist incidents, physical attacks, sexual attacks, motor vehicle accidents, and natural disasters. The response to such events may include strong fear, helplessness, or terror. Most people recover from traumatic events over time and return to normal life. In contrast, in patients with post-traumatic stress disorder, symptoms may persist and possibly worsen over time, hindering recovery to normal life.

Nepicastat, also known as SYN117 and RS-25560-197, is a highly selective inhibitor of dopamine beta-hydroxylase (DBH), has the following structural formula and can effectively treat PTSD.

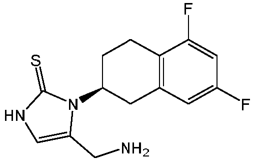

In addition, nepicastat has been studied as a possible treatment for congestive heart failure. Nepicastat and its analogues (such as etamicastat) have common potential uses in treating hypertension, cancer, and the like. Nepicastat has also been reported to effectively reduce the mortality rate of sepsis and alleviate the functional damage to multiple organs (e.g., heart, lung, liver, intestine, and the like) induced by sepsis. Additionally, nepicastat has been reported to be able to treat autoimmune diseases.

The polymorphism of drugs has become an indispensable and important part in drug research and development and drug quality control. The research on drug polymorphism facilitates the selection of the biological activity of the drug compound, and helps to increase drug stability, solubility and other properties, which in turn is beneficial to the development of drug preparations, the storage of drugs, the improvement of the quality of drug production, and the like. It can also improve the bioavailability of compounds and enhance the clinical efficacy.

However, there is no relevant report of polymorphs of an acid addition salt of nepicastat in the prior art.

### SUMMARY OF THE INVENTION

The technical problem to be solved by the present invention is to provide polymorphs of an acid addition salt of nepicastat and preparation methods and uses thereof. The inventors have discovered preparation methods being able to prepare polymorphs of an acid addition salt of nepicastat after extensive experimental research, and have conducted detections by X-ray powder diffraction, TGA, DSC and the like and stability studies on the obtained polymorphs. As a result, the crystal forms of the acid addition salt of nepicastat with good stability have been found.

The present invention solves the above technical problem through the following technical solutions:
The present invention provides a crystal form of an acid addition salt of nepicastat, wherein the acid addition salt is selected from the group consisting of hydrochloride salt, sulfate salt, phosphate salt, methanesulfonate salt, benzenesulfonate salt, p-toluenesulfonate salt, hydrobromide salt, maleate salt, tartrate salt, citrate salt, and fumarate salt, preferably hydrochloride salt. The crystal form of the acid addition salt is an anhydride, hydrate or solvate.

In some embodiments, the crystal form of the acid addition salt of nepicastat is crystal form I of hydrochloride salt of nepicastat, crystal form II of hydrochloride salt of nepicastat, crystal form III of hydrochloride salt of nepicastat or crystal form IV of hydrochloride salt of nepicastat.

The crystal form I of hydrochloride salt of nepicastat is a monohydrate, and the X-ray powder diffraction pattern thereof comprises characteristic peaks at diffraction angles (2θ) of 15.48±0.2°, 20.66±0.2°, 22.64±0.2°, 25.60±0.2°, 27.06±0.2°, 29.70±0.2°, 31.84±0.2° and 41.94±0.2°; and preferably, further comprises at least one characteristic peak, for example 1, 2, 3, 4, 5, 6 or 7 characteristic peaks, at diffraction angles (2θ) of 17.04±0.2°, 23.82±0.2°, 25.08±0.2°, 34.34±0.2°, 36.78±0.2°, 43.88±0.2° and 44.12±0.2°; and more preferably, further comprises at least one characteristic peak, for example 1, 2, 3, 4 or 5 characteristic peaks, at diffraction angles (2θ) of 5.12±0.2°, 12.98±0.2°, 27.68±0.2°, 28.20±0.2° and 39.94±0.2°.

Particularly, the crystal form I of hydrochloride salt of nepicastat is characterized by the XRPD characteristic peaks as shown in Table 1:

**Table 1**

| Peak No. | 2θ | d value | Peak height | Peak height % | Area | Area % |
|---|---|---|---|---|---|---|
| 1 | 5.116 | 17.2595 | 61 | 13.0 | 362 | 3.7 |
| 2 | 12.981 | 6.8144 | 73 | 15.6 | 807 | 8.2 |
| 3 | 13.743 | 6.4384 | 25 | 5.3 | 347 | 3.5 |
| 4 | 15.478 | 5.7202 | 282 | 60.3 | 5060 | 51.3 |
| 5 | 17.038 | 5.1998 | 99 | 21.2 | 1927 | 20.0 |
| 6 | 18.821 | 4.7111 | 38 | 8.1 | 363 | 3.7 |
| 7 | 20.661 | 4.2955 | 237 | 50.6 | 3586 | 36.3 |
| 8 | 22.641 | 3.9241 | 468 | 100.0 | 8581 | 86.9 |
| 9 | 23.819 | 3.7326 | 109 | 23.3 | 1541 | 15.6 |
| 10 | 25.078 | 3.5480 | 122 | 26.1 | 3435 | 34.8 |
| 11 | 25.602 | 3.4766 | 433 | 92.5 | 9872 | 100.0 |
| 12 | 27.060 | 3.2924 | 205 | 43.8 | 3037 | 30.8 |
| 13 | 27.682 | 3.2199 | 87 | 18.6 | 2186 | 22.1 |
| 14 | 28.203 | 3.1616 | 66 | 14.1 | 1425 | 14.4 |
| 15 | 28.781 | 3.0994 | 24 | 5.1 | 260 | 2.6 |
| 16 | 29.700 | 3.0055 | 175 | 37.4 | 3058 | 31.0 |
| 17 | 31.839 | 2.8083 | 146 | 31.2 | 2784 | 28.2 |
| 18 | 32.737 | 2.7333 | 34 | 7.3 | 511 | 5.2 |
| 19 | 33.581 | 2.6665 | 18 | 3.8 | 269 | 2.7 |
| 20 | 34.340 | 2.6092 | 127 | 27.1 | 2640 | 26.7 |
| 21 | 36.780 | 2.4416 | 121 | 25.9 | 2234 | 22.6 |
| 22 | 38.760 | 2.3213 | 40 | 8.5 | 1282 | 13.0 |
| 23 | 39.319 | 2.2896 | 38 | 8.1 | 689 | 7.0 |
| 24 | 39.937 | 2.2555 | 68 | 14.5 | 2414 | 24.5 |
| 25 | 40.506 | 2.2252 | 34 | 7.3 | 382 | 3.9 |
| 26 | 41.939 | 2.1524 | 175 | 37.4 | 3484 | 35.3 |
| 27 | 43.880 | 2.0616 | 103 | 22.0 | 2951 | 29.9 |
| 28 | 44.119 | 2.0510 | 96 | 20.3 | 2932 | 29.7 |
| 29 | 49.217 | 1.8498 | 38 | 8.1 | 547 | 5.5 |

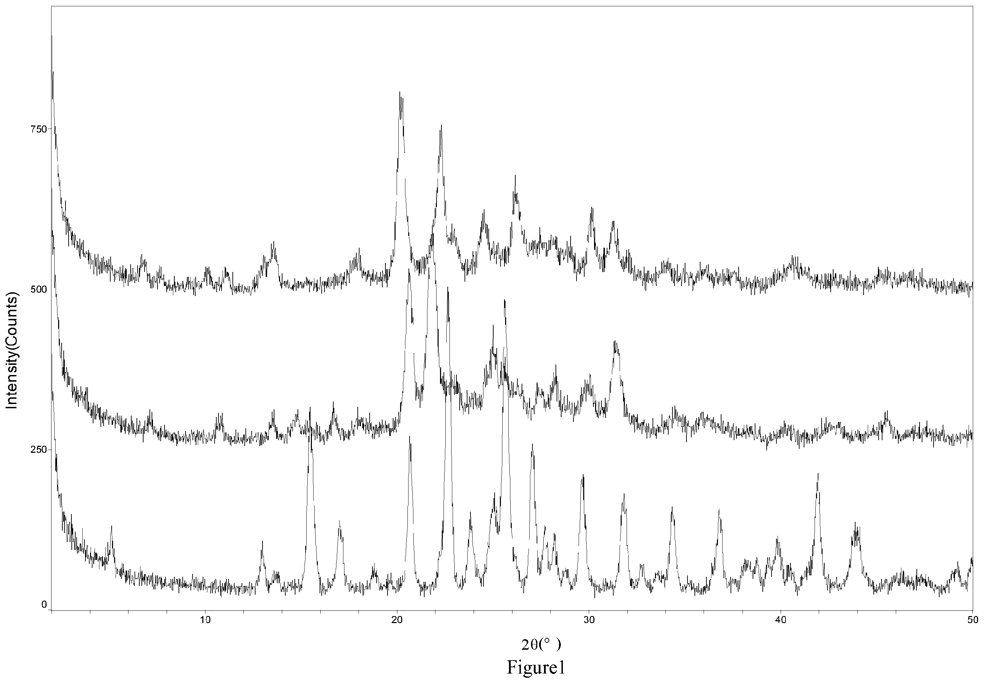

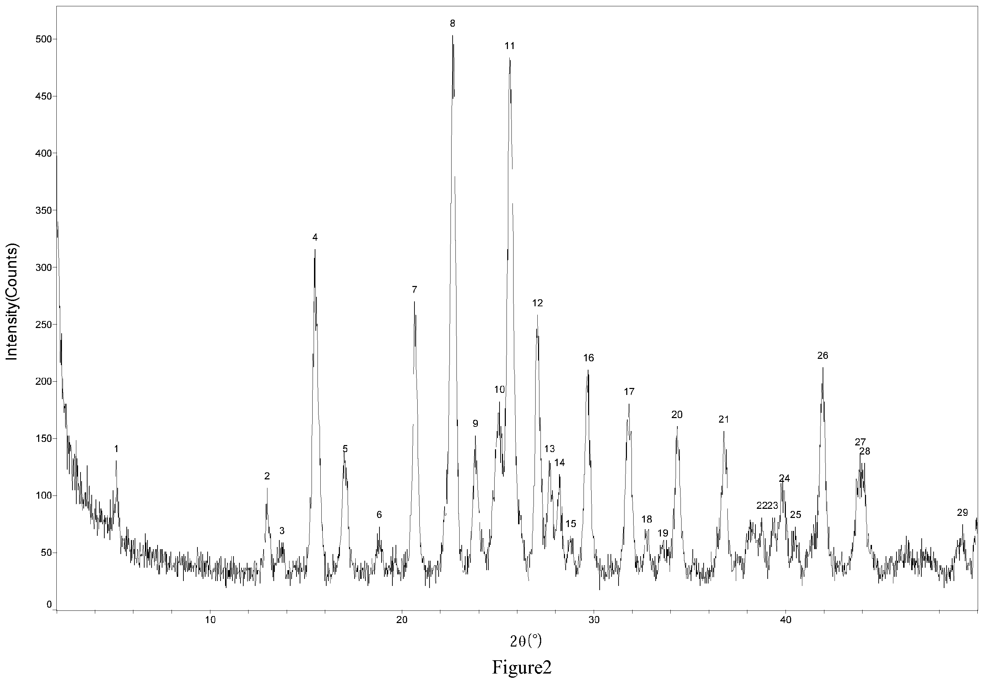

More particularly, the X-ray powder diffraction pattern of the crystal form I of hydrochloride salt of nepicastat is substantially as shown in Figures 1 and 2.

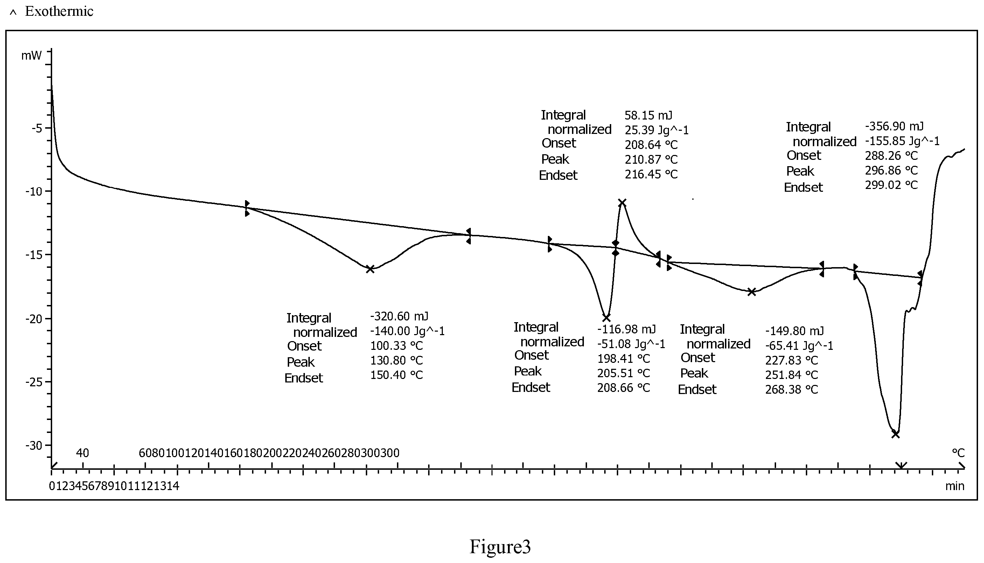

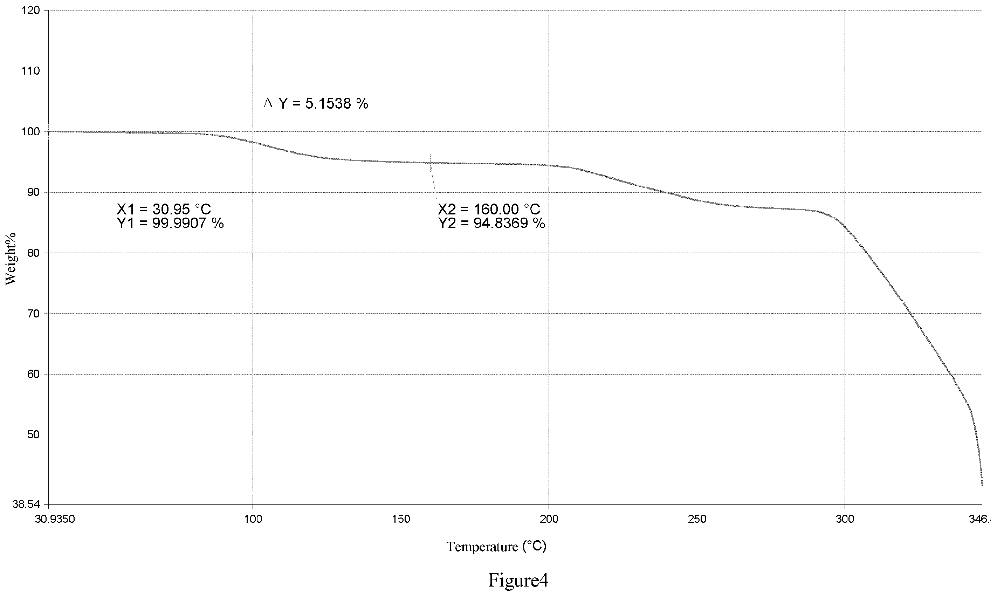

Advantageously, the DSC spectrum of the crystal form I of hydrochloride salt of nepicastat shows 4 endothermic peaks at 130.8°C, 205.5°C, 251.8°C and 296.9°C and one exothermic peak at 210.9°C, and more advantageously, is substantially as shown in Figure 3; the TGA spectrum of the crystal form I of hydrochloride salt of nepicastat shows a weight loss of 5.15% from room temperature to 120°C, and more advantageously, is substantially as shown in Figure 4.

The crystal form II of hydrochloride salt of nepicastat is an anhydride, and the X-ray powder diffraction pattern thereof comprises characteristic peaks at diffraction angles (2θ) of 20.62±0.2°, 21.90±0.2°, 25.04±0.2°, 28.28±0.2°, 30.05±0.2° and 31.46±0.2°; and preferably, further comprises at least one characteristic peak, for example 1, 2, 3, 4 or 5 characteristic peaks, at diffraction angles (2θ) of 10.88±0.2°, 13.60±0.2°, 14.84±0.2°, 17.96±0.2° and 27.32±0.2°; and more preferably, further comprises at least one characteristic peak, for example 1, 2, 3, 4, 5, 6, 7 or 8 characteristic peaks, at diffraction angles (2θ) of 7.12±0.2°, 16.74±0.2°, 18.57±0.2°, 23.01±0.2°, 24.64±0.2°, 34.54±0.2°, 40.17±0.2° and 45.56±0.2°.

Particularly, the crystal form II of hydrochloride salt of nepicastat is characterized by the XRPD characteristic peaks as shown in Table 2:

**Table 2**

| Peak No. | 2θ | d value | Peak height | Peak height % | Area | Area % |
|---|---|---|---|---|---|---|
| 1 | 7.120 | 12.4043 | 35 | 13.8 | 265 | 4.0 |
| 2 | 10.876 | 8.1278 | 40 | 15.8 | 521 | 7.9 |
| 3 | 15.598 | 6.5066 | 44 | 17.4 | 411 | 6.2 |
| 4 | 14.839 | 5.9650 | 40 | 15.8 | 738 | 11.2 |
| 5 | 16.736 | 5.2931 | 35 | 13.8 | 706 | 10.7 |
| 6 | 17.960 | 4.9349 | 41 | 16.2 | 588 | 8.9 |
| 7 | 18.566 | 4.7753 | 28 | 11.1 | 360 | 5.5 |
| 8 | 20.621 | 4.3037 | 253 | 100.0 | 6578 | 100.0 |
| 9 | 21.898 | 4.0555 | 250 | 98.8 | 6416 | 97.5 |
| 10 | 23.005 | 3.8629 | 33 | 13.0 | 315 | 4.8 |
| 11 | 23.136 | 3.8413 | 25 | 9.9 | 299 | 4.5 |
| 12 | 24.029 | 3.7004 | 19 | 7.5 | 169 | 2.6 |
| 13 | 24.644 | 3.6095 | 43 | 17.0 | 603 | 9.2 |
| 14 | 25.035 | 3.5540 | 69 | 27.3 | 2602 | 39.6 |
| 15 | 25.672 | 3.4673 | 25 | 9.9 | 516 | 7.8 |
| 16 | 26.270 | 3.3897 | 32 | 12.6 | 377 | 5.7 |
| 17 | 27.315 | 3.2622 | 39 | 15.4 | 499 | 7.6 |
| 18 | 28.283 | 3.1528 | 68 | 26.9 | 1021 | 155 |
| 19 | 29.782 | 2.9975 | 50 | 19.8 | 1245 | 18.9 |
| 20 | 29.918 | 2.9641 | 48 | 19.0 | 1107 | 16.8 |
| 21 | 30.047 | 2.9716 | 57 | 22.5 | 1085 | 16.5 |
| 22 | 31.460 | 2.8413 | 114 | 45.1 | 3128 | 47.6 |
| 23 | 34.536 | 2.5949 | 29 | 11.5 | 728 | 11.1 |
| 24 | 35.886 | 2.5003 | 23 | 9.1 | 326 | 5.0 |
| 25 | 36.180 | 2.4807 | 24 | 9.5 | 752 | 11.4 |
| 26 | 40.165 | 2.2433 | 28 | 11.1 | 602 | 9.2 |
| 27 | 42.642 | 2.1185 | 21 | 8.3 | 1051 | 16.0 |
| 28 | 43.092 | 2.0975 | 22 | 8.7 | 841 | 9.7 |
| 29 | 45.561 | 1.9883 | 35 | 13.8 | 442 | 6.7 |

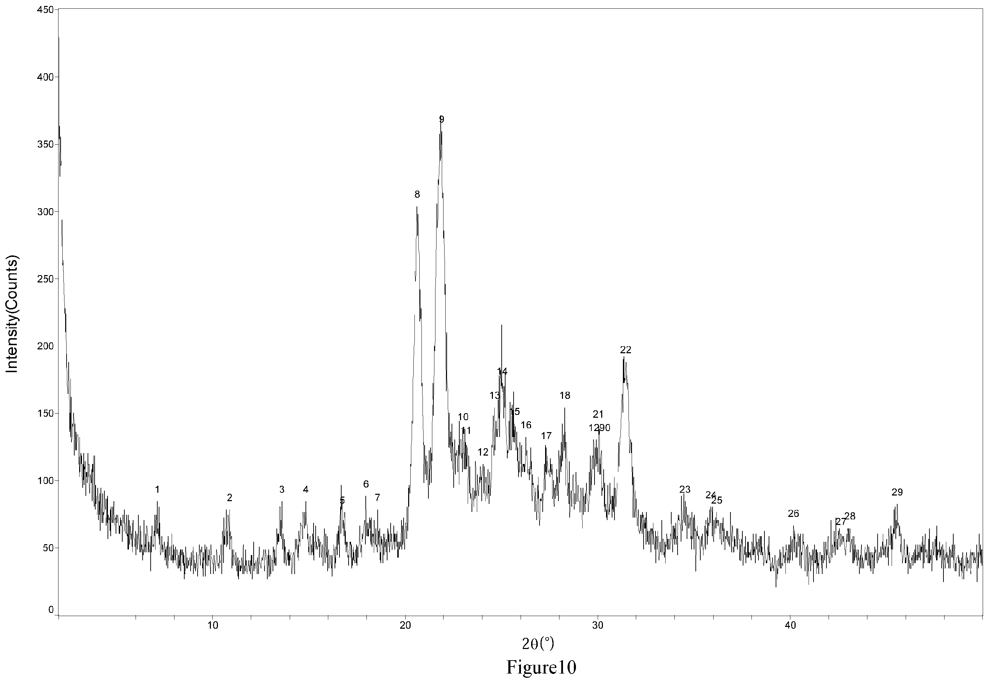

More particularly, the X-ray powder diffraction pattern of the crystal form II of hydrochloride salt of nepicastat is substantially as shown in Figures 1 and 10.

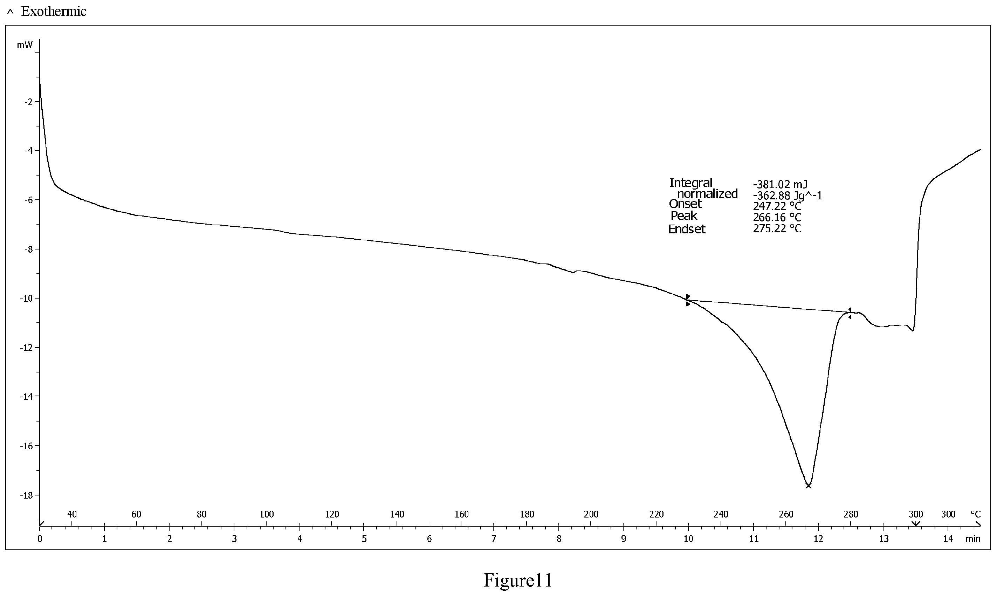

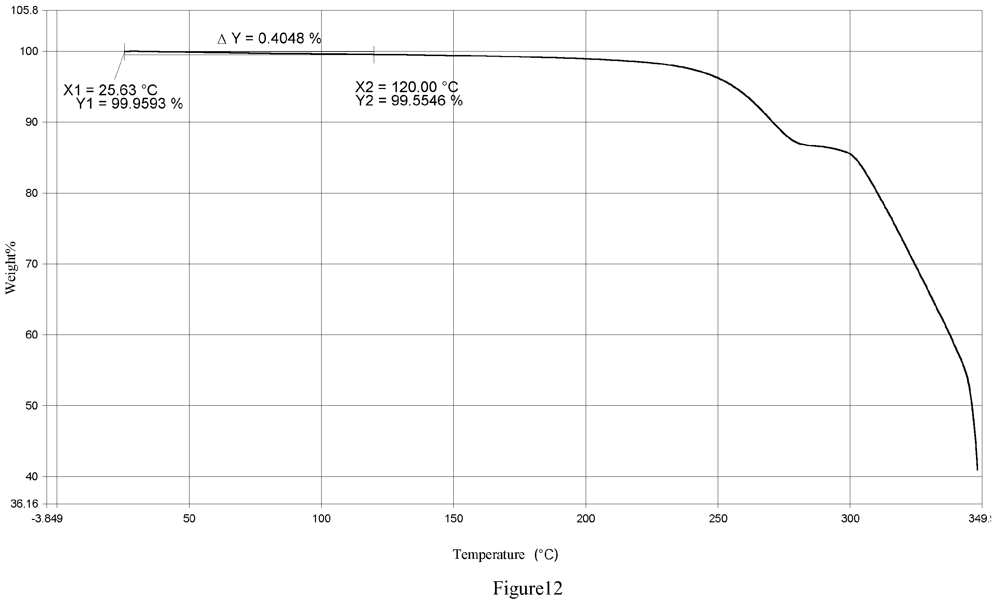

Advantageously, the DSC spectrum of the crystal form II of hydrochloride salt of nepicastat shows one endothermic peak at 266.2°C, and more advantageously, is substantially as shown in Figure 11; the TGA spectrum of the crystal form II of hydrochloride salt of nepicastat shows a weight loss of 0.40% from room temperature to 120°C, and more advantageously, is substantially as shown in Figure 12.

The crystal form III of hydrochloride salt of nepicastat is characterized by the X-ray powder diffraction pattern comprising characteristic peaks at diffraction angles (2θ) of 13.58±0.2°, 20.22±0.2°, 22.32±0.2°, 24.54±0.2°, 26.16±0.2°, 30.14±0.2° and 31.26±0.2°; and preferably, further comprising at least one characteristic peak, for example 1, 2, 3, 4, 5 or 6 characteristic peaks, at diffraction angles (2θ) of 6.86±0.2°, 7.62±0.2°, 10.26±0.2°, 11.14±0.2°, 13.03±0.2° and 40.76±0.2°.

Particularly, the crystal form III of hydrochloride salt of nepicastat is characterized by the XRPD characteristic peaks as shown in Table 3:

**Table 3**

| Peak No. | 2θ | d value | Peak height | Peak height % | Area | Area % |
|---|---|---|---|---|---|---|
| 1 | 6.861 | 12.8733 | 31 | 11.3 | 504 | 7.4 |
| 2 | 7.622 | 11.5897 | 21 | 7.7 | 32 | 0.5 |
| 3 | 10.258 | 8.6159 | 22 | 8.0 | 326 | 4.8 |
| 4 | 11.140 | 7.9360 | 22 | 8.0 | 281 | 4.1 |
| 5 | 13.025 | 6.7912 | 48 | 17.5 | 1355 | 19.9 |
| 6 | 13.578 | 6.5161 | 69 | 25.2 | 1978 | 29.1 |
| 7 | 17.743 | 4.9948 | 34 | 12.4 | 979 | 14.4 |
| 8 | 18.019 | 4.9189 | 49 | 17.9 | 921 | 13.6 |
| 9 | 20.220 | 4.3881 | 274 | 100.0 | 6796 | 100.0 |
| 10 | 22.321 | 3.9796 | 218 | 79.6 | 5979 | 88.0 |
| 11 | 22.977 | 3.8874 | 23 | 8.4 | 40 | 0.6 |
| 12 | 24.542 | 3.6243 | 83 | 30.3 | 1763 | 25.9 |
| 13 | 26.160 | 3.4037 | 126 | 46.0 | 2444 | 36.0 |
| 14 | 27.494 | 3.2414 | 19 | 6.9 | 659 | 9.7 |
| 15 | 28.043 | 3.1792 | 29 | 10.6 | 638 | 9.4 |
| 16 | 28.274 | 3.1538 | 30 | 10.9 | 949 | 14.0 |
| 17 | 28.947 | 3.0819 | 28 | 10.2 | 555 | 8.2 |
| 18 | 29.135 | 3.0625 | 21 | 7.7 | 556 | 8.2 |
| 19 | 30.143 | 2.9624 | 91 | 33.2 | 1454 | 21.4 |
| 20 | 31.261 | 2.8589 | 66 | 24.1 | 1274 | 18.7 |
| 21 | 33.895 | 2.6425 | 28 | 10.2 | 574 | 8.4 |
| 22 | 34.179 | 2.6212 | 25 | 9.1 | 676 | 9.9 |
| 23 | 34.996 | 2.5620 | 26 | 9.5 | 146 | 2.1 |
| 24 | 35.800 | 2.5062 | 31 | 11.3 | 478 | 7.0 |
| 25 | 36.216 | 2.4783 | 27 | 9.9 | 478 | 7.0 |
| 26 | 37.582 | 2.3913 | 16 | 5.8 | 236 | 3.5 |
| 27 | 40.582 | 2.2212 | 34 | 12.4 | 789 | 11.6 |
| 28 | 40.759 | 2.2119 | 35 | 12.8 | 1014 | 149 |
| 29 | 45.220 | 2.0036 | 20 | 7.3 | 366 | 5.4 |
| 30 | 45.624 | 1.9868 | 20 | 7.3 | 490 | 7.2 |
| 31 | 46.759 | 1.9411 | 20 | 7.3 | 394 | 5.8 |

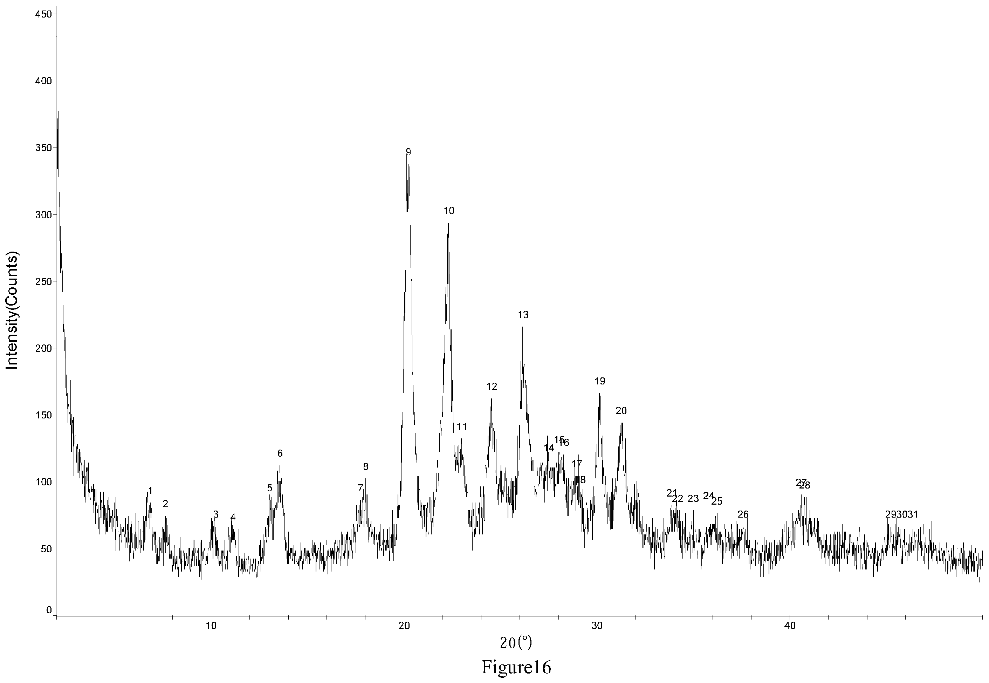

More particularly, the X-ray powder diffraction pattern of the crystal form III of hydrochloride salt of nepicastat is substantially as shown in Figures 1 and 16.

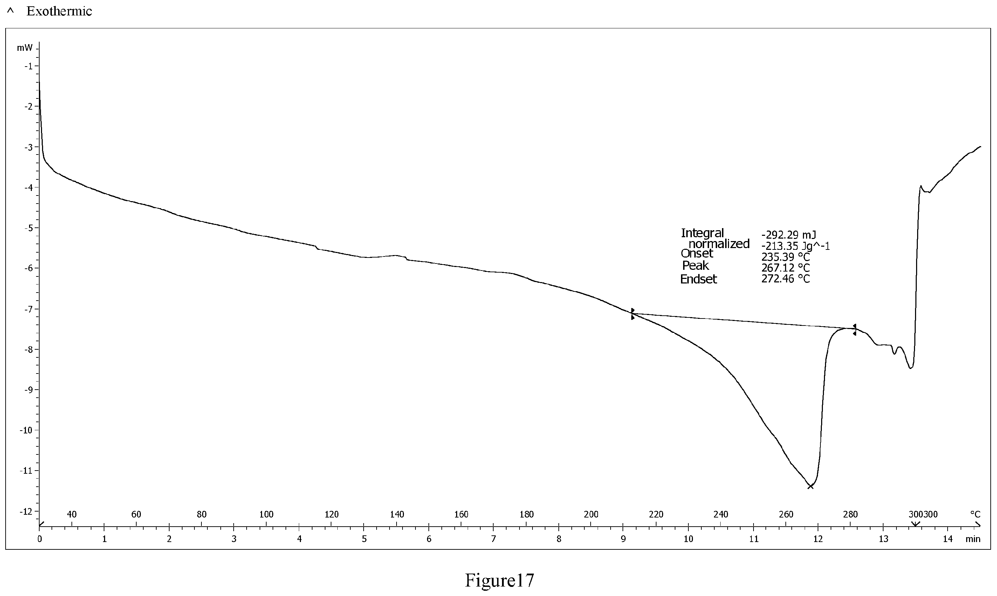

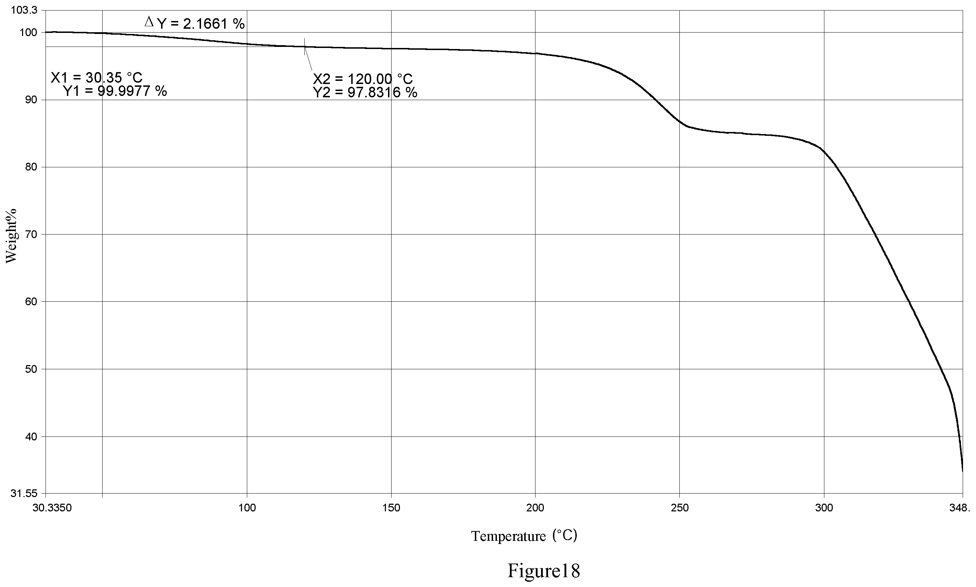

Advantageously, the DSC spectrum of the crystal form III of hydrochloride salt of nepicastat shows one endothermic peak at 267.1°C, and more advantageously, is substantially as shown in Figure 17; the TGA spectrum of the crystal form III ofhydrochloride salt of nepicastat shows a weight loss of 2.17% from room temperature to 120°C, and more advantageously, is substantially as shown in Figure 18.

The crystal form IV of hydrochloride salt of nepicastat is characterized by the X-ray powder diffraction pattern comprising characteristic peaks at diffraction angles (2θ) of 13.60±0.2°, 20.70±0.2°, 21.94±0.2°, 24.99±0.2° and 25.64±0.2°; and preferably, further comprising at least one characteristic peak, for example 1, 2, 3, 4, 5, 6 or 7 characteristic peaks, at diffraction angles (2θ) of 15.56±0.2°, 16.78±0.2°, 22.68±0.2°, 26.18±0.2°, 27.32±0.2°, 28.23±0.2° and 31.44±0.2°; and more preferably, further comprising at least one characteristic peak, for example 1, 2, 3, 4 or 5 characteristic peaks, at diffraction angles (2θ) of 10.95±0.2°, 13.06±0.2°, 14.84±0.2°, 17.93±0.2° and 29.95±0.2°; and even more preferably, further comprising at least one characteristic peak, for example 1, 2, 3, 4, 5 or 6 characteristic peaks, at diffraction angles (2θ) of 5.23±0.2°, 7.12±0.2°, 23.80±0.2°, 34.52±0.2°, 33.16±0.2° and 38.48±0.2°.

Particularly, the crystal form IV of hydrochloride salt of nepicastat is characterized by the XRPD characteristic peaks as shown in Table 4:

**Table 4**

| Peak No. | 2θ | d value | Peak height |
|---|---|---|---|
| 1 | 5.23(3) | 16.88(9) | 34.1(18) |
| 2 | 7.12(2) | 12.40(4) | 43(2) |
| 3 | 10.95(2) | 8.073(17) | 125(5) |
| 4 | 13.056(9) | 6.775(4) | 134(5) |
| 5 | 13.599(8) | 6.506(4) | 523(15) |
| 6 | 14.840(12) | 5.965(5) | 175(6) |
| 7 | 15.563(10) | 5.689(3) | 343(11) |
| 8 | 16.776(13) | 5.281(4) | 316(10) |
| 9 | 17.93(2) | 4.943(6) | 120(4) |
| 10 | 20.704(7) | 4.2867(14) | 686(16) |
| 11 | 21.938(8) | 4.0483(14) | 656(15) |
| 12 | 22.683(7) | 3.9169(11) | 367(9) |
| 13 | 23.804(17) | 3.735(3) | 87(3) |
| 14 | 24.986(9) | 3.5609(12) | 614(14) |
| 15 | 25.642(7) | 3.4713(9) | 681(15) |
| 16 | 26.181(16) | 3.401(2) | 314(8) |
| 17 | 27.319(12) | 3.2619(14) | 341(9) |
| 18 | 28.227(9) | 3.1589(9) | 391(10) |
| 19 | 29.949(18) | 2.9811(18) | 144(5) |
| 20 | 31.442(13) | 2.8429(12) | 441(12) |
| 21 | 34.52(5) | 2.596(4) | 37.0(14) |
| 22 | 36.16(4) | 2.482(3) | 55(2) |
| 23 | 38.48(12) | 2.338(7) | 42.5(17) |

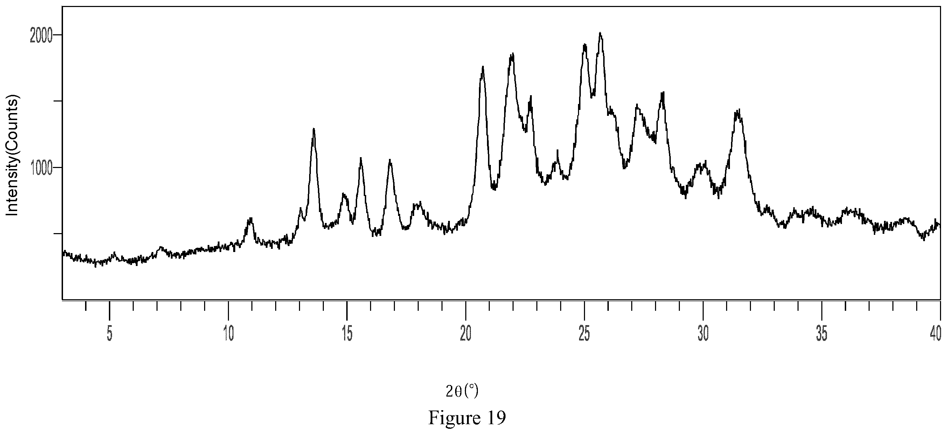

More particularly, the X-ray powder diffraction pattern of the crystal form IV of hydrochloride salt of nepicastat is substantially as shown in Figure 19.

In some embodiments, the crystal form of the acid addition salt of nepicastat is crystal form I of sulfate salt (1:0.5) of nepicastat, crystal form II of sulfate salt (1:0.5) of nepicastat or crystal form III of sulfate salt (1:1) of nepicastat. It can be understood that, the compound corresponding to the crystal form I of sulfate salt (1:0.5) of nepicastat and the crystal form II of sulfate salt (1:0.5) of nepicastat is sulfate salt of nepicastat, while the compound corresponding to the crystal form III of sulfate salt (1:1) of nepicastat is bisulfate salt of nepicastat.

The X-ray powder diffraction pattern of the crystal form I of sulfate salt (1:0.5) of nepicastat comprises characteristic peaks at diffraction angles (2θ) of 9.90±0.2°, 14.78±0.2°, 16.72±0.2°, 19.82±0.2°, 22.08±0.2°, 22.40±0.2° and 24.86±0.2°; and preferably, further comprises at least one characteristic peak, for example 1, 2, 3 or 4 characteristic peaks, at diffraction angles (2θ) of 25.56±0.2°, 26.19±0.2°, 28.42±0.2° and 30.68±0.2°; and more preferably, further comprises at least one characteristic peak, for example 1, 2 or 3 characteristic peaks, at diffraction angles (2θ) of 35.16±0.2°, 35.80±0.2° and 40.26±0.2°.

Particularly, the crystal form I of sulfate salt (1:0.5) of nepicastat is characterized by the XRPD characteristic peaks as shown in Table 5:

**Table 5**

| Peak No. | 2θ | d value | Peak height | Peak height % | Area | Area % |
|---|---|---|---|---|---|---|
| 1 | 9.901 | 8.9264 | 74 | 14.7 | 1638 | 13.4 |
| 2 | 14.780 | 5.9887 | 151 | 29.9 | 3126 | 25.5 |
| 3 | 16.720 | 5.2980 | 147 | 29.1 | 3229 | 26.4 |
| 4 | 19.819 | 4.4759 | 505 | 100.0 | 12251 | 100.0 |
| 5 | 22.080 | 4.0225 | 116 | 23.0 | 4452 | 36.3 |
| 6 | 22.399 | 3.9658 | 111 | 22.0 | 4238 | 34.6 |
| 7 | 24.860 | 3.5786 | 227 | 45.0 | 9865 | 80.5 |
| 8 | 25.560 | 3.4822 | 120 | 23.8 | 7915 | 64.6 |
| 9 | 26.189 | 3.4000 | 75 | 14.9 | 1845 | 15.1 |
| 10 | 28.419 | 3.1380 | 69 | 13.7 | 2708 | 22.1 |
| 11 | 30.680 | 2.9117 | 75 | 14.9 | 2823 | 23.0 |
| 12 | 35.160 | 2.5503 | 82 | 16.2 | 3181 | 26.0 |
| 13 | 35.799 | 2.5062 | 51 | 10.1 | 2744 | 22.4 |
| 14 | 40.260 | 2.2382 | 59 | 11.7 | 2329 | 19.0 |

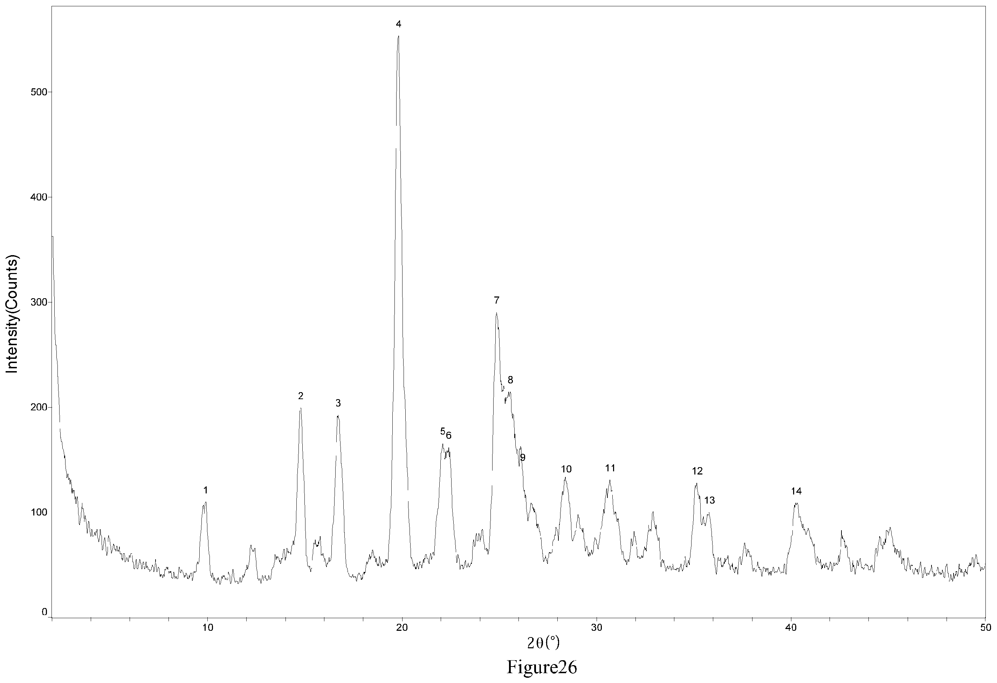

More particularly, the X-ray powder diffraction pattern of the crystal form I of sulfate salt (1:0.5) of nepicastat is substantially as shown in Figure 26.

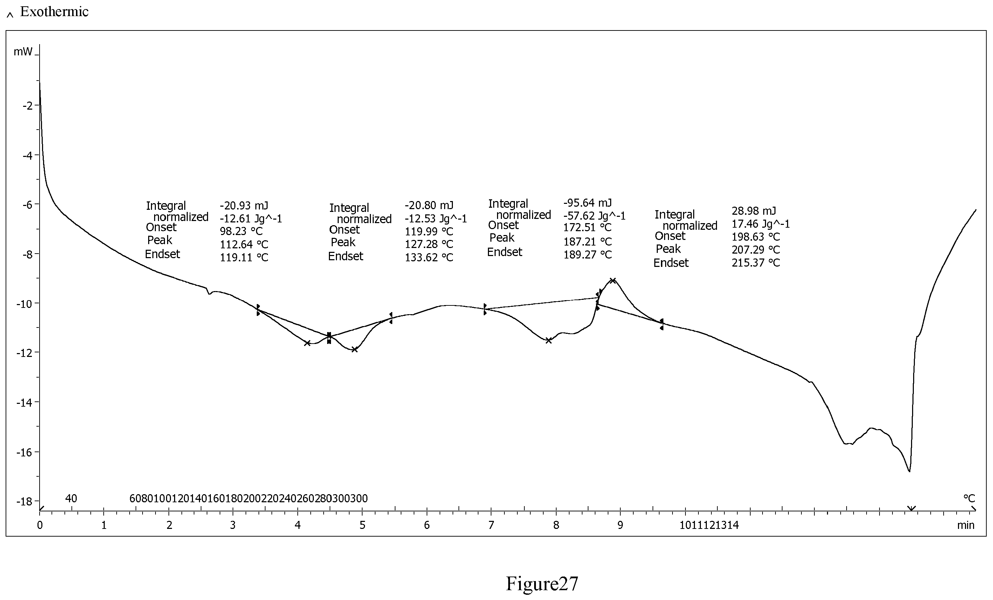

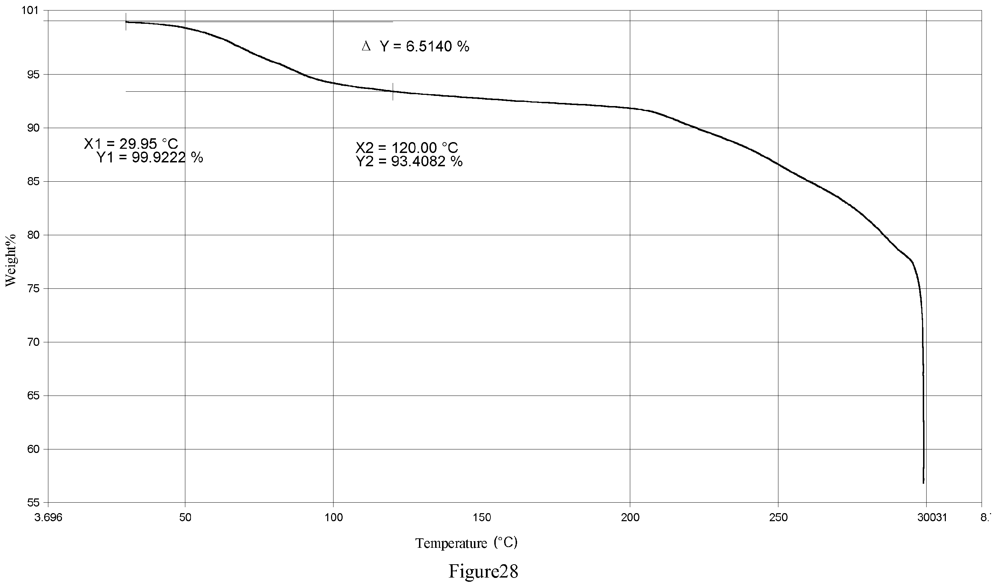

Advantageously, the DSC spectrum of the crystal form I of sulfate salt (1:0.5) of nepicastat shows 3 endothermic peaks at 112.6°C, 127.3°C and 187.2°C and one exothermic peak at 207.3°C, and more advantageously, is substantially as shown in Figure 27; the TGA spectrum of the crystal form I of sulfate salt (1:0.5) of nepicastat shows a weight loss of 6.51 % from room temperature to 120°C, and more advantageously, is substantially as shown in Figure 28.

The X-ray powder diffraction pattern of the crystal form II of sulfate salt (1:0.5) of nepicastat comprises characteristic peaks at diffraction angles (2θ) of 4.56±0.2°, 8.24±0.2°, 9.02±0.2°, 16.08±0.2°, 16.58±0.2°, 17.90±0.2°, 22.01±0.2° and 25.12±0.2°; and preferably, further comprises at least one characteristic peak, for example 1, 2 or 3 characteristic peaks, at diffraction angles (2θ) of 19.48±0.2°, 23.68±0.2° and 27.14±0.2°.

Particularly, the crystal form II of sulfate salt (1:0.5) of nepicastat is characterized by the XRPD characteristic peaks as shown in Table 6:

**Table 6**

| Peak No. | 2θ | d value | Peak height | Peak height % | Area | Area % |
|---|---|---|---|---|---|---|
| 1 | 4.559 | 19.3664 | 119 | 27.6 | 1901 | 22.4 |
| 2 | 8.240 | 10.7217 | 44 | 10.2 | 1061 | 12.5 |
| 3 | 9.021 | 9.7952 | 46 | 10.7 | 708 | 8.3 |
| 4 | 16.080 | 5.5074 | 107 | 24.8 | 1797 | 21.2 |
| 5 | 16.580 | 5.3424 | 129 | 29.9 | 4323 | 50.9 |
| 6 | 17.900 | 4.9511 | 338 | 78.4 | 7321 | 86.2 |
| 7 | 19.481 | 4.5527 | 72 | 16.7 | 1096 | 12.9 |
| 8 | 22.099 | 4.0191 | 431 | 100.0 | 8496 | 100.0 |
| 9 | 23.680 | 3.7542 | 77 | 17.9 | 1187 | 14.0 |
| 10 | 25.119 | 3.5422 | 153 | 35.5 | 5868 | 69.1 |
| 11 | 27.139 | 3.2830 | 56 | 13.0 | 3307 | 38.9 |

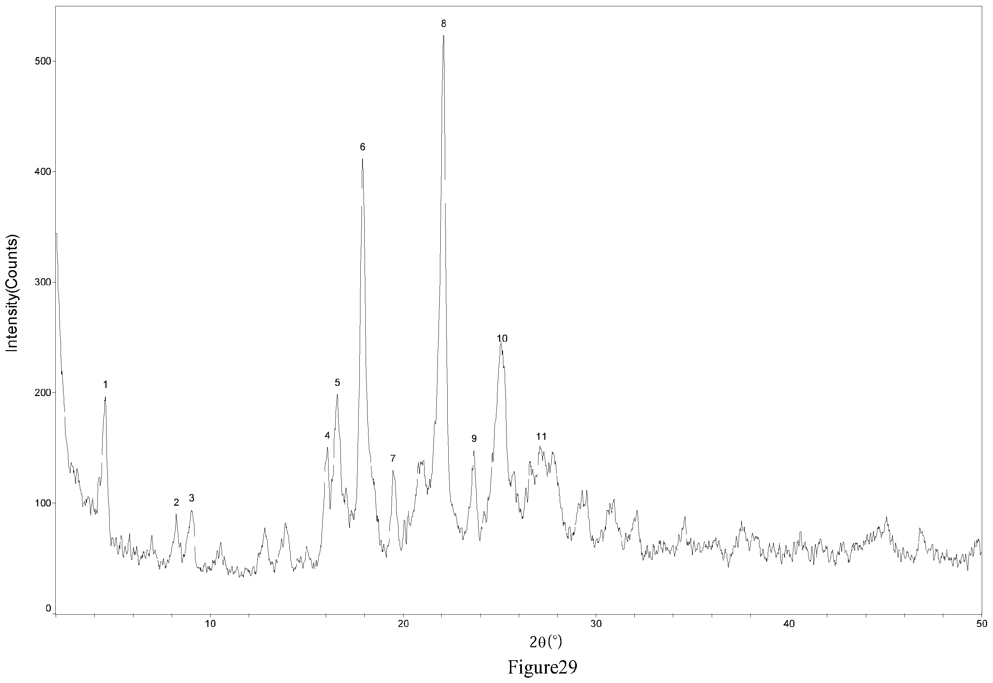

More particularly, the X-ray powder diffraction pattern of the crystal form II of sulfate salt (1:0.5) of nepicastat is substantially as shown in Figure 29.

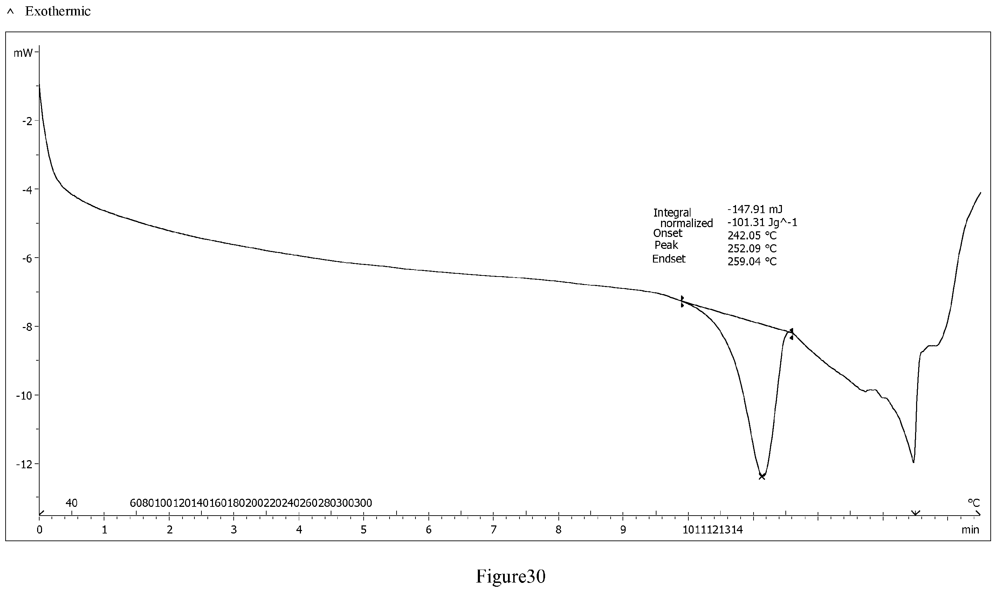

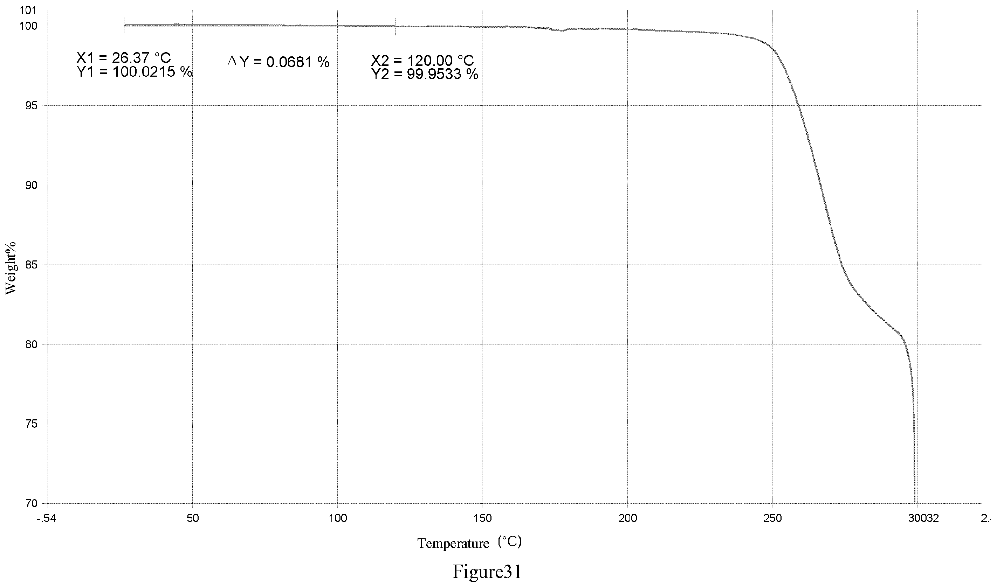

Advantageously, the DSC spectrum of the crystal form II of sulfate salt (1:0.5) of nepicastat shows one endothermic peak at 252.1°C, and more advantageously, is substantially as shown in Figure 30; the TGA spectrum of the crystal form II of sulfate salt (1:0.5) of nepicastat shows a weight loss of 0.07% from room temperature to 120°C, and more advantageously, is substantially as shown in Figure 31.

The X-ray powder diffraction pattern of the crystal form III of sulfate salt (1:1) of nepicastat comprises characteristic peaks at diffraction angles (2θ) of 9.92±0.2°, 16.82±0.2°, 19.84±0.2°, 22.10±0.2°, 22.44±0.2°, 25.18±0.2° and 28.42±0.2°; and preferably, further comprises at least one characteristic peak, for example 1, 2, 3, 4, 5 or 6 characteristic peaks, at diffraction angles (2θ) of 12.40±0.2°, 14.26±0.2°, 24.06±0.2°, 30.68±0.2°, 31.00±0.2° and 35.28±0.2°.

Particularly, the crystal form III of sulfate salt (1:1) of nepicastat is characterized by the XRPD characteristic peaks as shown in Table 7:

**Table 7**

| Peak No. | 2θ | d value | Peak height | Peak height % | Area | Area % |
|---|---|---|---|---|---|---|
| 1 | 9.920 | 8.9092 | 80 | 25.5 | 1784 | 15.4 |
| 2 | 12.399 | 7.1326 | 47 | 15.0 | 1054 | 9.1 |
| 3 | 14.258 | 6.2067 | 53 | 16.9 | 2882 | 24.9 |
| 4 | 16.820 | 5.2666 | 314 | 100.0 | 5939 | 51.3 |
| 5 | 19.842 | 4.4709 | 276 | 87.9 | 8792 | 76.0 |
| 6 | 22.100 | 4.0189 | 179 | 57.0 | 6659 | 57.6 |
| 7 | 22.440 | 3.9587 | 152 | 48.4 | 6504 | 56.2 |
| 8 | 24.060 | 3.6957 | 77 | 24.5 | 1544 | 13.3 |
| 9 | 25.180 | 3.5338 | 280 | 89.2 | 11566 | 100.0 |
| 10 | 28.420 | 3.1379 | 122 | 38.9 | 3887 | 33.6 |
| 11 | 30.679 | 2.9118 | 53 | 16.9 | 2598 | 22.5 |
| 12 | 30.999 | 2.8825 | 68 | 21.7 | 2103 | 18.2 |
| 13 | 32.280 | 2.5419 | 77 | 24.5 | 2377 | 20.6 |

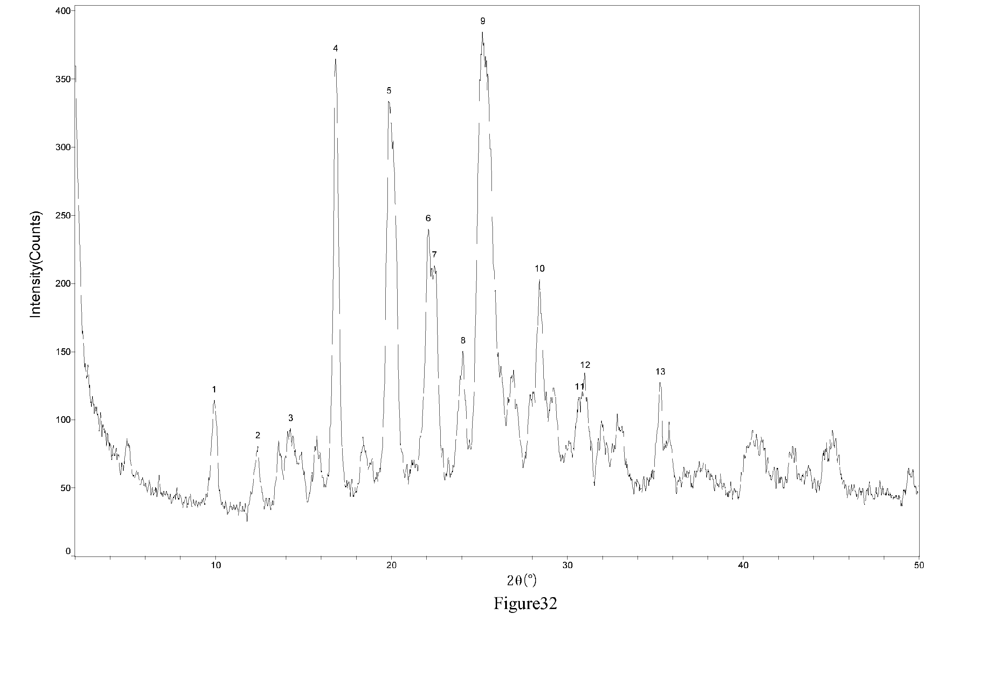

More particularly, the X-ray powder diffraction pattern of the crystal form III of sulfate salt (1:1) of nepicastat is substantially as shown in Figure 32.

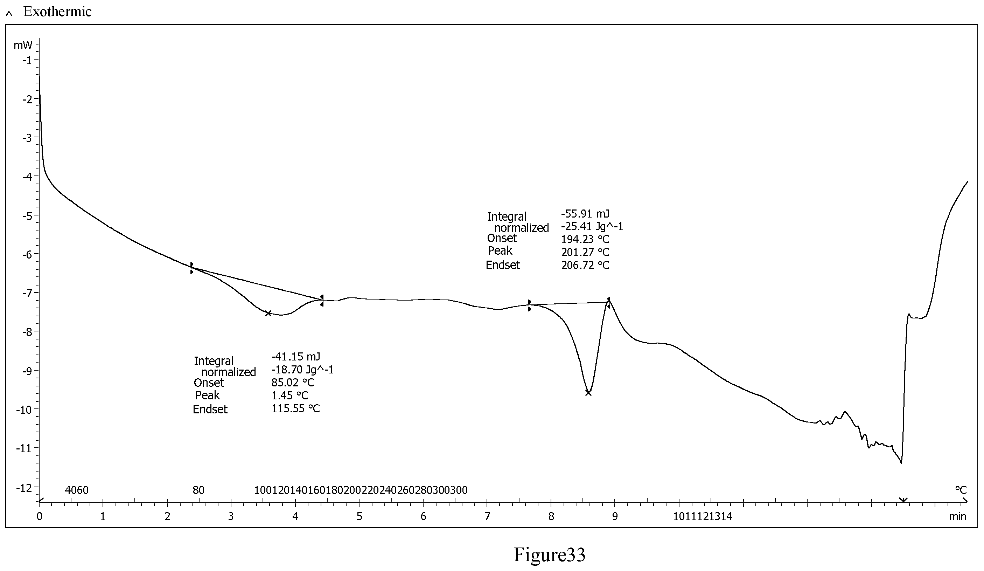

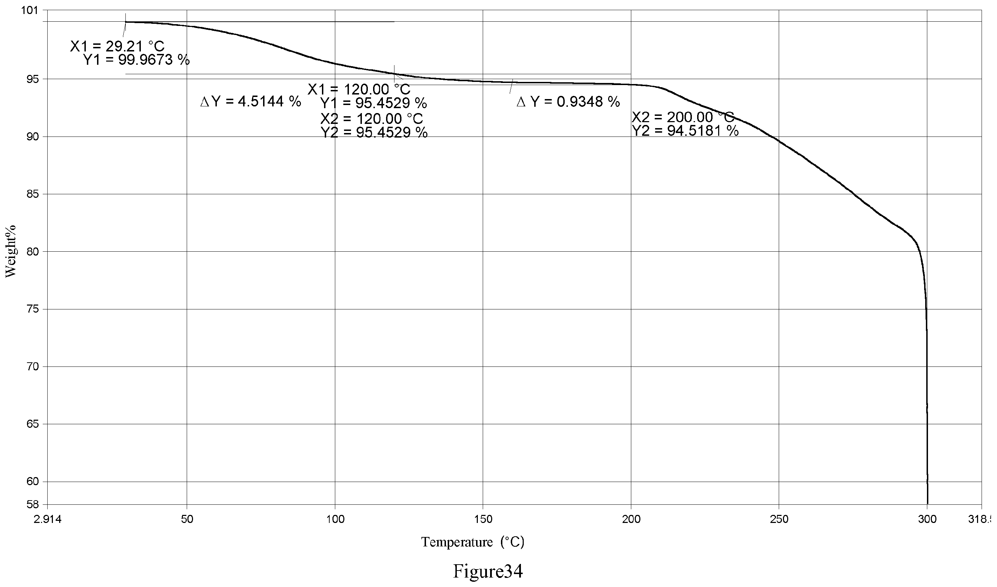

Advantageously, the DSC spectrum of the crystal form III of sulfate salt (1:1) of nepicastat shows 2 endothermic peaks at 101.5°C and 201.3°C, and more advantageously, is substantially as shown in Figure 33; the TGA spectrum of the crystal form III of sulfate salt (1:1) of nepicastat shows a weight loss of 4.51% from room temperature to 120°C, and more advantageously, is substantially as shown in Figure 34.

In some embodiments, the crystal form of the acid addition salt of nepicastat is crystal form I of phosphate salt of nepicastat.

The X-ray powder diffraction pattern of the crystal form I of phosphate salt of nepicastat comprises characteristic peaks at diffraction angles (2θ) of 5.24±0.2°, 10.48±0.2°, 21.14±0.2°, 22.76±0.2°, 23.74±0.2°, 25.54±0.2°, 26.52±0.2° and 29.74±0.2°; and preferably, further comprises at least one characteristic peak, for example 1, 2, 3 or 4 characteristic peaks, at diffraction angles (2θ) of 12.50±0.2°, 16.92±0.2°, 21.92±0.2° and 27.34±0.2°; and more preferably, further comprises at least one characteristic peak, for example 1, 2, 3, 4, 5, 6 or 7 characteristic peaks, at diffraction angles (2θ) of 13.58±0.2°, 15.48±0.2°, 19.40±0.2°, 20.68±0.2°, 34.66±0.2°, 37.62±0.2° and 39.90±0.2°.

Particularly, the crystal form I of phosphate salt of nepicastat is characterized by the XRPD characteristic peaks as shown in Table 8:

**Table 8**

| Peak No. | 2θ | d value | Peak height | Peak height % | Area | Area % |
|---|---|---|---|---|---|---|
| 1 | 5.240 | 16.8511 | 172 | 29.1 | 4350 | 34.2 |
| 2 | 10.480 | 8.4344 | 162 | 27.4 | 3560 | 28.0 |
| 3 | 12.500 | 7.0754 | 100 | 16.9 | 2272 | 17.9 |
| 4 | 13.580 | 6.5150 | 44 | 7.4 | 1231 | 9.7 |
| 5 | 15.480 | 5.7195 | 66 | 11.2 | 1569 | 12.4 |
| 6 | 16.920 | 5.2357 | 111 | 18.8 | 2303 | 18.1 |
| 7 | 19.399 | 4.5720 | 56 | 9.5 | 1689 | 13.3 |
| 8 | 20.680 | 4.2916 | 87 | 14.7 | 2794 | 22.0 |
| 9 | 21.140 | 4.1991 | 165 | 27.9 | 5230 | 41.2 |
| 10 | 21.920 | 4.0515 | 92 | 15.6 | 1370 | 10.8 |
| 11 | 22.760 | 3.9039 | 591 | 100.0 | 12704 | 100.0 |
| 12 | 23.740 | 3.7448 | 205 | 34.7 | 4656 | 36.6 |
| 13 | 25.540 | 3.4848 | 228 | 38.6 | 5380 | 42.3 |
| 14 | 26.520 | 3.3582 | 197 | 33.3 | 5662 | 44.6 |
| 15 | 27.340 | 3.2593 | 168 | 28.4 | 5590 | 44.0 |
| 16 | 29.740 | 3.0016 | 192 | 32.5 | 4589 | 36.1 |
| 17 | 34.661 | 2.5859 | 74 | 12.5 | 2073 | 16.3 |
| 18 | 37.620 | 2.3890 | 54 | 9.1 | 1511 | 11.9 |
| 19 | 39.898 | 2.2577 | 57 | 9.6 | 1648 | 13.0 |

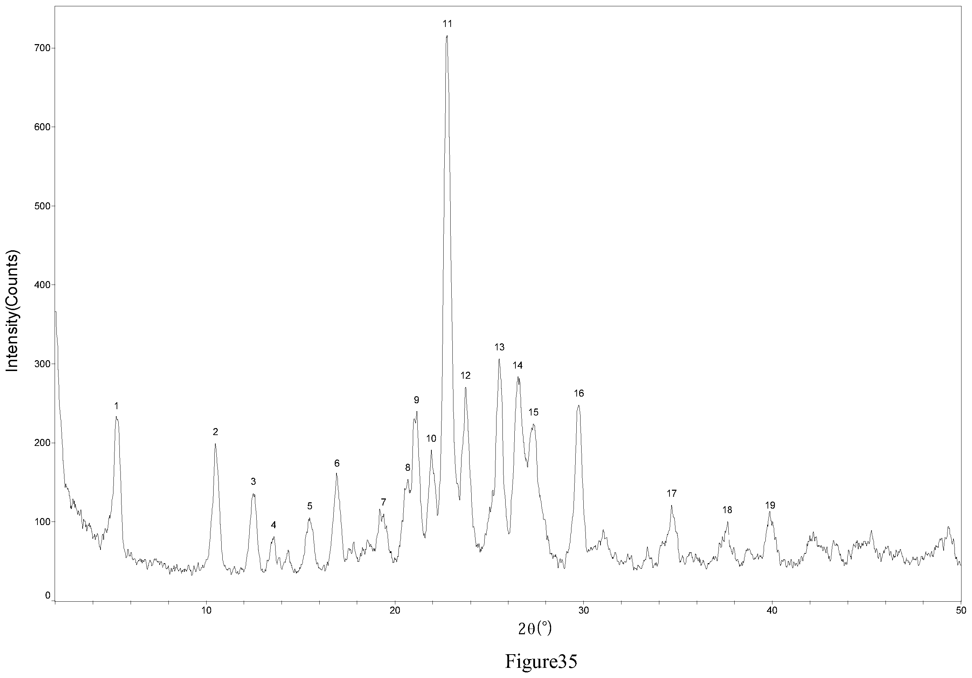

More particularly, the X-ray powder diffraction pattern of the crystal form I of phosphate salt of nepicastat is substantially as shown in Figure 35.

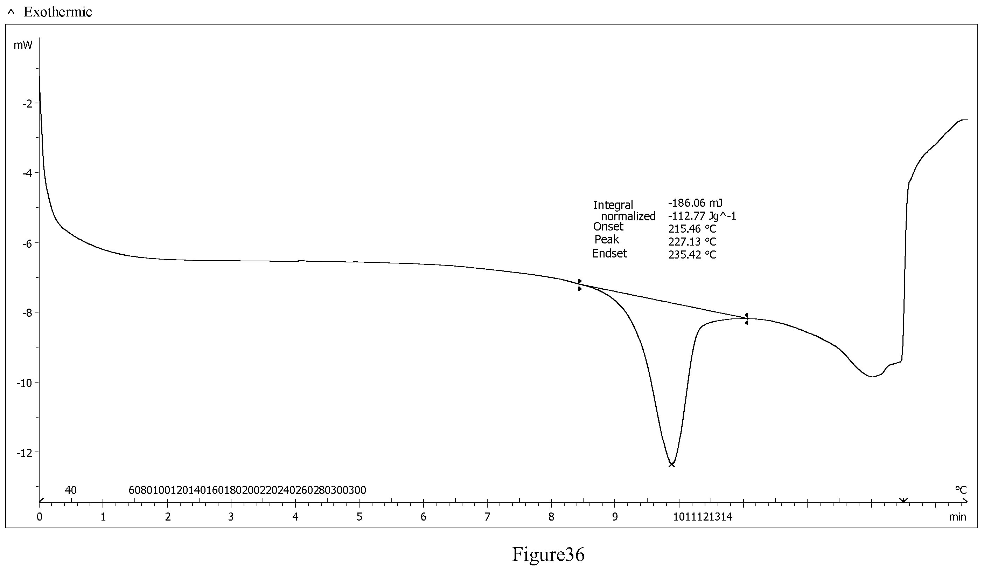

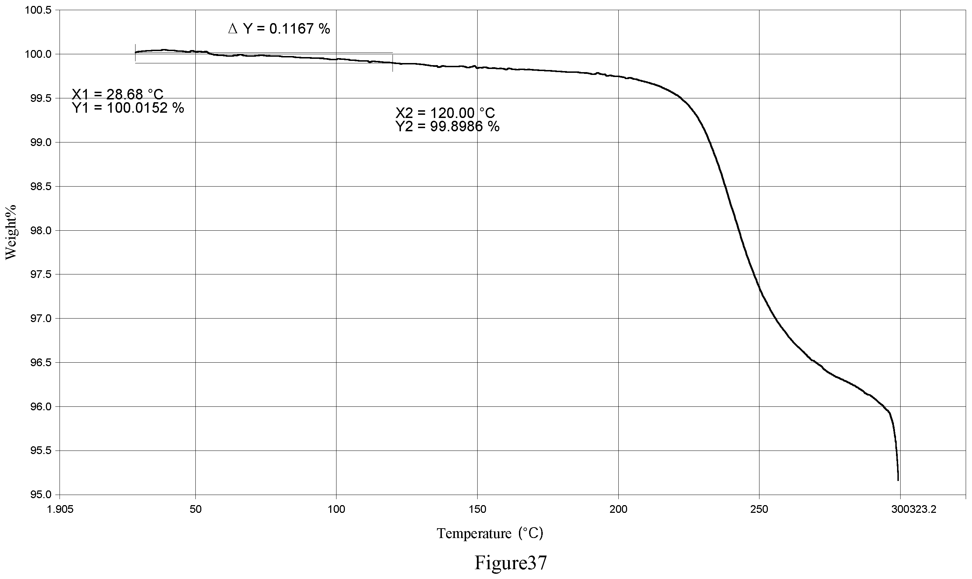

Advantageously, the DSC spectrum of the crystal form I of phosphate salt of nepicastat shows one endothermic peak at 227.1°C, and more advantageously, is substantially as shown in Figure 36; the TGA spectrum of the crystal form I of phosphate salt of nepicastat shows a weight loss of 0.12% from room temperature to 120°C, and more advantageously, is substantially as shown in Figure 37.

In some embodiments, the crystal form of the acid addition salt of nepicastat is crystal form I of methanesulfonate salt of nepicastat.

The X-ray powder diffraction pattern of the crystal form I of methanesulfonate salt of nepicastat comprises characteristic peaks at diffraction angles (2θ) of 17.52±0.2°, 19.74±0.2°, 20.38±0.2°, 20.10±0.2°, 25.00±0.2° and 27.36±0.2°; and preferably, further comprises at least one characteristic peak, for example 1, 2, 3, 4 or 5 characteristic peaks, at diffraction angles (2θ) of 7.90±0.2°, 13.48±0.2°, 15.06±0.2°, 22.88±0.2° and 23.28±0.2°; and more preferably, further comprises at least one characteristic peak, for example 1, 2, 3, 4 or 5 characteristic peaks, at diffraction angles (2θ) of 28.50±0.2°, 29.08±0.2°, 31.42±0.2°, 32.94±0.2° and 36.36±0.2°.

Particularly, the crystal form I of methanesulfonate salt of nepicastat is characterized by the XRPD characteristic peaks as shown in Table 9:

**Table 9**

| Peak No. | 2θ | d value | Peak height | Peak height % | Area | Area % |
|---|---|---|---|---|---|---|
| 1 | 7.900 | 11.1826 | 133 | 15.3 | 1906 | 15.6 |
| 2 | 13.480 | 6.5629 | 106 | 12.2 | 1701 | 13.9 |
| 3 | 15.061 | 5.8775 | 121 | 13.9 | 1778 | 14.6 |
| 4 | 17.520 | 5.0578 | 871 | 100.0 | 12208 | 100.0 |
| 5 | 19.740 | 4.4937 | 188 | 21.6 | 5207 | 42.7 |
| 6 | 20.380 | 4.3540 | 290 | 33.3 | 11951 | 97.9 |
| 7 | 20.999 | 4.2270 | 622 | 71.4 | 11079 | 90.8 |
| 8 | 22.881 | 3.8835 | 120 | 13.8 | 2640 | 21.6 |
| 9 | 23.280 | 3.8178 | 121 | 13.9 | 2183 | 17.9 |
| 10 | 24.122 | 3.6864 | 71 | 8.2 | 694 | 5.7 |
| 11 | 25.000 | 3.5589 | 176 | 20.2 | 3668 | 30.0 |
| 12 | 25.622 | 3.4739 | 73 | 8.4 | 1503 | 12.3 |
| 13 | 26.138 | 3.4064 | 56 | 6.4 | 985 | 8.1 |
| 14 | 27.360 | 3.2570 | 244 | 28.0 | 3732 | 30.6 |
| 15 | 28.500 | 3.1293 | 93 | 10.7 | 2174 | 17.8 |
| 16 | 29.080 | 3.0681 | 78 | 9.0 | 1346 | 11.0 |
| 17 | 31.419 | 2.8448 | 72 | 8.3 | 1795 | 14.7 |
| 18 | 32.939 | 2.7170 | 87 | 10.0 | 1663 | 13.6 |
| 19 | 36.360 | 2.4688 | 61 | 7.0 | 1830 | 15.0 |
| 20 | 38.620 | 2.3294 | 44 | 5.1 | 1191 | 9.8 |
| 21 | 40.758 | 2.2120 | 62 | 7.1 | 897 | 7.3 |
| 22 | 41.939 | 2.1524 | 53 | 6.1 | 964 | 7.9 |
| 23 | 42.811 | 2.1073 | 37 | 4.2 | 994 | 8.1 |
| 24 | 45.459 | 1.9936 | 49 | 5.6 | 1068 | 8.7 |
| 25 | 48.297 | 1.8828 | 28 | 3.2 | 826 | 6.8 |

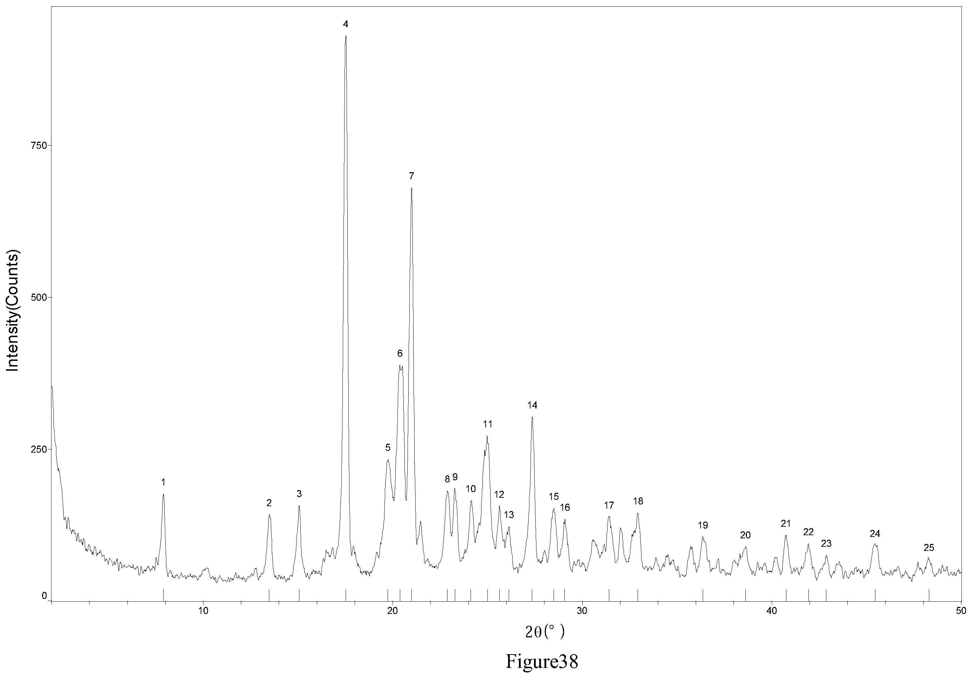

More particularly, the X-ray powder diffraction pattern of the crystal form I of methanesulfonate salt of nepicastat is substantially as shown in Figure 38.

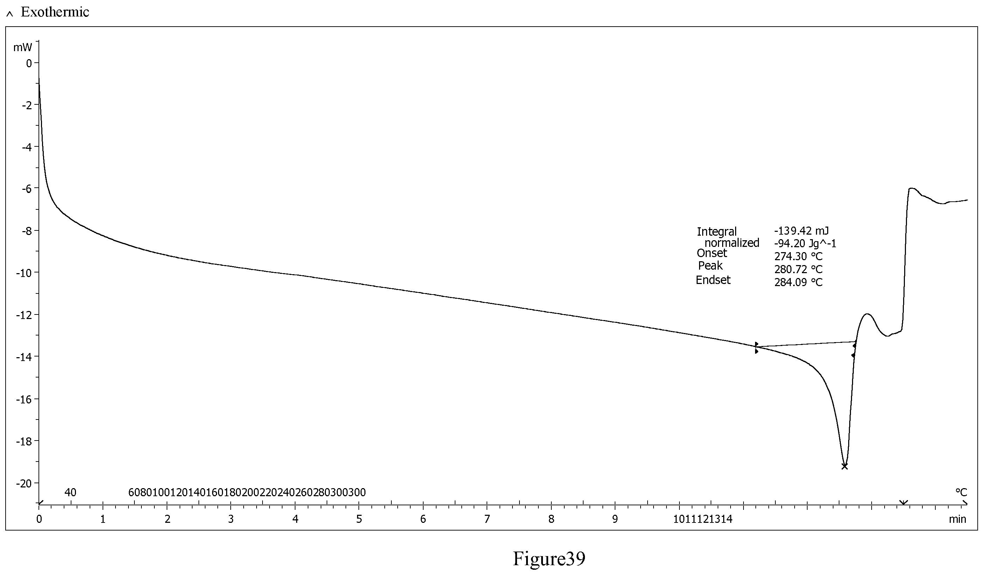

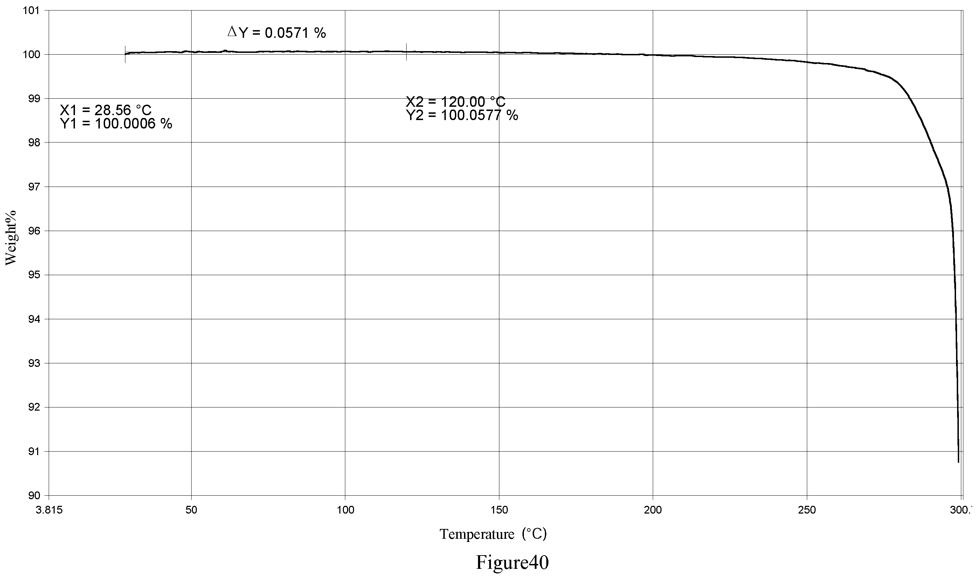

Advantageously, the DSC spectrum of the crystal form I of methanesulfonate salt of nepicastat shows one endothermic peak at 280.7°C, and more advantageously, is substantially as shown in Figure 39; the TGA spectrum of the crystal form I of methanesulfonate salt of nepicastat shows a weight loss of 0.06% from room temperature to 120°C, and more advantageously, is substantially as shown in Figure 40.

In some embodiments, the crystal form of the acid addition salt of nepicastat is crystal form I of benzenesulfonate salt of nepicastat.

The X-ray powder diffraction pattern of the crystal form I of benzenesulfonate salt of nepicastat comprises characteristic peaks at diffraction angles (2θ) of 14.00±0.2°, 16.62±0.2°, 17.96±0.2°, 22.70±0.2°, 24.66±0.2° and 26.86±0.2°; and preferably, further comprises at least one characteristic peak, for example 1, 2, 3 or 4 characteristic peaks, at diffraction angles (2θ) of 7.38±0.2°, 8.06±0.2°, 11.84±0.2° and 20.42±0.2°; and more preferably, further comprises at least one characteristic peak, for example 1, 2, 3 or 4 characteristic peaks, at diffraction angles (2θ) of 19.34±0.2°, 21.62±0.2°, 28.50±0.2° and 30.42±0.2°.

Particularly, the crystal form I of benzenesulfonate salt of nepicastat is characterized by the XRPD characteristic peaks as shown in Table 10:

**Table 10**

| Peak No. | 2θ | d value | Peak height | Peak height % | Area | Area % |
|---|---|---|---|---|---|---|
| 1 | 7.380 | 11.9685 | 64 | 14.6 | 1517 | 11.2 |
| 2 | 8.062 | 10.9581 | 59 | 13.5 | 902 | 6.6 |
| 3 | 11.839 | 7.4687 | 74 | 16.9 | 1716 | 12.6 |
| 4 | 14.000 | 6.3204 | 211 | 48.2 | 4270 | 31.4 |
| 5 | 16.620 | 5.3296 | 172 | 39.3 | 4857 | 35.8 |
| 6 | 17.960 | 4.9349 | 196 | 44.7 | 5321 | 39.2 |
| 7 | 19.339 | 4.5859 | 32 | 7.3 | 1196 | 8.8 |
| 8 | 20.420 | 4.3456 | 85 | 19.4 | 1701 | 12.5 |
| 9 | 21.618 | 4.1073 | 52 | 11.9 | 1150 | 8.5 |
| 10 | 22.700 | 3.9140 | 255 | 58.2 | 5985 | 44.1 |
| 11 | 24.659 | 3.6073 | 257 | 58.7 | 10344 | 76.2 |
| 12 | 26.860 | 3.3165 | 438 | 100.0 | 13579 | 100.0 |
| 13 | 28.501 | 3.1292 | 54 | 12.3 | 1142 | 8.4 |
| 14 | 30.419 | 2.9361 | 60 | 13.7 | 1737 | 12.8 |
| 15 | 37.460 | 2.3988 | 26 | 5.9 | 974 | 7.2 |

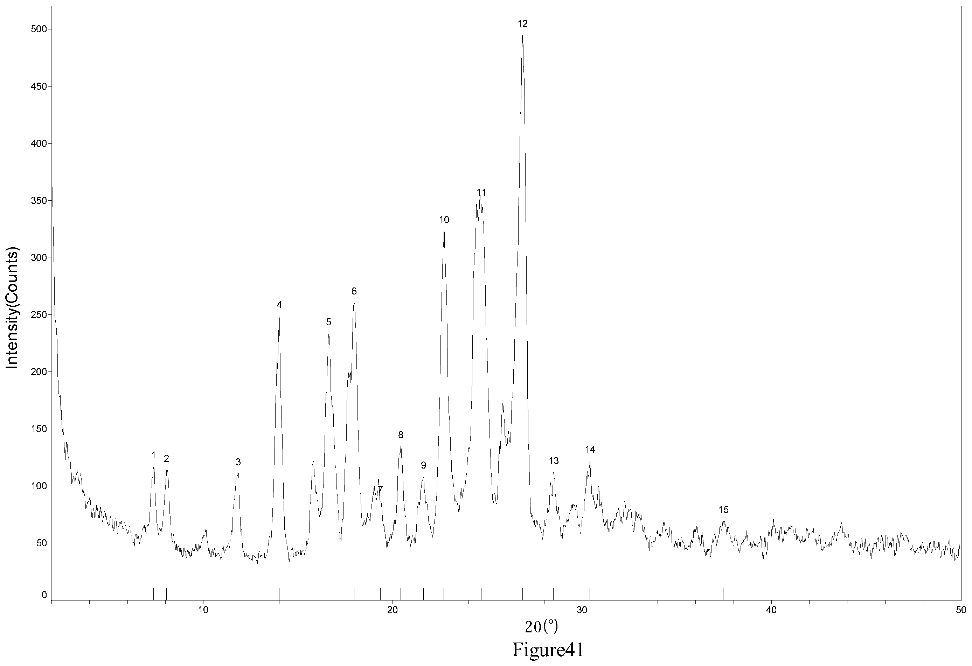

More particularly, the X-ray powder diffraction pattern of the crystal form I of benzenesulfonate salt of nepicastat is substantially as shown in Figure 41.

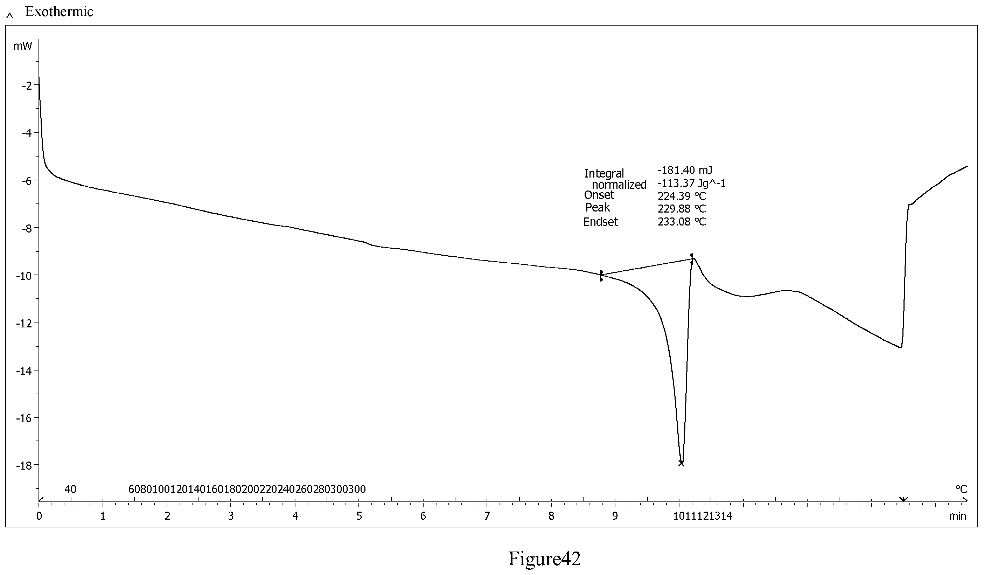

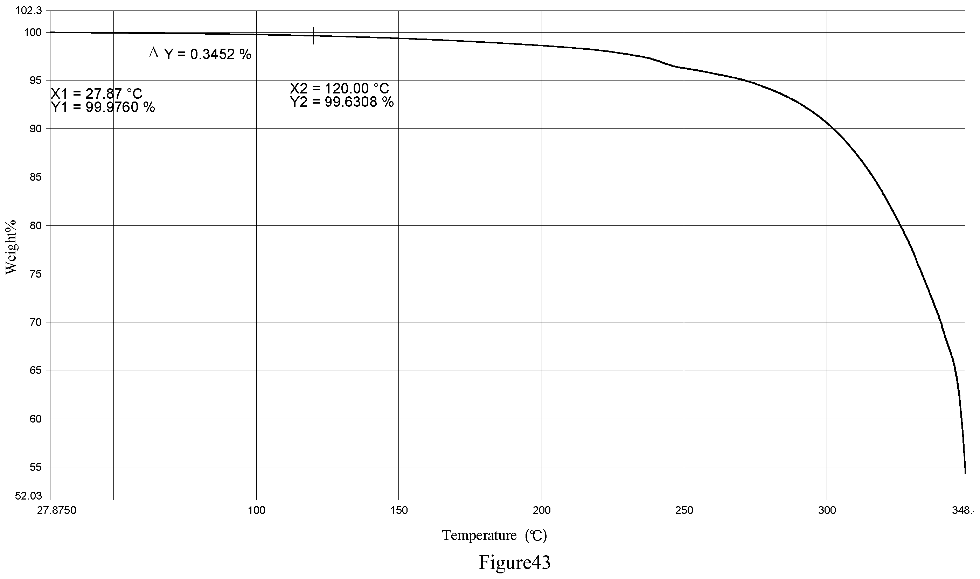

Advantageously, the DSC spectrum of the crystal form I of benzenesulfonate salt of nepicastat shows one endothermic peak at 229.9°C, and more advantageously, is substantially as shown in Figure 42; the TGA spectrum of the crystal form I of benzenesulfonate salt of nepicastat shows a weight loss of 0.35% from room temperature to 120°C, and more advantageously, is substantially as shown in Figure 43.

In some embodiments, the crystal form of the acid addition salt of nepicastat is crystal form I of p-toluenesulfonate salt of nepicastat or crystal form II of p-toluenesulfonate salt of nepicastat.

The X-ray powder diffraction pattern of the crystal form I of p-toluenesulfonate salt of nepicastat comprises characteristic peaks at diffraction angles (2θ) of 13.40±0.2°, 15.52±0.2°, 19.38±0.2°, 19.78±0.2°, 23.52±0.2° and 28.74±0.2°.

Particularly, the crystal form I of p-toluenesulfonate salt of nepicastat is characterized by the XRPD characteristic peaks as shown in Table 11:

**Table 11**

| Peak No. | 2θ | d value | Peak height | Peak height % | Area | Area % |
|---|---|---|---|---|---|---|
| 1 | 13.397 | 6.6035 | 41 | 26.6 | 1806 | 27.6 |
| 2 | 15.520 | 5.7047 | 73 | 47.4 | 1708 | 26.1 |
| 3 | 19.380 | 4.5765 | 154 | 100.0 | 5297 | 80.9 |
| 4 | 19.779 | 4.4850 | 109 | 70.8 | 6544 | 100.0 |
| 5 | 23.520 | 3.7794 | 86 | 55.8 | 1723 | 26.3 |
| 6 | 28.738 | 3.1039 | 40 | 26.0 | 1559 | 23.8 |

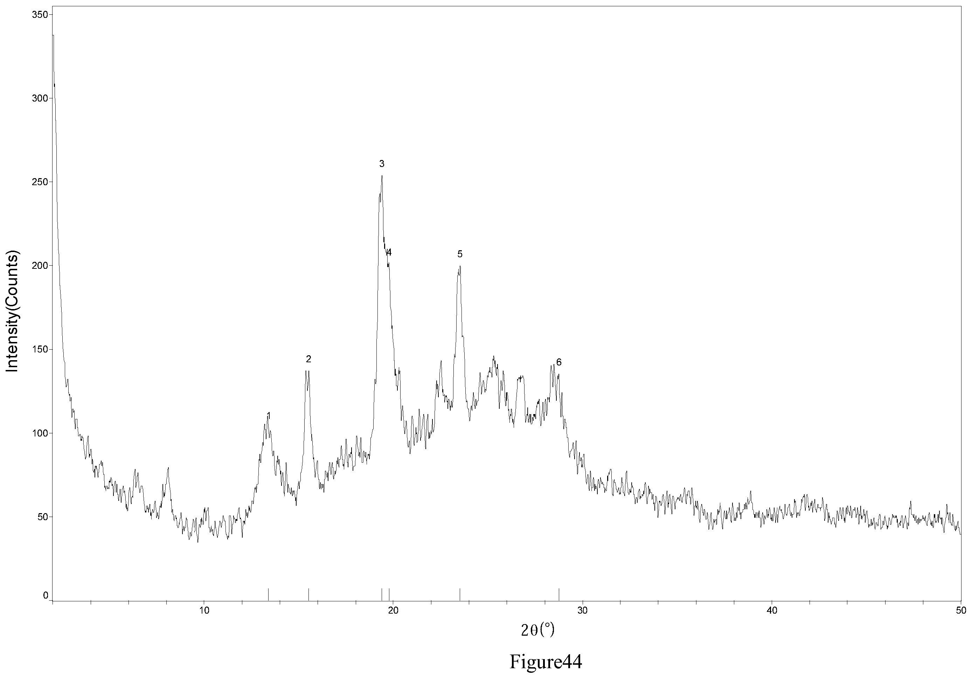

More particularly, the X-ray powder diffraction pattern of the crystal form I of p-toluenesulfonate salt of nepicastat is substantially as shown in Figure 44.

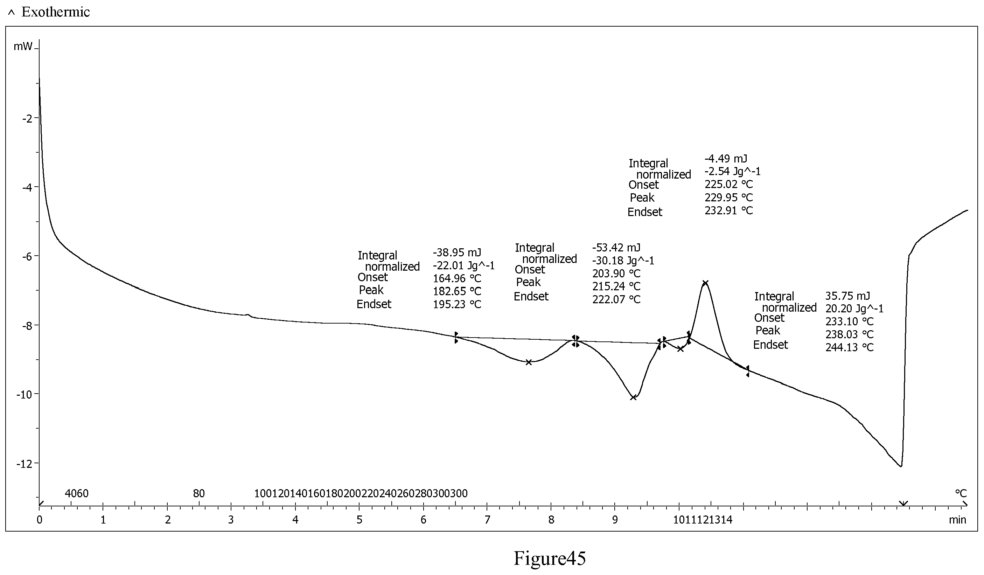

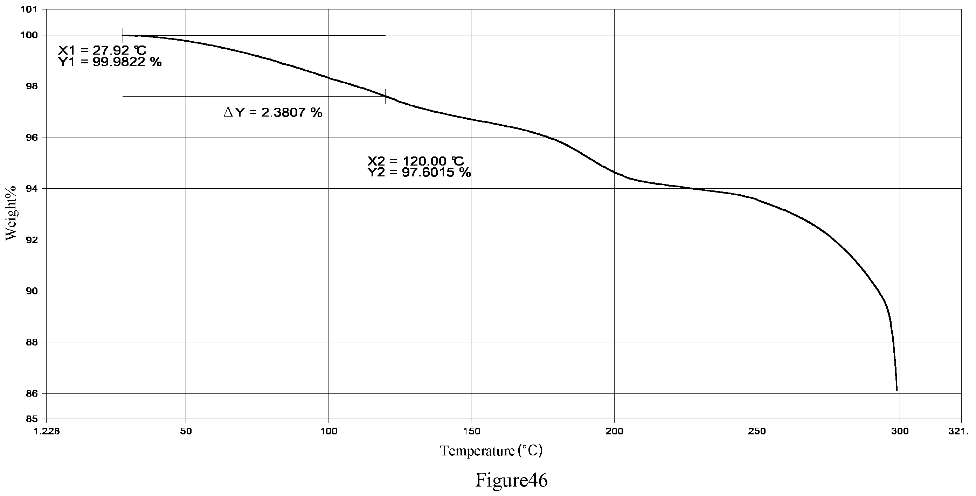

Advantageously, the DSC spectrum of the crystal form I of p-toluenesulfonate salt of nepicastat shows 3 endothermic peaks at 182.7°C, 215.2°C and 230.0°C and one exothermic peak at 238.0°C, and more advantageously, is substantially as shown in Figure 45; the TGA spectrum of the crystal form I of p-toluenesulfonate salt of nepicastat shows a weight loss of 2.38% from room temperature to 120°C, and more advantageously, is substantially as shown in Figure 46.

The X-ray powder diffraction pattern of the crystal form II of p-toluenesulfonate salt of nepicastat comprises characteristic peaks at diffraction angles (2θ) of 4.80±0.2°, 16.62±0.2°, 17.04±0.2°, 17.54±0.2°, 19.34±0.2° and 20.14±0.2°; and preferably, further comprises at least one characteristic peak, for example 1, 2 or 3 characteristic peaks, at diffraction angles (2θ) of 9.44±0.2°, 15.46±0.2° and 21.46±0.2°; and more preferably, further comprises at least one characteristic peak, for example 1, 2, 3 or 4 characteristic peaks, at diffraction angles (2θ) of 24.78±0.2°, 25.10±0.2°, 26.34±0.2° and 29.20±0.2°.

Particularly, the crystal form II of p-toluenesulfonate salt of nepicastat is characterized by the XRPD characteristic peaks as shown in Table 12:

**Table 12**

| Peak No. | 2θ | d value | Peak height | Peak height % | Area | Area % |
|---|---|---|---|---|---|---|
| 1 | 4.800 | 18.3961 | 947 | 100.0 | 14790 | 100.0 |
| 2 | 9.440 | 9.3611 | 74 | 7.8 | 1346 | 9.1 |
| 3 | 15.459 | 5.7271 | 108 | 11.4 | 1969 | 13.3 |
| 4 | 16.620 | 5.3296 | 139 | 14.7 | 4410 | 29.8 |
| 5 | 17.040 | 5.1990 | 145 | 15.3 | 7781 | 52.6 |
| 6 | 17.541 | 5.0517 | 87 | 9.2 | 1409 | 9.5 |
| 7 | 19.340 | 4.5858 | 172 | 18.2 | 3951 | 26.7 |
| 8 | 20.139 | 4.4055 | 206 | 21.8 | 4935 | 33.4 |
| 9 | 21.461 | 4.1372 | 98 | 10.3 | 4114 | 27.8 |
| 10 | 24.278 | 3.6630 | 95 | 10.0 | 1814 | 12.3 |
| 11 | 25.097 | 3.5453 | 84 | 8.9 | 1057 | 7.1 |
| 12 | 26.341 | 3.3807 | 106 | 11.2 | 4669 | 31.6 |
| 13 | 28.541 | 3.1248 | 55 | 5.8 | 3025 | 20.5 |
| 14 | 29.201 | 3.0558 | 93 | 9.8 | 2933 | 19.8 |
| 15 | 32.880 | 2.7217 | 42 | 4.4 | 1106 | 7.5 |

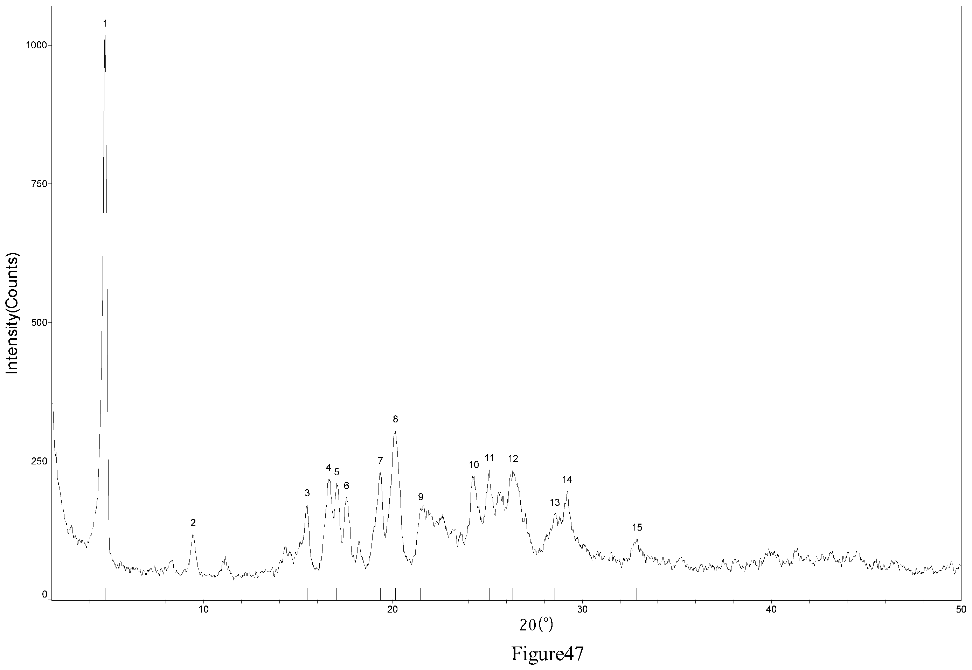

More particularly, the X-ray powder diffraction pattern of the crystal form II of p-toluenesulfonate salt of nepicastat is substantially as shown in Figure 47.

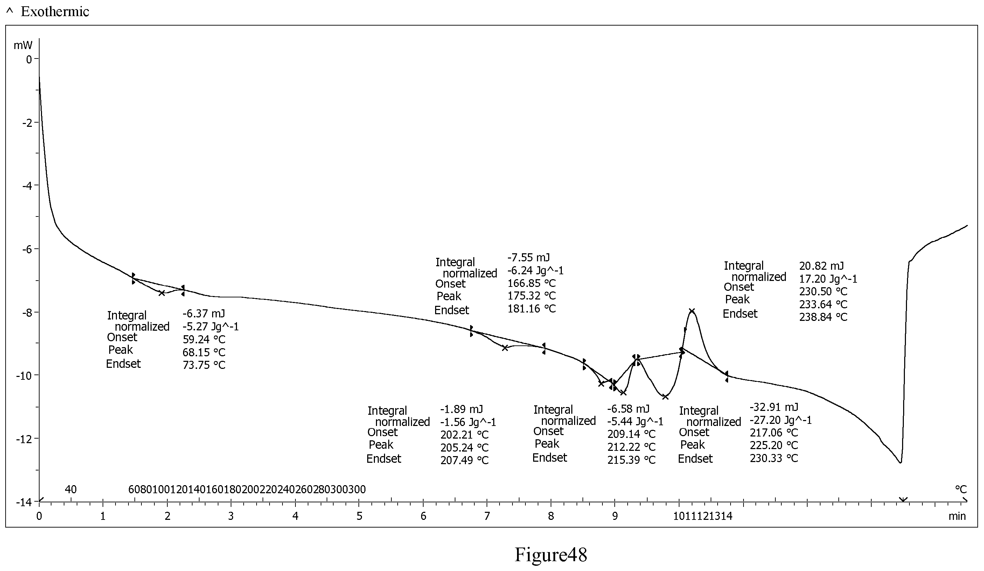

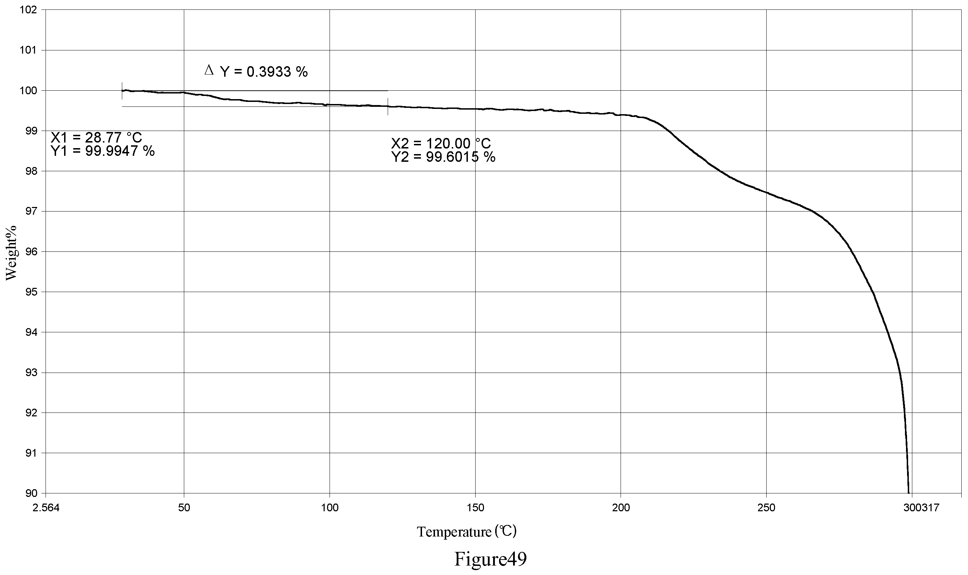

Advantageously, the DSC spectrum of the crystal form II of p-toluenesulfonate salt of nepicastat shows 5 endothermic peaks at 68.2°C, 175.3°C, 205.2°C, 212.2°C and 225.2°C and one exothermic peak at 233.6°C, and more advantageously, is substantially as shown in Figure 48; the TGA spectrum of the crystal form II of p-toluenesulfonate salt of nepicastat shows a weight loss of 0.39% from room temperature to 120°C, and more advantageously, is substantially as shown in Figure 49.

In some embodiments, the crystal form of the acid addition salt of nepicastat is crystal form I of hydrobromide salt of nepicastat or crystal form II of hydrobromide salt of nepicastat.

The X-ray powder diffraction pattern of the crystal form I of hydrobromide salt of nepicastat comprises characteristic peaks at diffraction angles (2θ) of 13.88±0.2°, 14.60±0.2°, 17.74±0.2°, 18.14±0.2°, 22.84±0.2° and 25.76±0.2°; and preferably, further comprises at least one characteristic peak, for example 1, 2 or 3 characteristic peaks, at diffraction angles (2θ) of 20.34±0.2°, 24.14±0.2° and 26.52±0.2°; and more preferably, further comprises at least one characteristic peak, for example 1, 2, 3 or 4 characteristic peaks, at diffraction angles (2θ) of 15.64±0.2°, 16.12±0.2°, 27.14±0.2° and 28.18±0.2°.

Particularly, the crystal form I of hydrobromide salt of nepicastat is characterized by the XRPD characteristic peaks as shown in Table 13:

**Table 13**

| Peak No. | 2θ | d value | Peak height | Peak height % | Area | Area % |
|---|---|---|---|---|---|---|
| 1 | 13.182 | 6.7111 | 51 | 11.0 | 1174 | 7.4 |
| 2 | 13.880 | 6.3749 | 116 | 25.1 | 2597 | 16.3 |
| 3 | 14.599 | 6.0624 | 162 | 35.1 | 2725 | 17.1 |
| 4 | 15.640 | 5.6613 | 56 | 12.1 | 1861 | 11.7 |
| 5 | 16.120 | 5.4937 | 57 | 12.3 | 1888 | 11.8 |
| 6 | 17.741 | 4.9954 | 462 | 100.0 | 15949 | 100.0 |
| 7 | 18.139 | 4.8865 | 256 | 55.4 | 6638 | 41.6 |
| 8 | 20.340 | 4.3625 | 110 | 23.8 | 2656 | 16.7 |
| 9 | 22.419 | 3.9624 | 54 | 11.7 | 1001 | 6.3 |
| 10 | 22.840 | 3.8903 | 295 | 63.9 | 7223 | 45.3 |
| 11 | 24.140 | 3.6836 | 138 | 29.9 | 3024 | 19.0 |
| 12 | 25.760 | 3.4555 | 270 | 58.4 | 5509 | 34.5 |
| 13 | 26.521 | 3.3582 | 95 | 20.6 | 1030 | 6.5 |
| 14 | 27.139 | 3.2831 | 39 | 8.4 | 885 | 5.5 |
| 15 | 28.178 | 3.1643 | 56 | 12.1 | 854 | 5.4 |
| 16 | 36.440 | 2.4636 | 33 | 7.1 | 983 | 6.2 |

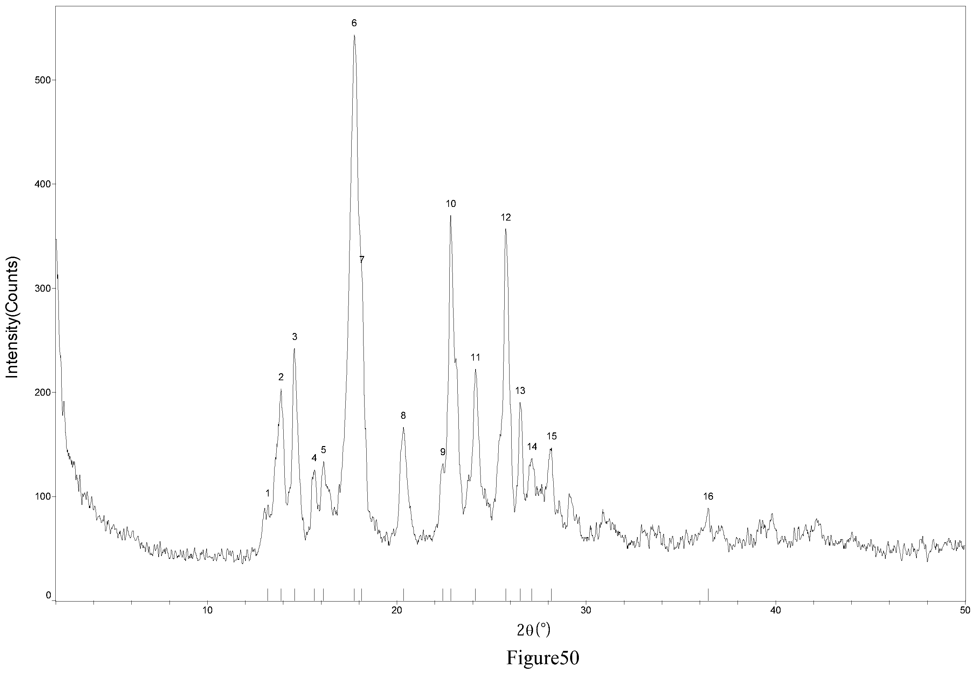

More particularly, the X-ray powder diffraction pattern of the crystal form I of hydrobromide salt of nepicastat is substantially as shown in Figure 50.

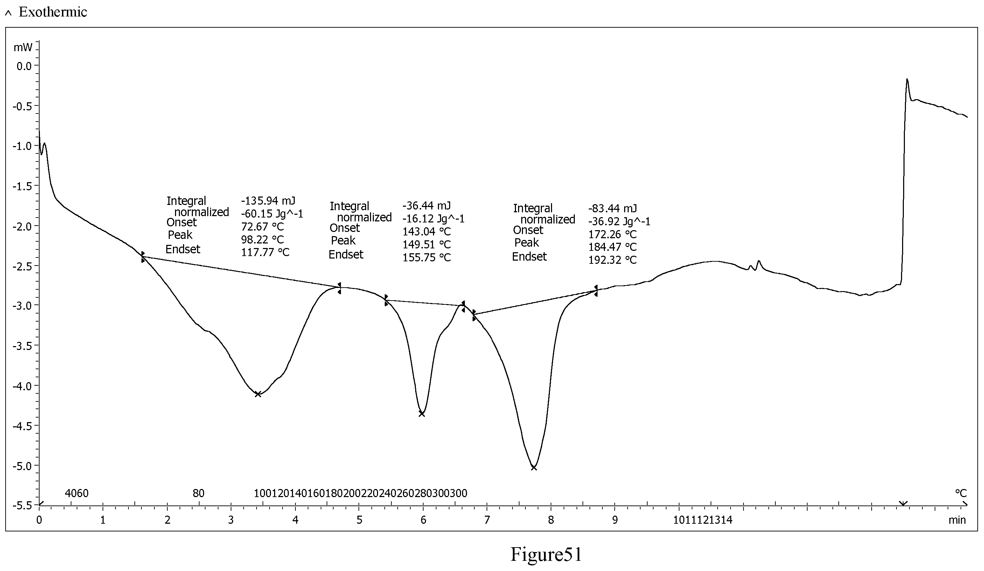

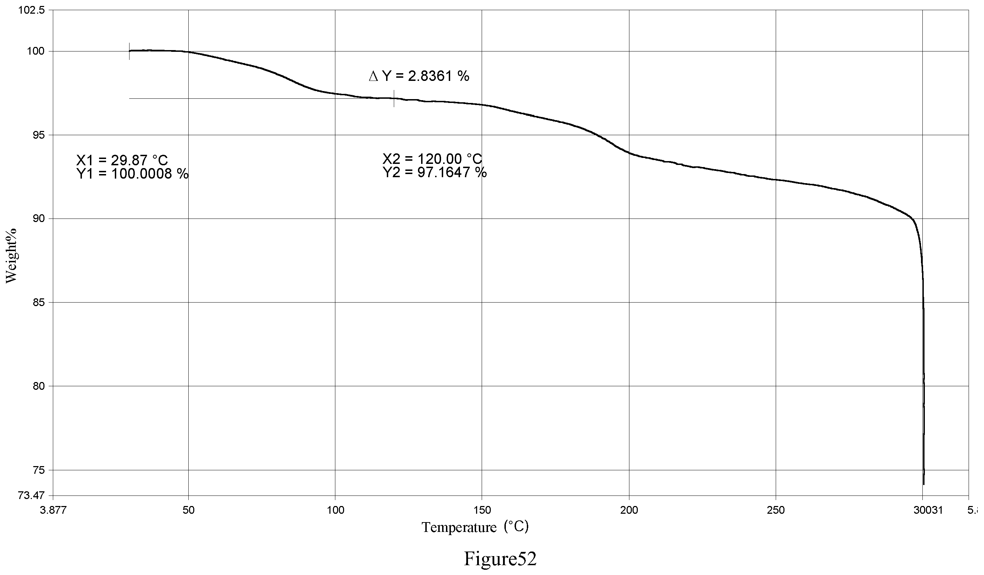

Advantageously, the DSC spectrum of the crystal form I of hydrobromide salt of nepicastat shows 3 endothermic peaks at 98.2°C, 149.5°C and 184.5°C, and more advantageously, is substantially as shown in Figure 51; the TGA spectrum of the crystal form I of hydrobromide salt of nepicastat shows a weight loss of 2.84% from room temperature to 120°C, and more advantageously, is substantially as shown in Figure 52.

The X-ray powder diffraction pattern of the crystal form II of hydrobromide salt of nepicastat comprises characteristic peaks at diffraction angles (2θ) of 16.50±0.2°, 18.34±0.2°, 21.60±0.2°, 22.16±0.2°, 23.96±0.2°, 24.82±0.2°, 29.90±0.2° and 31.18±0.2°; and preferably, further comprises at least one characteristic peak, for example 1, 2, 3 or 4 characteristic peaks, at diffraction angles (2θ) of 20.60±0.2°, 26.02±0.2°, 26.42±0.2° and 27.30±0.2°; and more preferably, further comprises at least one characteristic peak, for example 1, 2, 3, 4 or 5 characteristic peaks, at diffraction angles (2θ) of 7.34±0.2°, 14.74±0.2°, 28.20±0.2°, 34.98±0.2° and 37.16±0.2°.

Particularly, the crystal form II of hydrobromide salt of nepicastat is characterized by the XRPD characteristic peaks as shown in Table 14:

**Table 14**

| Peak No. | 2θ | d value | Peak height | Peak height % | Area | Area % |
|---|---|---|---|---|---|---|
| 1 | 7.340 | 12.0333 | 77 | 18.4 | 1337 | 11.4 |
| 2 | 13.441 | 6.5820 | 37 | 8.9 | 675 | 5.7 |
| 3 | 14.739 | 6.0052 | 99 | 23.7 | 2129 | 18.1 |
| 4 | 16.500 | 5.3680 | 139 | 33.3 | 2378 | 20.2 |
| 5 | 18.339 | 4.8336 | 136 | 32.5 | 2764 | 23.5 |
| 6 | 19.502 | 4.5479 | 45 | 10.8 | 676 | 5.7 |
| 7 | 20.600 | 4.3081 | 113 | 27.0 | 2497 | 21.2 |
| 8 | 21.600 | 4.1108 | 173 | 41.4 | 4810 | 40.8 |
| 9 | 22.161 | 4.0080 | 418 | 100.0 | 11777 | 100.0 |
| 10 | 23.960 | 3.7110 | 175 | 41.9 | 6434 | 54.6 |
| 11 | 24.820 | 3.5842 | 190 | 45.5 | 2948 | 25.0 |
| 12 | 26.020 | 3.4217 | 120 | 28.7 | 3677 | 31.2 |
| 13 | 26.418 | 3.3709 | 93 | 22.2 | 3670 | 31.2 |
| 14 | 27.300 | 3.2640 | 119 | 28.5 | 2183 | 18.5 |
| 15 | 28.200 | 3.1619 | 96 | 23.0 | 2914 | 24.7 |
| 16 | 28.639 | 3.1144 | 58 | 13.9 | 2463 | 20.9 |
| 17 | 29.899 | 2.9859 | 233 | 55.7 | 5484 | 46.6 |
| 18 | 31.180 | 2.8662 | 213 | 51.0 | 5441 | 46.2 |
| 19 | 31.597 | 2.8293 | 96 | 23.0 | 2610 | 22.2 |
| 20 | 33.860 | 2.6452 | 54 | 12.9 | 1490 | 12.7 |
| 21 | 34.979 | 2.5630 | 80 | 19.1 | 1977 | 16.8 |
| 22 | 35.919 | 2.4981 | 66 | 15.8 | 1587 | 13.5 |
| 23 | 37.159 | 2.4176 | 98 | 23.4 | 2291 | 19.5 |
| 24 | 40.761 | 2.2119 | 53 | 12.7 | 1853 | 15.7 |
| 25 | 42.380 | 2.1310 | 44 | 10.5 | 1168 | 9.9 |
| 26 | 45.238 | 2.0028 | 40 | 9.6 | 617 | 5.2 |
| 27 | 46.518 | 1.9506 | 37 | 8.9 | 1245 | 10.6 |

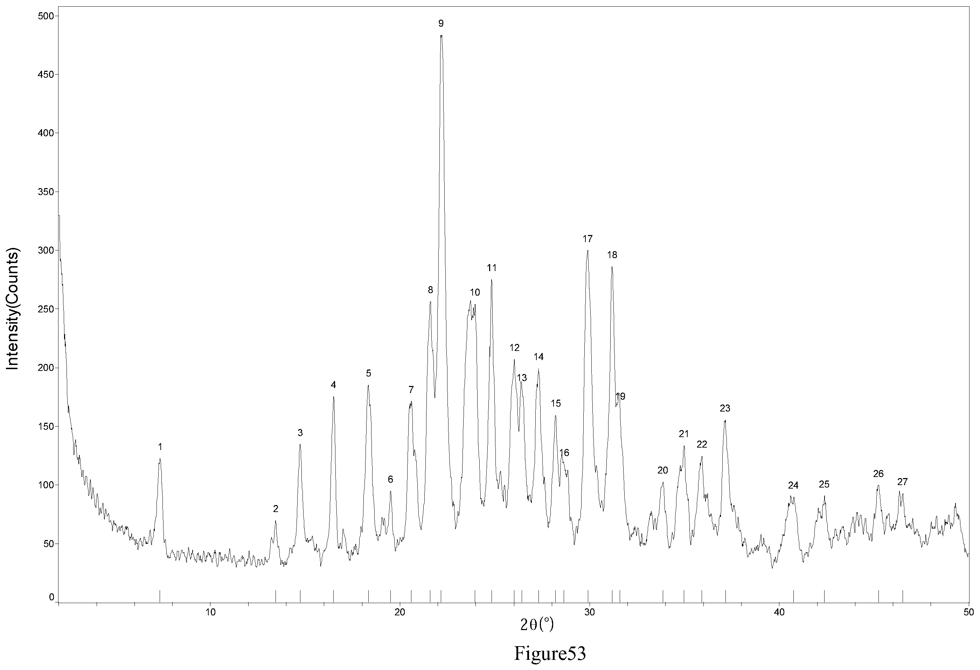

More particularly, the X-ray powder diffraction pattern of the crystal form II of hydrobromide salt of nepicastat is substantially as shown in Figure 53.

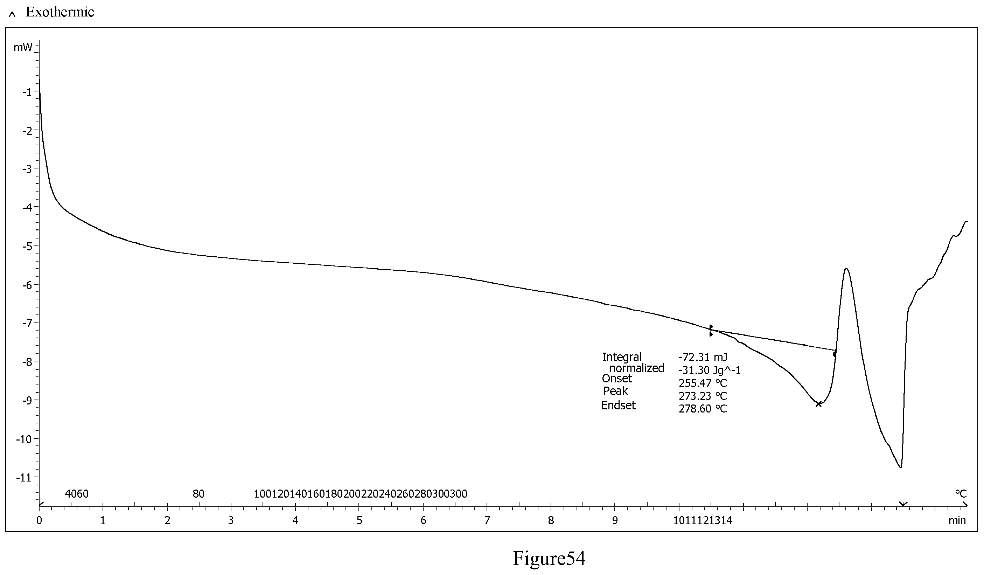

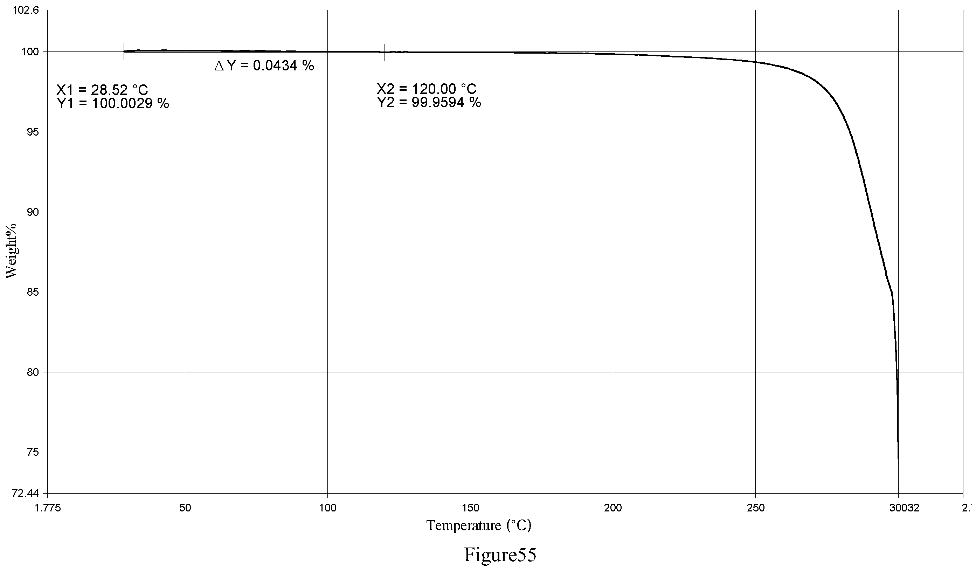

Advantageously, the DSC spectrum of the crystal form II of hydrobromide salt of nepicastat shows one endothermic peak at 273.2°C, and more advantageously, is substantially as shown in Figure 54; the TGA spectrum of the crystal form II of hydrobromide salt of nepicastat shows a weight loss of 0.04% from room temperature to 120°C, and more advantageously, is substantially as shown in Figure 55.

In some embodiments, the crystal form of the acid addition salt of nepicastat is crystal form I of maleate salt of nepicastat.

The X-ray powder diffraction pattern of the crystal form I of maleate salt of nepicastat comprises characteristic peaks at diffraction angles (2θ) of 12.81±0.2°, 16.76±0.2°, 23.96±0.2°, 24.58±0.2°, 25.02±0.2°, 25.94±0.2°, 26.34±0.2° and 28.38±0.2°; and preferably, further comprises at least one characteristic peak, for example 1, 2, 3, 4 or 5 characteristic peaks, at diffraction angles (2θ) of 14.98±0.2°, 18.64±0.2°, 19.36±0.2°, 20.56±0.2° and 21.92±0.2°; and more preferably, further comprises at least one characteristic peak, for example 1, 2, 3, 4 or 5 characteristic peaks, at diffraction angles (2θ) of 16.32±0.2°, 27.72±0.2°, 31.50±0.2°, 36.36±0.2° and 39.46±0.2°.

Particularly, the crystal form I of maleate salt of nepicastat is characterized by the XRPD characteristic peaks as shown in Table 15:

**Table 15**

| Peak No. | 2θ | d value | Peak height | Peak height % | Area | Area % |
|---|---|---|---|---|---|---|
| 1 | 12.181 | 7.2598 | 203 | 12.4 | 4190 | 12.6 |
| 2 | 14.979 | 5.9096 | 121 | 7.4 | 2693 | 8.1 |
| 3 | 16.320 | 5.4268 | 99 | 6.1 | 5212 | 15.6 |
| 4 | 16.760 | 5.2854 | 434 | 26.6 | 9258 | 27.7 |
| 5 | 18.641 | 4.7562 | 132 | 8.1 | 4479 | 13.4 |
| 6 | 19.361 | 4.5809 | 189 | 11.6 | 3273 | 9.8 |
| 7 | 20.560 | 4.3162 | 105 | 6.4 | 2244 | 6.7 |
| 8 | 21.921 | 4.0514 | 103 | 6.3 | 1898 | 5.7 |
| 9 | 23.960 | 3.7110 | 1632 | 100.0 | 33366 | 100.0 |
| 10 | 24.580 | 3.6187 | 442 | 27.1 | 10350 | 31.0 |
| 11 | 25.020 | 3.5560 | 321 | 19.7 | 5830 | 17.5 |
| 12 | 25.941 | 3.4319 | 461 | 28.2 | 11590 | 34.7 |
| 13 | 26.341 | 3.3807 | 304 | 18.6 | 11337 | 34.0 |
| 14 | 27.719 | 3.2156 | 77 | 4.7 | 1783 | 5.3 |
| 15 | 28.380 | 3.1422 | 665 | 40.7 | 15247 | 45.7 |
| 16 | 31.500 | 2.8377 | 173 | 10.6 | 3750 | 11.2 |
| 17 | 36.360 | 2.4688 | 79 | 4.8 | 1794 | 5.4 |
| 18 | 39.459 | 2.2818 | 75 | 4.6 | 1674 | 5.0 |

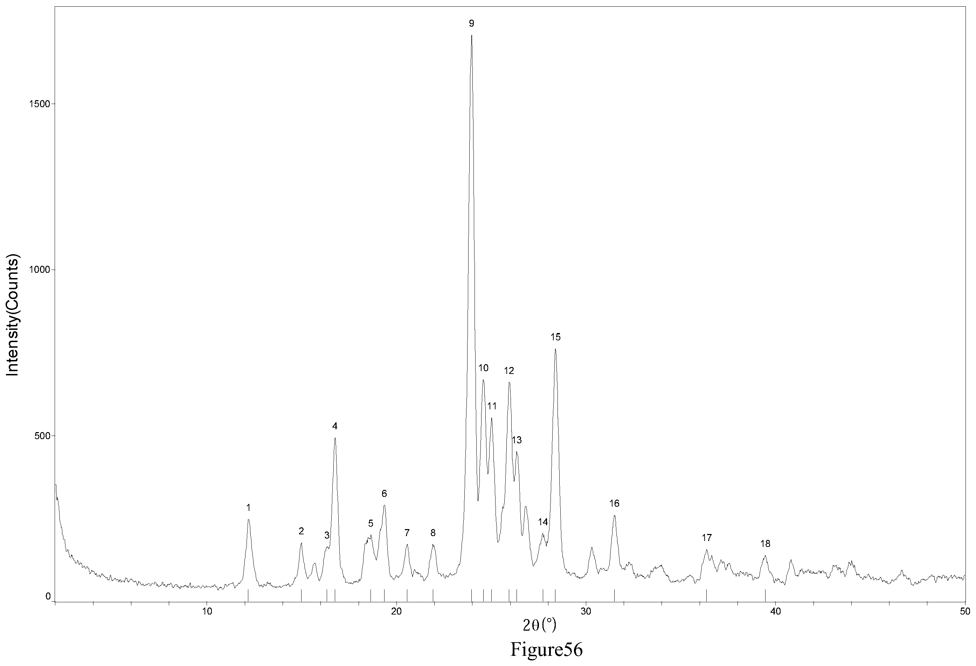

Particularly, the X-ray powder diffraction pattern of the crystal form I of maleate salt of nepicastat is substantially as shown in Figure 56.

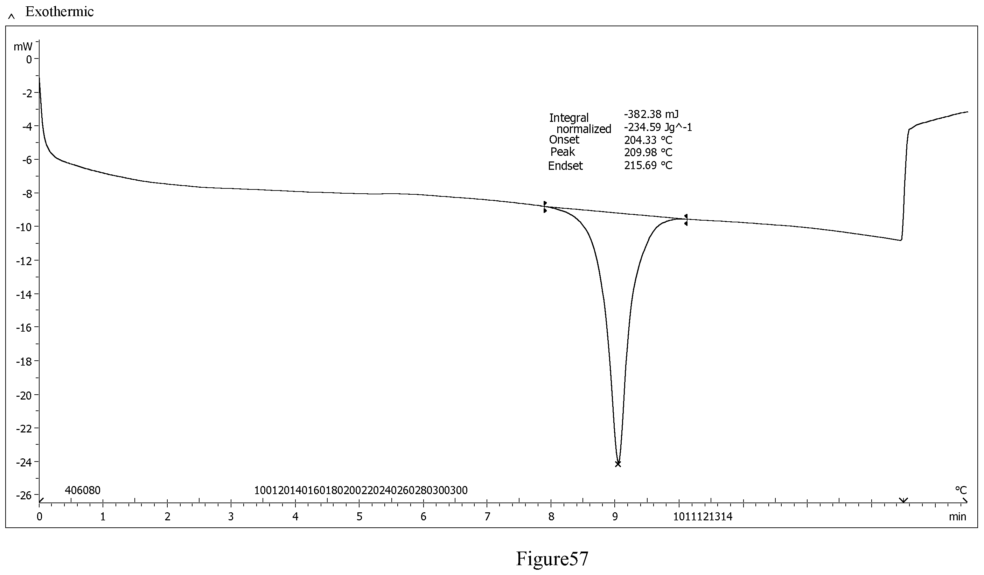

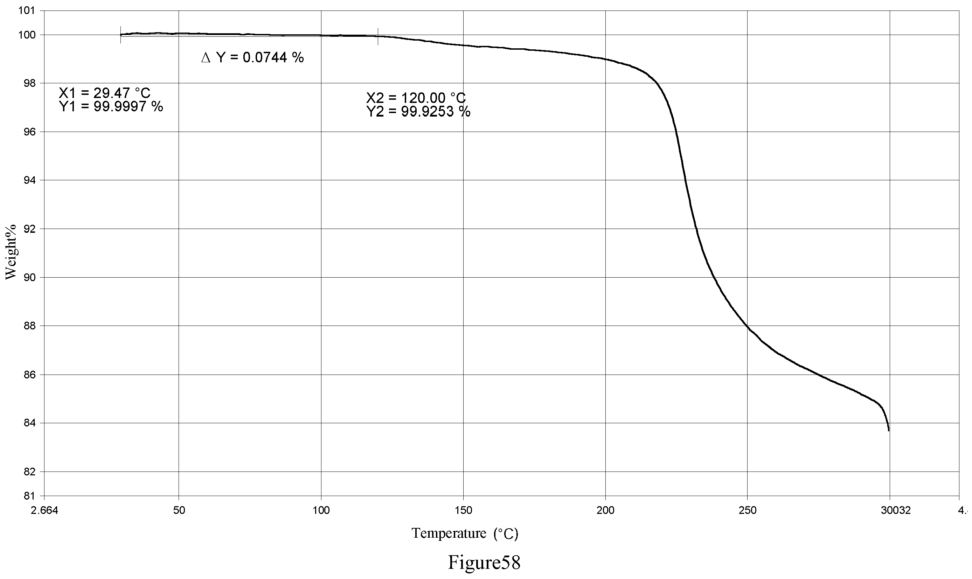

Advantageously, the DSC spectrum of the crystal form I of maleate salt of nepicastat shows one endothermic peak at 210.0°C, and more advantageously, is substantially as shown in Figure 57; the TGA spectrum of the crystal form I of maleate salt of nepicastat shows a weight loss of 0.07% from room temperature to 120°C, and more advantageously, is substantially as shown in Figure 58.

In some embodiments, the crystal form of the acid addition salt of nepicastat is crystal form I of tartrate salt of nepicastat or crystal form II of tartrate salt of nepicastat.

The X-ray powder diffraction pattern of the crystal form I of tartrate salt of nepicastat comprises characteristic peaks at diffraction angles (2θ) of 14.28±0.2°, 19.08±0.2°, 23.66±0.2°, 24.58±0.2°, 26.34±0.2°, 26.98±0.2°, 28.58±0.2° and 31.34±0.2°; and preferably, further comprises at least one characteristic peak, for example 1, 2, 3, 4 or 5 characteristic peaks, at diffraction angles (2θ) of 14.28±0.2°, 15.92±0.2°, 17.84±0.2°, 21.06±0.2° and 28.00±0.2°; and more preferably, further comprises characteristic peak(s), for example 1, 2, 3, 4 or 5 characteristic peaks, at diffraction angles (2θ) of 33.44±0.2°, 37.34±0.2°, 40.34±0.2°, 42.26±0.2° and 46.82±0.2°.

Particularly, the crystal form I of tartrate salt of nepicastat is characterized by the XRPD characteristic peaks as shown in Table 16:

**Table 16**

| Peak No. | 2θ | d value | Peak height | Peak height % | Area | Area % |
|---|---|---|---|---|---|---|
| 1 | 13.319 | 6.6419 | 88 | 22.6 | 2040 | 20.2 |
| 2 | 14.280 | 6.1972 | 196 | 50.3 | 4870 | 48.2 |
| 3 | 15.919 | 5.5626 | 29 | 7.4 | 1144 | 11.3 |
| 4 | 17.840 | 4.9678 | 119 | 30.5 | 3305 | 32.7 |
| 5 | 19.081 | 4.6474 | 390 | 100.0 | 10100 | 100.0 |
| 6 | 21.059 | 4.2152 | 113 | 29.0 | 3754 | 37.2 |
| 7 | 23.660 | 3.7573 | 108 | 27.7 | 2251 | 22.3 |
| 8 | 24.582 | 3.6185 | 80 | 20.5 | 1216 | 12.0 |
| 9 | 26.341 | 3.3806 | 136 | 34.9 | 5669 | 56.1 |
| 10 | 26.979 | 3.3022 | 129 | 33.1 | 5568 | 55.1 |
| 11 | 28.000 | 3.1840 | 60 | 15.4 | 2972 | 29.4 |
| 12 | 28.580 | 3.1206 | 220 | 56.4 | 7579 | 75.0 |
| 13 | 31.340 | 2.8519 | 178 | 45.6 | 6137 | 60.8 |
| 14 | 33.440 | 2.6774 | 67 | 17.2 | 1386 | 13.7 |
| 15 | 37.340 | 2.4062 | 59 | 15.1 | 1829 | 18.1 |
| 16 | 40.340 | 2.2339 | 63 | 16.2 | 1895 | 18.8 |
| 17 | 42.260 | 2.1368 | 35 | 9.0 | 1850 | 18.3 |
| 18 | 46.821 | 1.9387 | 34 | 8.7 | 1051 | 10.4 |

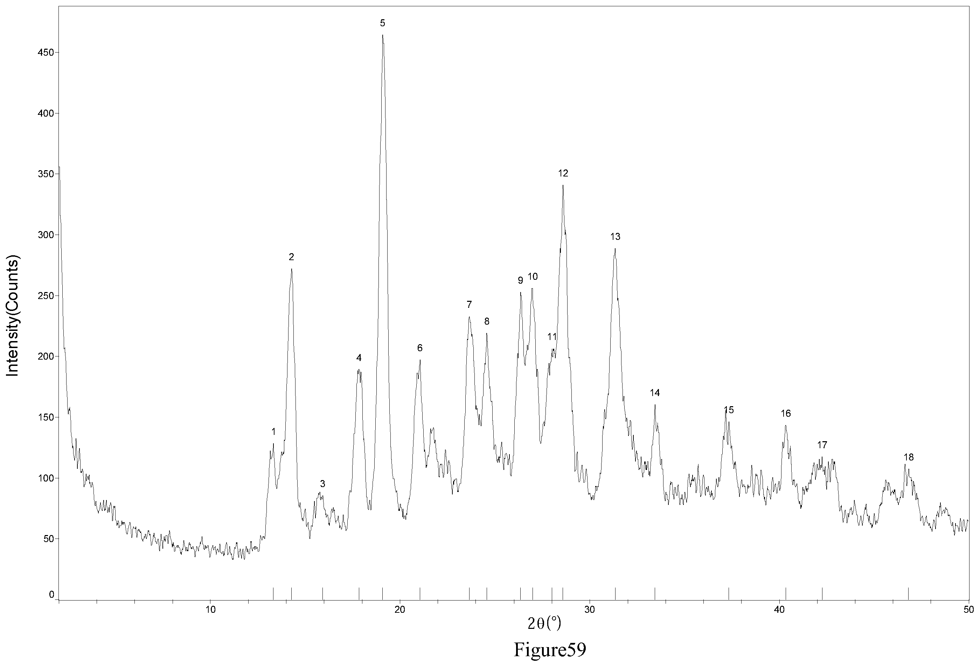

Particularly, the X-ray powder diffraction pattern of the crystal form I of tartrate salt of nepicastat is substantially as shown in Figure 59.

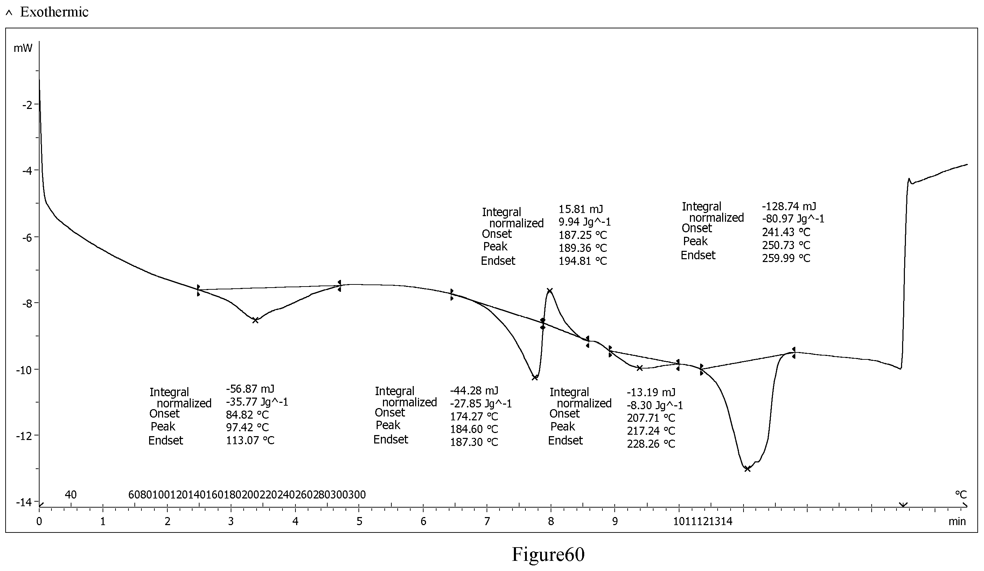

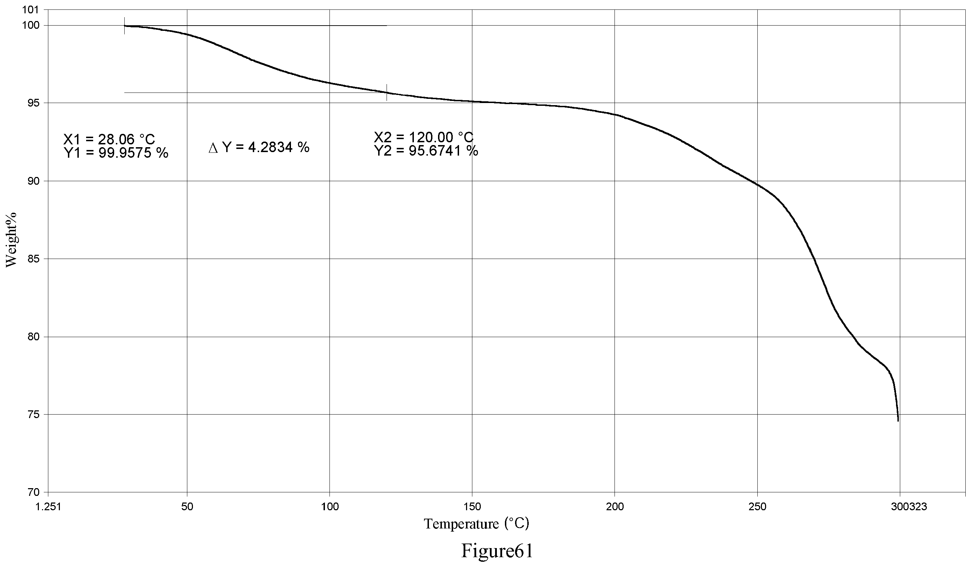

Advantageously, the DSC spectrum of the crystal form I of tartrate salt of nepicastat shows 4 endothermic peaks at 97.4°C, 184.6°C, 217.2°C and 250.7°C and one exothermic peak at 189.4°C, and more advantageously, is substantially as shown in Figure 60; the TGA spectrum of the crystal form I of tartrate salt of nepicastat shows a weight loss of 4.28% from room temperature to 120°C, and more advantageously, is substantially as shown in Figure 61.

The X-ray powder diffraction pattern of the crystal form II of tartrate salt of nepicastat comprises characteristic peaks at diffraction angles (2θ) of 17.72±0.2°, 19.22±0.2°, 21.30±0.2°, 23.82±0.2°, 25.00±0.2°, 25.96±0.2°, 26.62±0.2° and 28.48±0.2°; and preferably, further comprises at least one characteristic peak, for example 1, 2, 3 or 4 characteristic peaks, at diffraction angles (2θ) of 14.22±0.2°, 19.72±0.2°, 21.84±0.2° and 22.42±0.2°; and more preferably, further comprises characteristic peak(s), for example 1, 2, 3, 4, 5 or 6 characteristic peaks, at diffraction angles (2θ) of 13.00±0.2°, 15.20±0.2°, 31.50±0.2°, 33.60±0.2°, 36.08±0.2° and 37.42±0.2°.

Particularly, the crystal form II of tartrate salt of nepicastat is characterized by the XRPD characteristic peaks as shown in Table 17:

**Table 17**

| Peak No. | 2θ | d value | Peak height | Peak height % | Area | Area % |
|---|---|---|---|---|---|---|
| 1 | 10.560 | 8.3703 | 31 | 8.9 | 834 | 11.0 |
| 2 | 12.999 | 6.8049 | 51 | 14.6 | 1659 | 21.9 |
| 3 | 14.219 | 6.2237 | 172 | 49.3 | 3036 | 40.2 |
| 4 | 15.200 | 5.8242 | 72 | 20.6 | 2810 | 37.2 |
| 5 | 15.819 | 5.5976 | 51 | 14.6 | 2730 | 36.1 |
| 6 | 17.720 | 5.0012 | 244 | 69.9 | 6241 | 82.6 |
| 7 | 18.196 | 4.8714 | 68 | 19.5 | 2957 | 39.1 |
| 8 | 19.220 | 4.6141 | 237 | 67.9 | 7559 | 100.0 |
| 9 | 19.719 | 4.4983 | 168 | 48.1 | 6028 | 79.7 |
| 10 | 21.299 | 4.1682 | 169 | 48.4 | 2823 | 37.3 |
| 11 | 21.840 | 4.0660 | 164 | 47.0 | 5042 | 66.7 |
| 12 | 22.419 | 3.9624 | 114 | 32.7 | 2117 | 28.0 |
| 13 | 23.821 | 3.7323 | 186 | 53.3 | 2739 | 36.2 |
| 14 | 25.000 | 3.5589 | 349 | 100.0 | 6960 | 92.1 |
| 15 | 25.961 | 3.4293 | 189 | 54.2 | 5185 | 68.6 |
| 16 | 26.620 | 3.3459 | 159 | 45.6 | 2872 | 38.0 |
| 17 | 28.480 | 3.1314 | 184 | 52.7 | 4246 | 56.2 |
| 18 | 31.498 | 2.8379 | 80 | 22.9 | 2349 | 31.1 |
| 19 | 33.601 | 2.6650 | 59 | 16.9 | 1288 | 17.0 |
| 20 | 36.080 | 2.4873 | 97 | 27.8 | 2366 | 31.3 |
| 21 | 37.420 | 2.4013 | 70 | 20.1 | 1866 | 24.7 |
| 22 | 38.838 | 2.3168 | 35 | 10.0 | 662 | 8.8 |
| 23 | 45.401 | 1.9960 | 43 | 12.3 | 1067 | 14.1 |

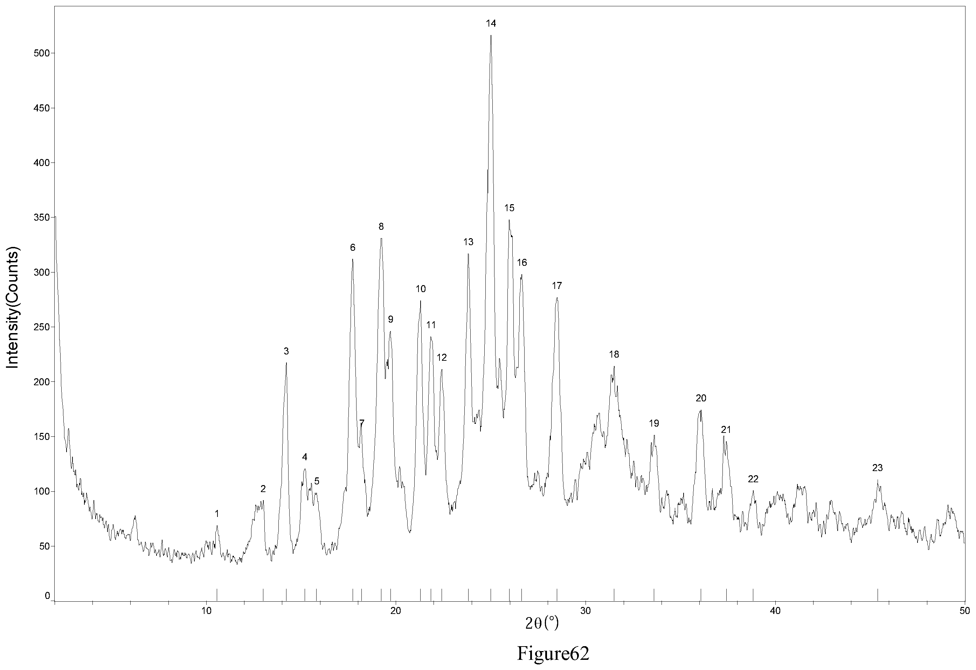

Particularly, the X-ray powder diffraction pattern of the crystal form II of tartrate salt of nepicastat is substantially as shown in Figure 62.

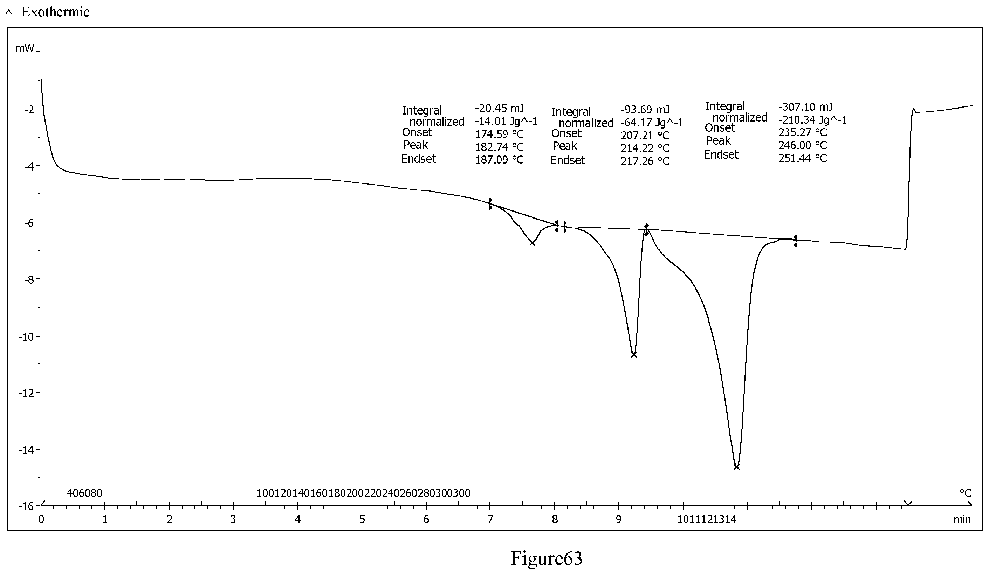

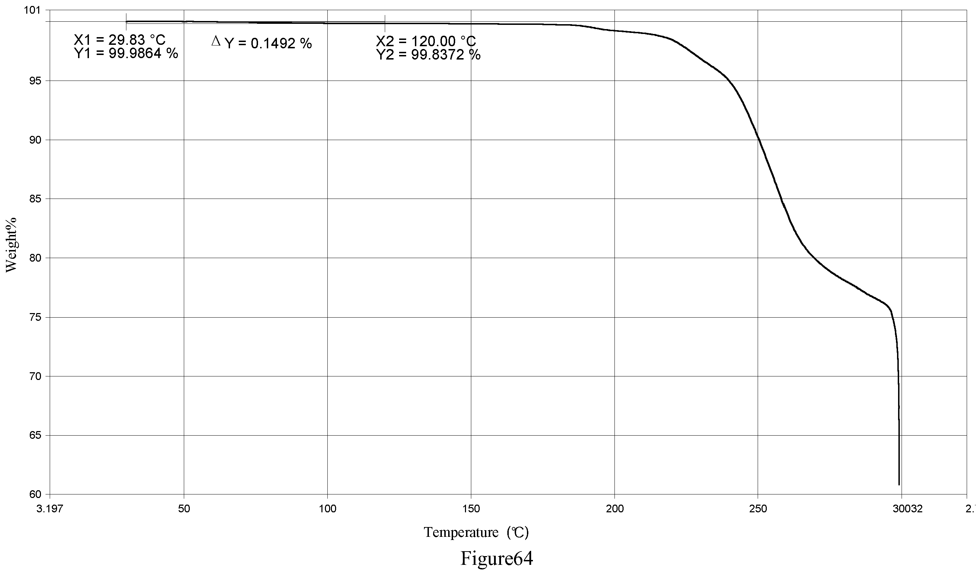

Advantageously, the DSC spectrum of the crystal form II of tartrate salt of nepicastat shows endothermic peaks at 182.7°C, 214.2°C and 246.0°C, and more advantageously, is substantially as shown in Figure 63; the TGA spectrum of the crystal form II of tartrate salt of nepicastat shows a weight loss of 0.15% from room temperature to 120°C, and more advantageously, is substantially as shown in Figure 64.

In some embodiments, the crystal form of the acid addition salt of nepicastat is crystal form I of citrate salt of nepicastat.

The X-ray powder diffraction pattern of the crystal form I of citrate salt of nepicastat comprises characteristic peaks at diffraction angles (2θ) of 12.98±0.2°, 14.30±0.2°, 16.36±0.2°, 17.64±0.2°, 19.38±0.2°, 22.68±0.2°, 25.10±0.2° and 26.34±0.2°; and preferably, further comprises at least one characteristic peak, for example 1, 2, 3 or 4 characteristic peaks, at diffraction angles (2θ) of 22.28±0.2°, 30.26±0.2°, 40.26±0.2° and 40.86±0.2°.

Particularly, the crystal form I of citrate salt of nepicastat is characterized by the XRPD characteristic peaks as shown in Table 18:

**Table 18**

| Peak No. | 2θ | d value | Peak height | Peak height % | Area | Area % |
|---|---|---|---|---|---|---|
| 1 | 12.980 | 6.8151 | 101 | 33.4 | 3193 | 48.2 |
| 2 | 14.300 | 6.1885 | 48 | 15.9 | 847 | 12.8 |
| 3 | 16.358 | 5.4145 | 42 | 13.9 | 1064 | 16.1 |
| 4 | 17.641 | 5.0235 | 53 | 17.5 | 1110 | 16.8 |
| 5 | 19.378 | 4.5767 | 67 | 22.2 | 2984 | 45.1 |
| 6 | 22.280 | 3.9868 | 107 | 35.4 | 6620 | 100.0 |
| 7 | 22.680 | 3.9174 | 114 | 37.7 | 4041 | 61.0 |
| 8 | 25.100 | 3.5449 | 111 | 36.8 | 2396 | 36.2 |
| 9 | 26.339 | 3.3809 | 302 | 100.0 | 5718 | 86.4 |
| 10 | 30.258 | 2.9513 | 53 | 17.5 | 2203 | 33.3 |
| 11 | 40.260 | 2.2382 | 38 | 12.6 | 1887 | 28.5 |
| 12 | 40.862 | 2.2066 | 46 | 15.2 | 1273 | 19.2 |

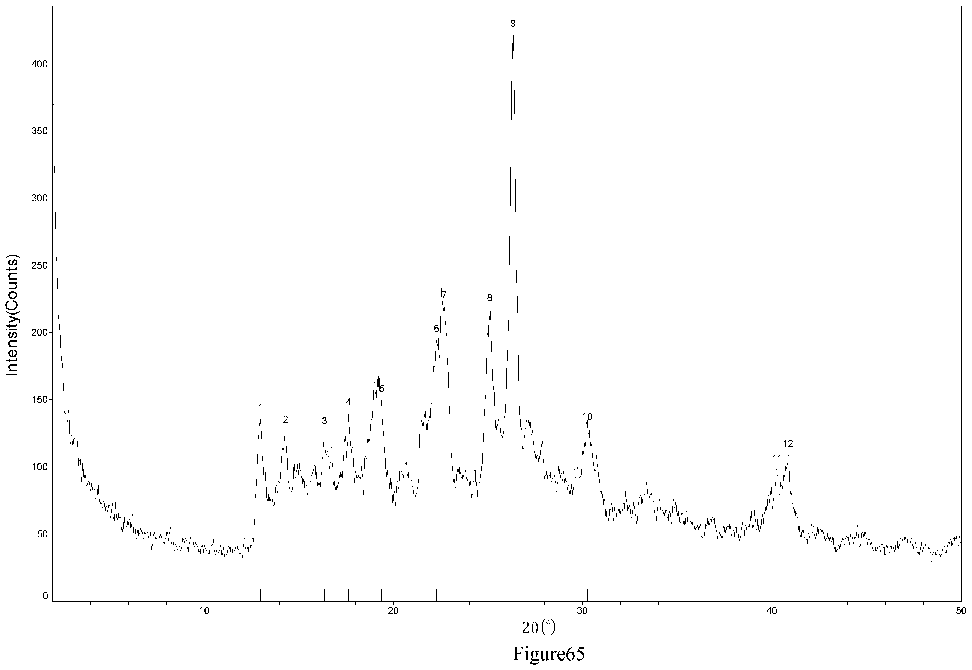

Particularly, the X-ray powder diffraction pattern of the crystal form I of citrate salt of nepicastat is substantially as shown in Figure 65.

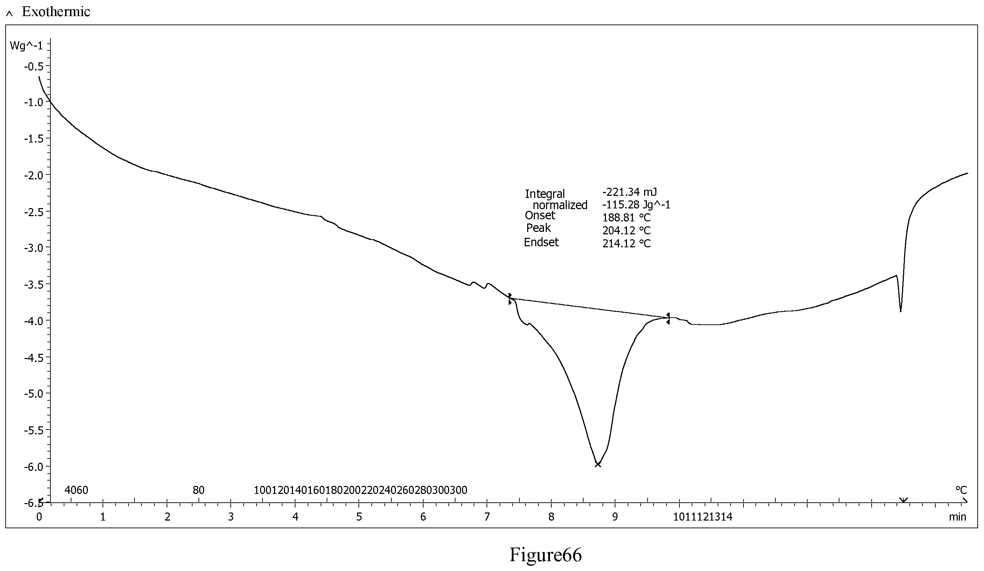

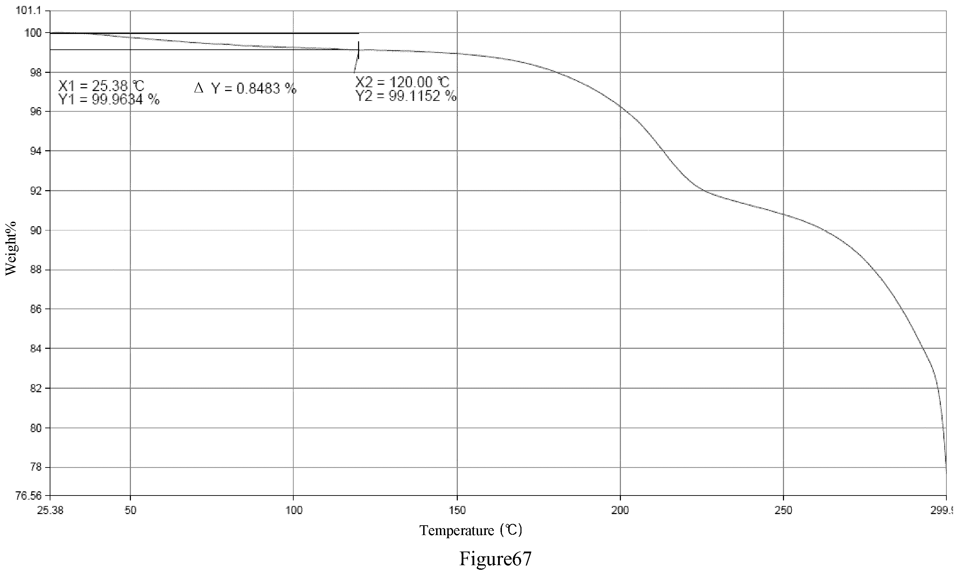

Advantageously, the DSC spectrum of the crystal form I of citrate salt of nepicastat shows one endothermic peak at 204.1°C, and more advantageously, is substantially as shown in Figure 66; the TGA spectrum of the crystal form I of citrate salt of nepicastat shows a weight loss of 0.85% from room temperature to 120°C, and more advantageously, is substantially as shown in Figure 67.

In some embodiments, the crystal form of the acid addition salt of nepicastat is crystal form I of fumarate salt of nepicastat.

The X-ray powder diffraction pattern of the crystal form I of fumarate salt of nepicastat comprises characteristic peaks at diffraction angles (2θ) of 13.58±0.2°, 14.12±0.2°, 15.56±0.2°, 17.18±0.2°, 21.86±0.2°, 23.22±0.2°, 23.98±0.2° and 26.40±0.2°; and preferably, further comprises at least one characteristic peak, for example 1, 2, 3, 4 or 5 characteristic peaks, at diffraction angles (2θ) of 16.20±0.2°, 24.66±0.2°, 25.00±0.2°, 27.92±0.2° and 28.60±0.2°; and more preferably, further comprises characteristic peak(s), for example 1, 2, 3, 4 or 5 characteristic peaks, at diffraction angles (2θ) of 10.78±0.2°, 18.22±0.2°, 19.96±0.2°, 30.32±0.2° and 33.06±0.2°.

Particularly, the crystal form I of fumarate salt of nepicastat is characterized by the XRPD characteristic peaks as shown in Table 19:

**Table 19**

| Peak No. | 2θ | d value | Peak height | Peak height % | Area | Area % |
|---|---|---|---|---|---|---|
| 1 | 10.781 | 8.1997 | 33 | 7.8 | 614 | 5.4 |
| 2 | 13.580 | 6.5150 | 310 | 73.6 | 8369 | 73.9 |
| 3 | 14.121 | 6.2666 | 184 | 43.7 | 5127 | 45.3 |
| 4 | 15.560 | 5.6901 | 244 | 58.0 | 5925 | 52.3 |
| 5 | 16.199 | 5.4671 | 148 | 35.2 | 3409 | 30.1 |
| 6 | 17.180 | 5.1572 | 219 | 52.0 | 3912 | 34.5 |
| 7 | 18.220 | 4.8649 | 45 | 10.7 | 755 | 6.7 |
| 8 | 19.961 | 4.4444 | 52 | 12.4 | 1215 | 10.7 |
| 9 | 21.860 | 4.0624 | 177 | 42.0 | 3677 | 32.5 |
| 10 | 23.220 | 3.8275 | 421 | 100.0 | 7625 | 67.3 |
| 11 | 23.981 | 3.7077 | 239 | 56.8 | 4133 | 36.5 |
| 12 | 24.660 | 3.6072 | 109 | 25.9 | 5050 | 44.6 |
| 13 | 25.003 | 3.5584 | 98 | 23.3 | 2988 | 26.4 |
| 14 | 26.400 | 3.3732 | 323 | 76.7 | 11324 | 100.0 |
| 15 | 26.940 | 3.3068 | 119 | 28.3 | 5092 | 45.0 |
| 16 | 27.919 | 3.1931 | 89 | 21.1 | 3982 | 35.2 |
| 17 | 28.603 | 3.1183 | 74 | 17.6 | 891 | 7.9 |
| 18 | 30.320 | 2.9455 | 114 | 27.1 | 3625 | 32.0 |
| 19 | 33.060 | 2.7073 | 84 | 20.0 | 2649 | 23.4 |
| 20 | 35.598 | 2.5199 | 47 | 11.2 | 1385 | 12.2 |
| 21 | 39.381 | 2.2861 | 52 | 12.4 | 1502 | 13.3 |
| 22 | 40.920 | 2.2036 | 62 | 14.7 | 2418 | 21.4 |
| 23 | 41.238 | 2.1874 | 57 | 13.5 | 2413 | 21.3 |
| 24 | 43.060 | 2.0989 | 33 | 7.8 | 869 | 7.7 |

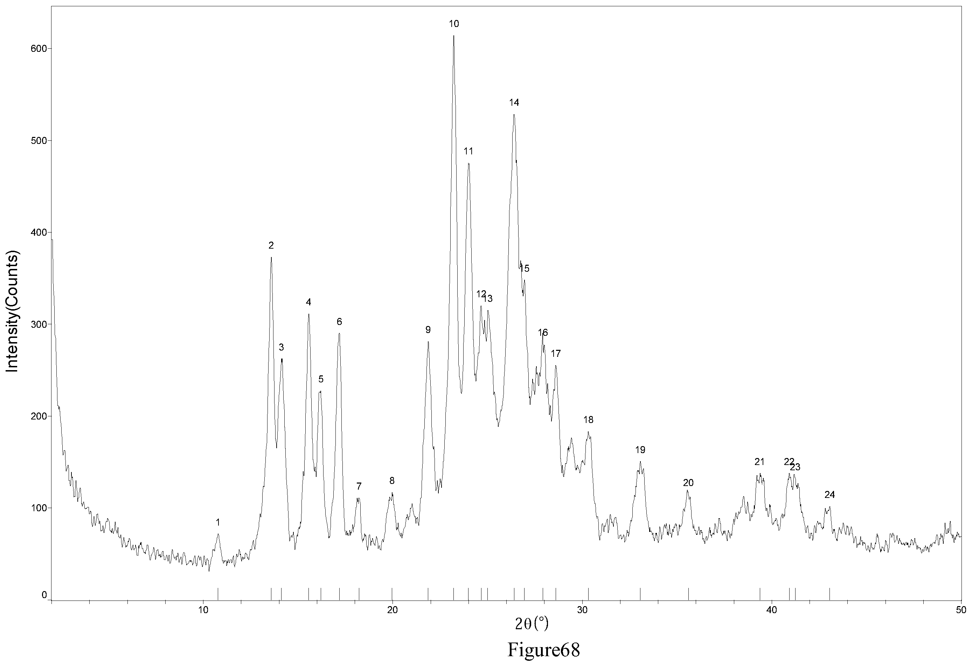

Particularly, the X-ray powder diffraction pattern of the crystal form I of fumarate salt of nepicastat is substantially as shown in Figure 68.

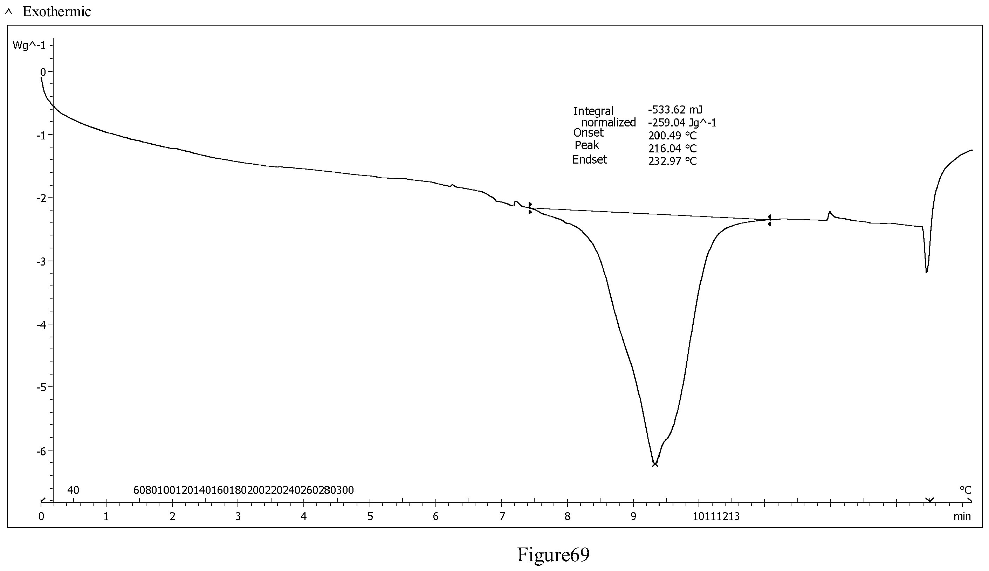

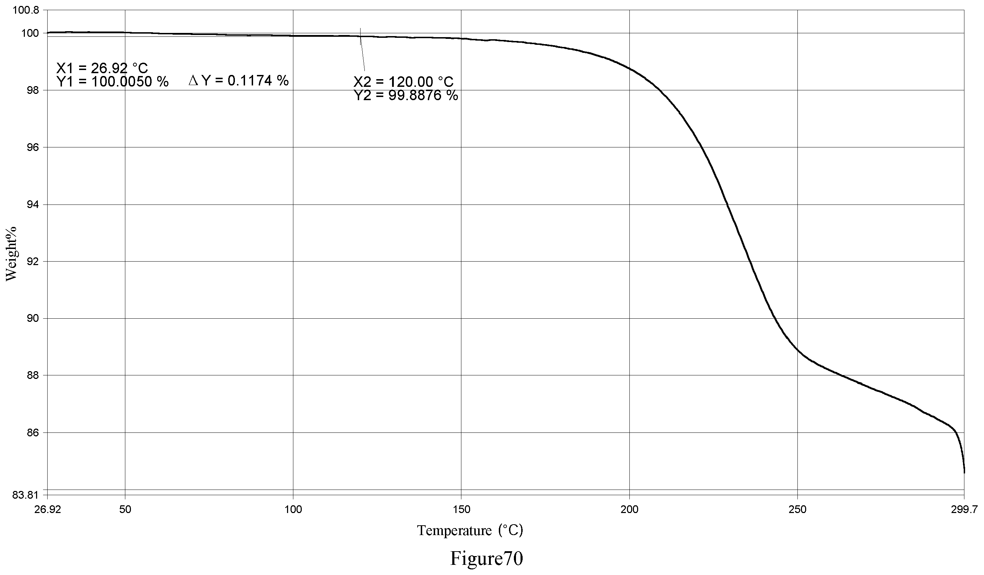

Advantageously, the DSC spectrum of the crystal form I of fumarate salt of nepicastat shows one endothermic peak at 216.0°C, and more advantageously, is substantially as shown in Figure 69; the TGA spectrum of the crystal form I of fumarate salt of nepicastat shows a weight loss of 0.12% from room temperature to 120°C, and more advantageously, is substantially as shown in Figure 70.

The present invention also provides a method for preparing the crystal form of hydrochloride salt of nepicastat, including:
1) suspension-trituration method
   mixing hydrochloride salt of nepicastat and an organic solvent under stirring, and centrifuging the resulting suspension to collect a solid, wherein the organic solvent is selected from one or more selected from C₁-C₄ alcohol, C₄-C₆ ether and C₂-C₆ nitrile;
   specifically, mixing hydrochloride salt of nepicastat and an organic solvent under stirring at a temperature ranging from room temperature to 50°C, preferably at 40°C, for 2-4 days, preferably for 3 days, and centrifuging the resulting suspension to collect a solid, optionally further drying the solid, wherein the organic solvent is one or more selected from ethanol, methyl tert-butyl ether, tetrahydrofuran and acetonitrile; advantageously, the ratio (W/V) of hydrochloride salt of nepicastat to the organic solvent is 50:1;
2) solvent evaporation method
   dissolving hydrochloride salt of nepicastat in a good solvent, evaporating the solvent naturally, and then collecting a solid, wherein the good solvent is one or more of C₁-C₄ alcohol and C₄-C₆ ether or a mixed solvent with water thereof, preferably one or more of methanol, 50% isopropanol and 50% tetrahydrofuran; the ratio (W/V) of hydrochloride salt of nepicastat to the good solvent is 50:1;
3) anti-solvent method
   dissolving hydrochloride salt of nepicastat in a good solvent, adding an anti-solvent, and then collecting a solid, wherein the good solvent is one or more of C₁-C₄ alcohol and C₄-C₆ ether or a mixed solvent with water thereof, preferably one or more of methanol, 50% isopropanol and 50% tetrahydrofuran; the anti-solvent is one or more selected from C₃-C₆ ester, C₄-C₆ ether, and C₃-C₆ ketone, preferably one or more of ethyl acetate, 2-methyltetrahydrofuran, methyl tert-butyl ether, 1,4-dioxane and 2-butanone; advantageously, the ratio (W/V) of hydrochloride salt of nepicastat to the good solvent is 50:0.5-2, such as 50:1.5, 50:2, or 50:0.5; and the volume ratio of the good solvent to the anti-solvent is 1:1 to 1:10, such as 1.5:4, 1.5:2, or 1:8;
4) mixing hydrochloride salt of nepicastat, C₁-C₄ alcohol and water, heating the resulting suspension to 45-55°C, stirring until dissolution, cooling down to 30-40°C, performing concentration to remove some solvent, cooling the residue down to 0-10°C, and collecting a solid after solid-liquid separation;
   specifically, mixing hydrochloride salt of nepicastat, C₁-C₄ alcohol and water, heating the resulting suspension to 45-55°C, stirring until dissolution, cooling down to 30-40°C, stirring for 1-3 hours, performing concentration to remove some solvent, cooling the residue down to 0-10°C, collecting a solid after solid-liquid separation, and optionally further drying the solid, wherein the C₁-C₄ alcohol is ethanol; and advantageously, the ratio (W/W) of hydrochloride salt of nepicastat to the C₁-C₄ alcohol is 10:1 to 15:1, such as 12:1; the volume ratio of the C₁-C₄ alcohol to water is 10:1 to 20:1, such as 16:1;
5) stirring (S)-N-[3-(5,7-difluoro-1,2,3,4-tetrahydronaphthalen-2-yl)-2-thioxo-2,3-dihydro-1H-imidazol-4-ylmethyl]formamide, C₁-C₄ alcohol and concentrated hydrochloric acid under reflux, cooling down to precipitate a solid, collecting the solid after solid-liquid separation, adding C₁-C₄ alcohol and water, heating until dissolution, performing concentration to remove some solvent, cooling the residue down to 0-10°C, and collecting a solid after solid-liquid separation;
   specifically, stirring (S)-N-[3-(5,7-difluoro-1,2,3,4-tetrahydronaphthalen-2-yl)-2-thioxo-2,3-dihydro-1H-imidazol-4-ylmethyl]formamide, C₁-C₄ alcohol and 36% concentrated hydrochloric acid under reflux for 5 hours, cooling down to 20-30°C, precipitating a solid, collecting the solid after solid-liquid separation, adding C₁-C₄ alcohol and water, heating to 65-70°C, stirring until dissolution, performing concentration to remove some solvent, cooling the residue down to 0-10°C, collecting a solid after solid-liquid separation, and optionally further drying the solid, wherein the C₁-C₄ alcohol is isopropanol or methanol.

The present invention also provides a method for preparing a crystal form of an acid addition salt of nepicastat, comprising:
1) mixing nepicastat with C₁-C₄ alcohol, preferably ethanol, under stirring, then adding an acid: if precipitating a large amount of solid, collecting the solid by centrifugation, or, if obtaining a sample as a clear solution or precipitating a small amount of solid, concentrating the solvent by nitrogen purging or natural evaporation, precipitating and collecting the solid; wherein the acid is selected from the group consisting of sulfuric acid, phosphoric acid, methanesulfonic acid, benzenesulfonic acid, p-toluenesulfonic acid, hydrobromic acid, maleic acid, tartaric acid, citric acid, and fumaric acid;
specifically, mixing nepicastat and C₁-C₄ alcohol under stirring at 40-60°C, preferably at 50 °C to prepare a suspension, then adding an acid, cooling down to room temperature and stirring overnight, wherein the ratio (W/V) of nepicastat to the C₁-C₄ alcohol is 50:1, and the molar ratio of nepicastat to the acid is 1:1.05 or 1:0.55;
or, 2) mixing nepicastat and C₃-C₆ ketone, preferably acetone, under stirring, then adding an acid: if precipitating a large amount of solid, collecting the solid by centrifugation, or, if obtaining a sample as a clear solution or precipitating a small amount of solid, concentrating the solvent by nitrogen purging or natural evaporation, then adding an anti-solvent, precipitating and collecting the solid; wherein the acid is selected from the group consisting of sulfuric acid, phosphoric acid, methanesulfonic acid, benzenesulfonic acid, p-toluenesulfonic acid, hydrobromic acid, maleic acid, tartaric acid, citric acid, and fumaric acid, and the anti-solvent is C₄-C₆ ether, preferably methyl tert-butyl ether;
specifically, mixing nepicastat and C₃-C₆ ketone under stirring at 40-60°C, preferably at 50 °C to prepare a solution, then adding an acid, cooling down to room temperature and stirring overnight, wherein the ratio (W/V) of nepicastat to the C₃-C₆ ketone is 50:1, the molar ratio of nepicastat to the acid is 1:1.05 or 1:0.55, and the volume ratio of the C₃-C₆ ketone to the anti-solvent is 1:1 to 1:10, such as 1:5.

The present invention also provides a pharmaceutical composition comprising a crystal form of an acid addition salt of nepicastat and an auxiliary material, wherein the auxiliary material is preferably a pharmaceutically acceptable carrier, diluent or excipient.

The present invention relates to a use of a crystal form of an acid addition salt of nepicastat or a pharmaceutical composition comprising the same in the preparation of a medicament for treating autoimmune disease, post-traumatic stress disorder, congestive heart failure, hypertension, cancer or sepsis.

The present invention relates to a crystal form of an acid addition salt of nepicastat or a pharmaceutical composition comprising the same, for use in the treatment of autoimmune disease, post-traumatic stress disorder, congestive heart failure, hypertension, cancer or sepsis.

The present invention also relates to a method of treating autoimmune disease, post-traumatic stress disorder, congestive heart failure, hypertension, cancer or sepsis, comprising administering an effective amount of a crystal form of an acid addition salt of nepicastat or a pharmaceutical composition comprising the same, to a patient in need thereof.

In some embodiments, the autoimmune disease is selected from the group consisting of autoimmune colitis, ophthalmoneuromyelitis, rheumatoid arthritis, scleroderma, psoriasis and uveitis, and further, the autoimmune colitis is Crohn's disease or ulcerative colitis.

In some embodiments, the cancer is selected from the group consisting of colon cancer, breast cancer, liver cancer, melanoma, lung cancer, prostate cancer, ovarian cancer, pancreatic cancer, cervical cancer, renal cell cancer, bladder cancer cancer, and gastric cancer.

The expression "pharmaceutically acceptable" as described herein is one which is useful for preparing a pharmaceutical composition that is generally safe, have neither biological toxicity nor other undesirable toxicity, and are acceptable for veterinary use and human pharmaceutical use.

The term "carrier" as described herein refers to a diluent, adjuvant or excipient administered together with the compound. The pharmaceutically acceptable carrier can be a liquid, for example water and oil, including petroleum, oils of animal, plant or synthetic origin, for example peanut oil, soybean oil, mineral oil, rapeseed oil, and the like. The pharmaceutically acceptable carrier can also be physiological saline, gum arabic, gelatin, starch paste, talc, keratin, silica gel, urea and the like. In addition, an aid, stabilizer, thickener, lubricant, colorant, and the like can also be used.

Those skilled in the art are able to understand that the pharmaceutical composition of the present invention can be formulated according to the specific administration route into various formulations well-known in the art, for example oral formulations (powder, tablet, capsule, soft capsule, liquid medicine, syrup, elixir, pulvis, sachet, granule), or formulations for topical administration (cream, ointment, lotion, gel, balm, plaster, paste, spray, aerosol, and the like), or formulations for injection (solution, suspension, emulsion). In the pharmaceutical compositions of the present invention, mention may notably be made of those suitable for oral, parenteral (intravenous or subcutaneous) or nasal administration, for example, tablet or dragee, sublingual tablet, gelatin capsule, lozenge, suppository, cream, ointment, skin gel, injection, drinkable suspension, and the like.

The pharmaceutical composition according to the present invention can comprise a pharmaceutically acceptable carrier, adjuvant or diluent, for example a filler, disintegrant, lubricant, suspending agent, binder, sweetener, flavoring agent, preservative, matrix, and the like. The filler is for example starch, pregelatinized starch, lactose, mannitol, chitin, microcrystalline cellulose, sucrose, and the like; the disintegrant is for example starch, pregelatinized starch, microcrystalline cellulose, sodium carboxymethyl starch, cross-linked polyvinylpyrrole, low-substituted hydroxypropyl cellulose, cross-linked sodium carboxymethyl cellulose, and the like; the lubricant is for example magnesium stearate, sodium lauryl sulfate, talc, silicon dioxide, and the like; the suspending agent is for example polyvinylpyrrolidone, microcrystalline cellulose, sucrose, agar, hydroxypropyl methylcellulose, and the like; the binder is for example starch slurry, polyvinylpyrrolidone, hydroxypropyl methylcellulose, and the like. The composition of the present invention can be prepared by any method known in the art, so as to provide rapid, sustained or slow release of the active ingredient after administration to a patient.

The pharmaceutical composition of the present invention is administered to an individual animal such as mammal (rat, mouse, domesticated animal or human) by various routes, all the administration routes are contemplated, and for example, the administration route can be oral, topical, rectal administration or intravenous, intramuscular, transdermal, intrathecal, epidural or intraventricular injection.

The administration dose of the active ingredient of the present invention can vary according to the condition and weight of the individual, the nature and severity of the disease, the form of drug, the administration route, and the administration period, and can also be selected by those skilled in the art. The dose can vary from 1 to 1500 mg/day, and the drug can be administered daily in a single dose or in divided doses.

### DESCRIPTION OF THE DRAWINGS

Figure 1 is the XRPD comparison patterns of the crystal forms I, II and III of hydrochloride salt of nepicastat. In the figure, from top to bottom, they are XRPD patterns of the crystal form III, the crystal form II, and the crystal form I.
Figure 2 is the XRPD pattern of the crystal form I of hydrochloride salt of nepicastat.
Figure 3 is the DSC spectrum of the crystal form I of hydrochloride salt of nepicastat.
Figure 4 is the TGA spectrum of the crystal form I of hydrochloride salt of nepicastat.
Figure 5 is the PLM spectrum of the crystal form I of hydrochloride salt of nepicastat.
Figure 6 is the DVS spectrum of the crystal form I of hydrochloride salt of nepicastat.
Figure 7 is the XRPD patterns of the crystal form I of hydrochloride salt of nepicastat before and after DVS. In the figure, from top to bottom, they are the XRPD pattern after DVS and the XRPD pattern before DVS.
Figure 8 is the XRPD pattern of the crystal form I of hydrochloride salt of nepicastat after heating to 170°C. In the figure, from top to bottom, they are the XRPD pattern of the crystal form I after heating to 170°C and the XRPD pattern of the initial crystal form I.
Figure 9 is the TGA spectrum of the crystal form I of hydrochloride salt of nepicastat after heating to 170°C.
Figure 10 is the XRPD pattern of the crystal form II of hydrochloride salt of nepicastat.
Figure 11 is the DSC spectrum of the crystal form II of hydrochloride salt of nepicastat.
Figure 12 is the TGA spectrum of the crystal form II of hydrochloride salt of nepicastat.
Figure 13 is the PLM spectrum of the crystal form II of hydrochloride salt of nepicastat.
Figure 14 is the DVS spectrum of the crystal form II of hydrochloride salt of nepicastat.
Figure 15 is the XRPD patterns of the crystal form II of hydrochloride salt of nepicastat before and after DVS. In the figure, from top to bottom, they are the XRPD pattern after DVS and the XRPD pattern before DVS.
Figure 16 is the XRPD pattern of the crystal form III of hydrochloride salt of nepicastat.
Figure 17 is the DSC spectrum of the crystal form III of hydrochloride salt of nepicastat.
Figure 18 is the TGA spectrum of the crystal form III of hydrochloride salt of nepicastat.
Figure 19 is the XRPD pattern of the crystal form IV of hydrochloride salt of nepicastat.
Figure 20 is the XRPD comparison patterns for the suspension-trituration method (40°C). In the figure, from top to bottom, they are the XRPD pattern of the product obtained by trituration with anhydrous ethanol, the XRPD pattern of the product obtained by trituration with acetonitrile, the XRPD pattern of the product obtained by trituration with methyl tert-butyl ether, the XRPD pattern of the product obtained by trituration with tetrahydrofuran, and the XRPD pattern of the initial crystal form I.
Figure 21 is the XRPD comparison patterns for the solvent evaporation method. In the figure, from top to bottom, they are the XRPD pattern of the product obtained by methanol evaporation and the XRPD pattern of the initial crystal form I.

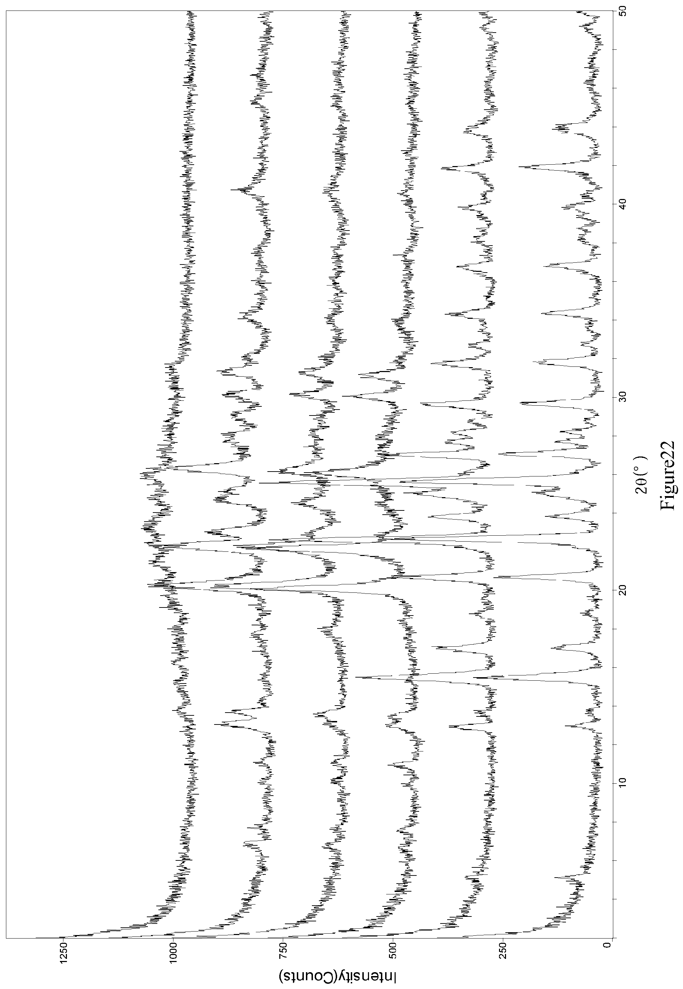

Figure 22 is the XRPD comparison patterns for the anti-solvent method. In the figure, from top to bottom, they are the XRPD pattern of the product obtained by using 50% isopropanol as the good solvent and 1,4-dioxane as the anti-solvent, the XRPD pattern of the product obtained by using methanol as the good solvent and methyl tert-butyl ether as the anti-solvent, the XRPD pattern of the product obtained by using methanol as the good solvent and 2-methyltetrahydrofuran as the anti-solvent, the XRPD pattern of the product obtained by using methanol as the good solvent and ethyl acetate as the anti-solvent, the XRPD pattern of the product obtained by using 50% tetrahydrofuran as the good solvent and 2-butanone as the anti-solvent, and the XRPD pattern of the initial crystal form I.

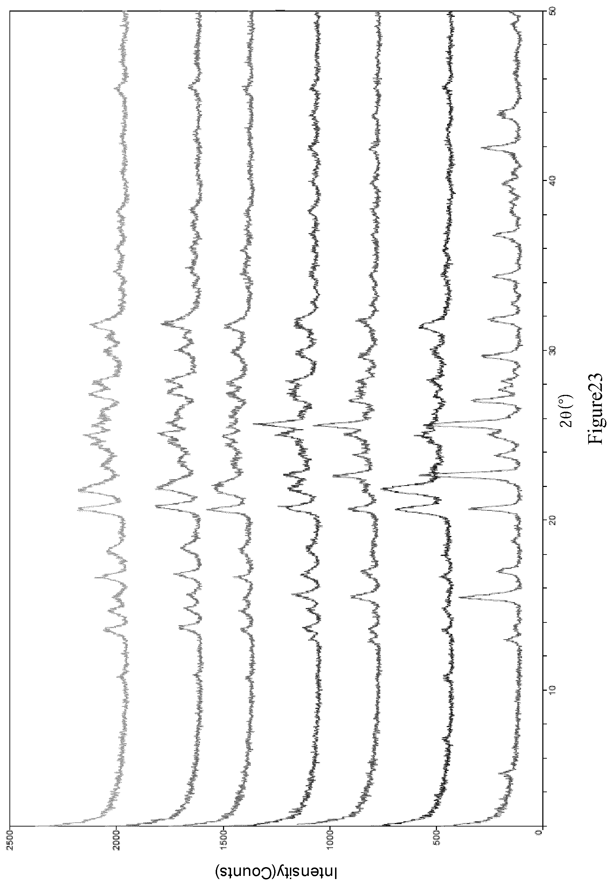

Figure 23 is the XRPD comparison patterns of the products after the suspensions of the crystal forms I and II of hydrochloride salt of nepicastat are subjected to competition for 3 days at room temperature. In the figure, from top to bottom, they are the XRPD pattern of the product in 95% ethanol solution, the XRPD pattern of the product in 90% ethanol solution, the XRPD pattern of the product in 65% ethanol solution, the XRPD pattern of the product in 15% ethanol solution, the XRPD pattern of the product in water, the XRPD pattern of the crystal form II and the XRPD pattern of the crystal form I.

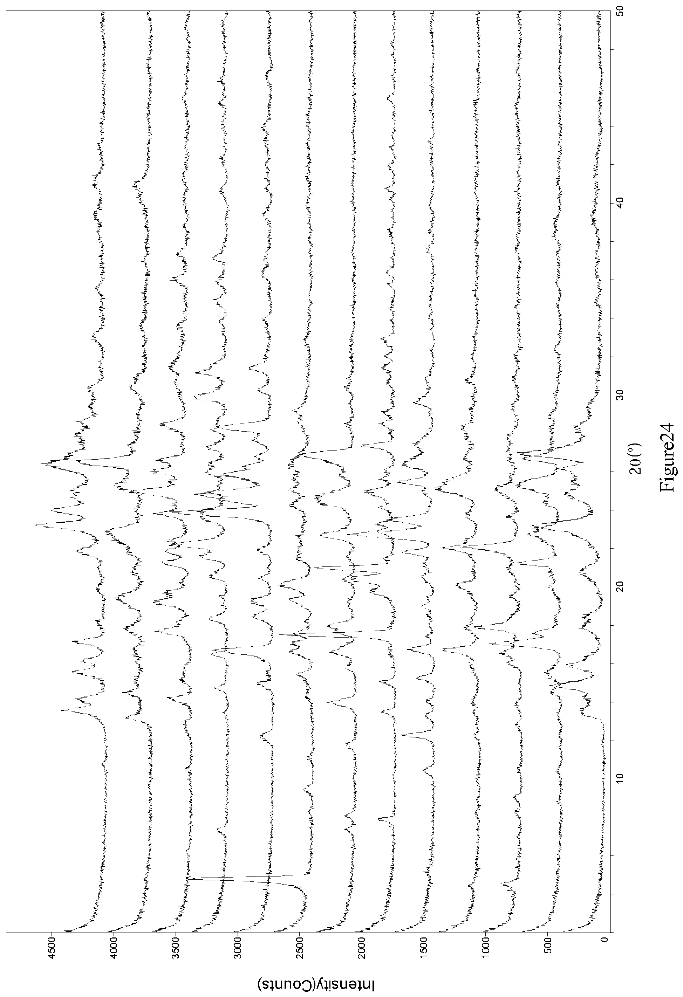

Figure 24 is the XRPD comparison patterns for the salt form screening of a free base of nepicastat in the ethanol system. In the figure, from top to bottom, they are the XRPD patterns of the crystal form of fumarate salt, the crystal form of citrate salt, the crystal form II of tartrate salt, the crystal form II of hydrobromide salt, the crystal form of maleate salt, the crystal form II of p-toluenesulfonate salt, the crystal form of benzenesulfonate salt, the crystal form of methanesulfonate salt, the crystal form of phosphate salt, the crystal form III of sulfate salt (1:1), the crystal form II of sulfate salt (1:0.5), blank ethanol, and the free base of nepicastat.

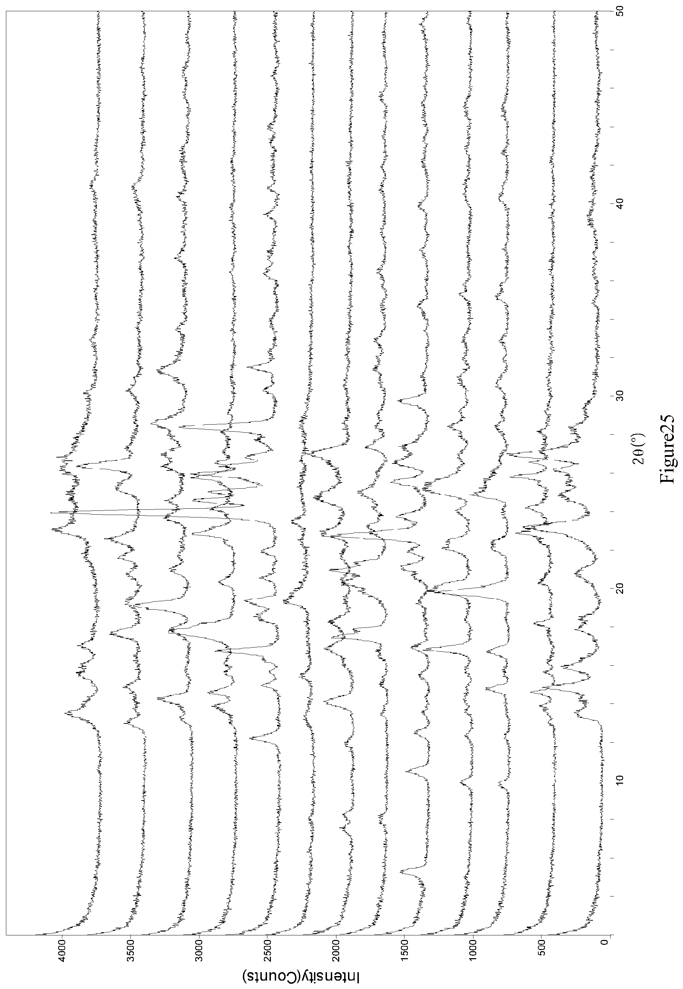

Figure 25 is the XRPD comparison patterns for the salt form screening of a free base of nepicastat in the acetone system. In the figure, from top to bottom, they are the XRPD patterns of the crystal form of fumarate salt, the crystal form of citrate salt, the crystal form I of tartrate salt, the crystal form I of hydrobromide salt, the crystal form of maleate salt, the crystal form I of p-toluenesulfonate salt, the crystal form of benzenesulfonate salt, the crystal form of methanesulfonate salt, the crystal form of phosphate salt, the crystal form III of sulfate salt (1:1), the crystal form I of sulfate salt (1:0.5), blank acetone, and the free base of nepicastat.
Figure 26 is the XRPD pattern of the crystal form I of sulfate salt (1:0.5) of nepicastat.
Figure 27 is the DSC spectrum of the crystal form I of sulfate salt (1:0.5) of nepicastat.
Figure 28 is the TGA spectrum of the crystal form I of sulfate salt (1:0.5) of nepicastat.
Figure 29 is the XRPD pattern of the crystal form II of sulfate salt (1:0.5) of nepicastat.
Figure 30 is the DSC spectrum of the crystal form II of sulfate salt (1:0.5) of nepicastat.
Figure 31 is the TGA spectrum of the crystal form II of sulfate salt (1:0.5) of nepicastat.
Figure 32 is the XRPD pattern of the crystal form III of sulfate salt (1:1) of nepicastat.
Figure 33 is the DSC spectrum of the crystal form III of sulfate salt (1:1) of nepicastat.
Figure 34 is the TGA spectrum of the crystal form III of sulfate salt (1:1) of nepicastat.
Figure 35 is the XRPD pattern of the crystal form I of phosphate salt of nepicastat.
Figure 36 is the DSC spectrum of the crystal form I of phosphate salt of nepicastat.
Figure 37 is the TGA spectrum of the crystal form I of phosphate salt of nepicastat.
Figure 38 is the XRPD pattern of the crystal form I of methanesulfonate salt of nepicastat.
Figure 39 is the DSC spectrum of the crystal form I of methanesulfonate salt of nepicastat.
Figure 40 is the TGA spectrum of the crystal form I of methanesulfonate salt of nepicastat.
Figure 41 is the XRPD pattern of the crystal form I of benzenesulfonate salt of nepicastat.
Figure 42 is the DSC spectrum of the crystal form I of benzenesulfonate salt of nepicastat.
Figure 43 is the TGA spectrum of the crystal form I of benzenesulfonate salt of nepicastat.
Figure 44 is the XRPD pattern of the crystal form I of p-toluenesulfonate salt of nepicastat.
Figure 45 is the DSC spectrum of the crystal form I of p-toluenesulfonate salt of nepicastat.
Figure 46 is the TGA spectrum of the crystal form I of p-toluenesulfonate salt of nepicastat.
Figure 47 is the XRPD pattern of the crystal form II of p-toluenesulfonate salt of nepicastat.
Figure 48 is the DSC spectrum of the crystal form II of p-toluenesulfonate salt of nepicastat.
Figure 49 is the TGA spectrum of the crystal form II of p-toluenesulfonate salt of nepicastat.
Figure 50 is the XRPD pattern of the crystal form I of hydrobromide salt of nepicastat.
Figure 51 is the DSC spectrum of the crystal form I of hydrobromide salt of nepicastat.
Figure 52 is the TGA spectrum of the crystal form I of hydrobromide salt of nepicastat.
Figure 53 is the XRPD pattern of the crystal form II of hydrobromide salt of nepicastat.
Figure 54 is the DSC spectrum of the crystal form II of hydrobromide salt of nepicastat.
Figure 55 is the TGA spectrum of the crystal form II of hydrobromide salt of nepicastat.
Figure 56 is the XRPD pattern of the crystal form I of maleate salt of nepicastat.
Figure 57 is the DSC spectrum of the crystal form I of maleate salt of nepicastat.
Figure 58 is the TGA spectrum of the crystal form I of maleate salt of nepicastat.
Figure 59 is the XRPD pattern of the crystal form I of tartrate salt of nepicastat.
Figure 60 is the DSC spectrum of the crystal form I of tartrate salt of nepicastat.
Figure 61 is the TGA spectrum of the crystal form I of tartrate salt of nepicastat.
Figure 62 is the XRPD pattern of the crystal form II of tartrate salt of nepicastat.
Figure 63 is the DSC spectrum of the crystal form II of tartrate salt of nepicastat.
Figure 64 is the TGA spectrum of the crystal form II of tartrate salt of nepicastat.
Figure 65 is the XRPD pattern of the crystal form I of citrate salt of nepicastat.
Figure 66 is the DSC spectrum of the crystal form I of citrate salt of nepicastat.
Figure 67 is the TGA spectrum of the crystal form I of citrate salt of nepicastat.
Figure 68 is the XRPD pattern of the crystal form I of fumarate salt of nepicastat.
Figure 69 is the DSC spectrum of the crystal form I of fumarate salt of nepicastat.
Figure 70 is the TGA spectrum of the crystal form I of fumarate salt of nepicastat.

### DETAILED DESCRIPTION OF THE INVENTION

The present invention will be set out in more detail through examples below. The examples of the present invention are merely intended to describe the technical solutions of the present invention, and should not be considered as limiting the spirit and scope of the present invention.

### X-ray powder diffraction (XRPD)

The instrument is XRD-6000 from Shimadzu, and the samples are scanned according to the following parameters:

| Setting | Parameters |
|---|---|
| Ray source | Cu~Kα target (1.54056 Å) heating up |
| Minimum operating voltage and current of light tube | 40 kV and 30 mA |
| 2-Theta value | 2° to 50°; scanning speed of 5°/min |
| Thermogravimetric analysis (TGA) | |

The instrument is Pyris1 TGA from PerkinElmer. About 5 mg of the sample is weighed and placed into a crucible, and under nitrogen protection, the temperature is raised from 30°C to 350°C at a heating rate of 20°C/min, and is kept at 350°C for 1 minute.

### Differential scanning calorimetry (DSC)

The instrument is DSC3 from Mettler. About 0.5-5 mg of the powder sample is weighed and placed into a closed aluminum crucible, with a pinhole pierced on the crucible cover. Under nitrogen protection, the scanning for differential calorimetry is carried out by raising the temperature from 30°C to 300°C and being kept at 300°C for 1 minute. The heating rate is 20°C/min.

| Setting | Parameters |
|---|---|
| Heating rate | from 30°C to 300°C, at 20°C/min |
| Nitrogen purging | 50 mL/min |
| Polarized light microscope (PLM) | |

The instrument is XPV-203E from Shanghai Changfang Optical Instrument Co., Ltd. The sample is dispersed in a suitable medium and observed using an eyepiece and objective lens. Images are recorded using a camera computer system.

### Dynamic vapor sorption (DVS)

DVS curves are acquired on DVS Intrinsic from SMS (Surface Measurement Systems).
Testing temperature: 25°C
Sample amount: 10-20 mg
dm/dt: 0.002%/min;
RH range: 0%RH-95%RH-0%RH
RH gradient: 10%

### Example 1: Crystal forms of hydrochloride salt of nepicastat

The inventors have discovered four polymorphs of hydrochloride salt of nepicastat, named as crystal form I, crystal form II, crystal form III and crystal form IV respectively. The XRPD comparison patterns of the crystal form I, the crystal form II, the crystal form III and the crystal form IV are shown in Figure 1.

### 1.1 Preparation method of the crystal form I:

Ethanol (59.4 kg), purified water (88.00 kg), and anhydrous (S)-5-aminomethyl-1-(5,7-difluoro-1,2,3,4-tetrahydronaphthalen-2-yl)-1,3-dihydroimidazole-2-thione hydrochloride (5.00 kg, prepared according to Example 31 in CN1113874C) were added to a reaction kettle. The resulting suspension was heated to 45-55°C, stirred until dissolution, cooled down to 30-40°C and stirred for 1-3 hours. The resulting mixture was concentrated under vacuum to remove some solvent, until a residual of about 14.0-16.0V (based on the weight of anhydrous API, as 1V) was obtained. The residue was cooled down to 0-10°C and centrifuged to obtain a wet product. The wet product was dried under vacuum at 40-50°C to obtain 4.63 kg of (S)-5-aminomethyl-1-(5,7-difluoro-1,2,3,4-tetrahydronaphthalen-2-yl)-1,3-dihydroimidazole-2-thione hydrochloride monohydrate, with a yield of 52.4%, HPLC purity of 99.8%, chiral purity of 99.8%, as the crystal form I. The XRPD thereof is shown in Figure 2.

¹H NMR (ppm): δ12.35 (1 H, S), δ8.65 (3 H, S), δ7.09 (1 H, s), δ7.02 (1 H, td, J=9.68, 2.00Hz), δ6.90 (1 H, d, J=9.32 Hz), δ4.65 (1 H, br s), δ4.11 (3 H, s), δ2.96 (3 H, m), δ2.77 (1 H, M), δ2.04 (1 H, Brs).

### 1.2 Approximate solubility test

The approximate solubility test was performed on the crystal form I at room temperature. The test method was as follows: about 4 mg of the crystal form I was weighed, an appropriate volume of a solvent was added gradually thereto and shaken to observe whether the crystal form I was dissolved, and the volumes of the solvent were recorded, including the volume that enabled complete dissolution and the previous volume of the solvent that was not able to completely dissolve the crystal form I. For poor solubility, the total amount of the solvent added was no more than 4-5 mL. The results of approximate solubility are shown in Table 20.

**Table 20 Approximate solubility of the crystal form I of hydrochloride salt of nepicastat**

| Solvent | Approximate solubility (mg/mL) | Solvent | Approximate solubility (mg/mL) |
|---|---|---|---|
| 2-Butanone | <1 | 50% Isopropanol | 40~80 |
| Acetone | <1 | 30% Methanol | 21~42 |
| Methyl tert-butyl ether | <1 | 50% Ethanol | 20~27 |
| 1,4-Dioxane | <1 | 50% Acetonitrile | 80~100 |
| 15% Isopropanol | 20~27 | 50% Tetrahydrofuran | 100~130 |
| 2-Methyltetrahydrofuran | <1 | / | |
| N-hexane | <0.01 | Propanol | 11.7 |
| Ethanol | 6.5 | Isopropanol | 6.0 |
| Methanol | 33 | Propylene carbonate | 0.14 |
| Tetrahydrofuran | 0.09 | Acetonitrile | 0.04 |
| Chloroform | 0.38 | Dichloromethane | <0.01 |
| Ethyl acetate | 0.01 | Purified water | 7.4 |

### 1.3 Suspension-trituration method (40°C)

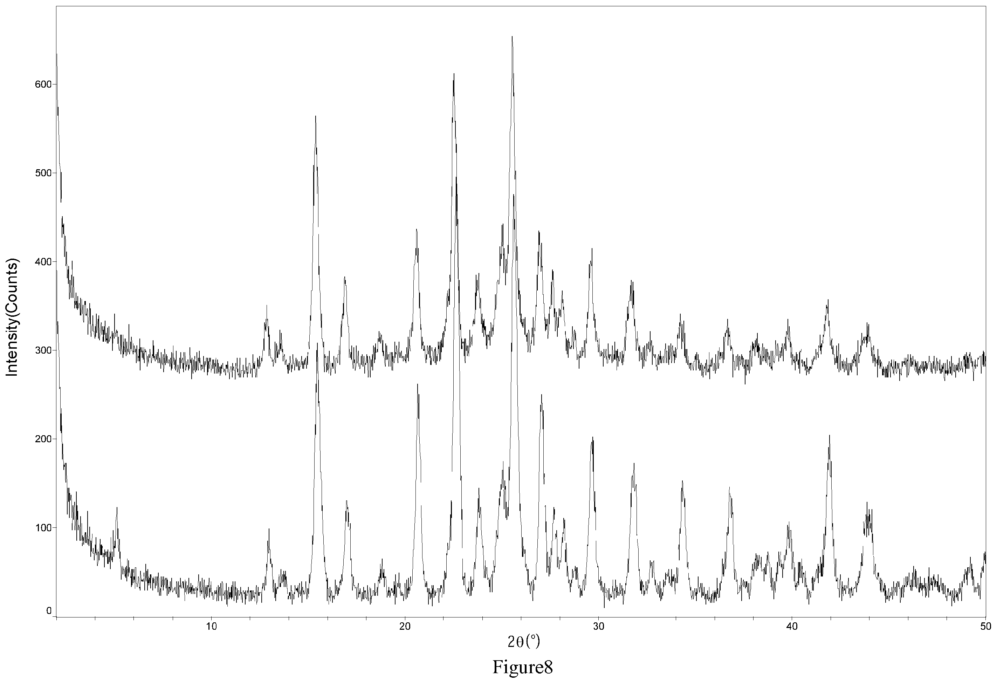

The starting material was the crystal form I of hydrochloride salt of nepicastat, which appeared as an off-white powder. The characterization results of XRPD, DSC, TGA, PLM, and DVS thereof and the results of the heating experiment are shown in Figures 2 to 9.

About 50 mg of the crystal form I of hydrochloride salt of nepicastat was weighed and placed into a liquid-phase vial, and then 1 mL of a corresponding solvent was added thereto. The sample was stirred for 3 days at 40°C, and the resulting suspension was centrifuged to collect a wet solid (12000 rpm, 10 min). The collected solid was further subjected to vacuum drying under reduced pressure in a vacuum drying oven for about 4 hours at 40°C. Then, the XRPD of the dried solid was determined. If the determined XRPD was different from the initial crystal form I, further studies were performed through DSC and TGA to confirm the new crystal form.

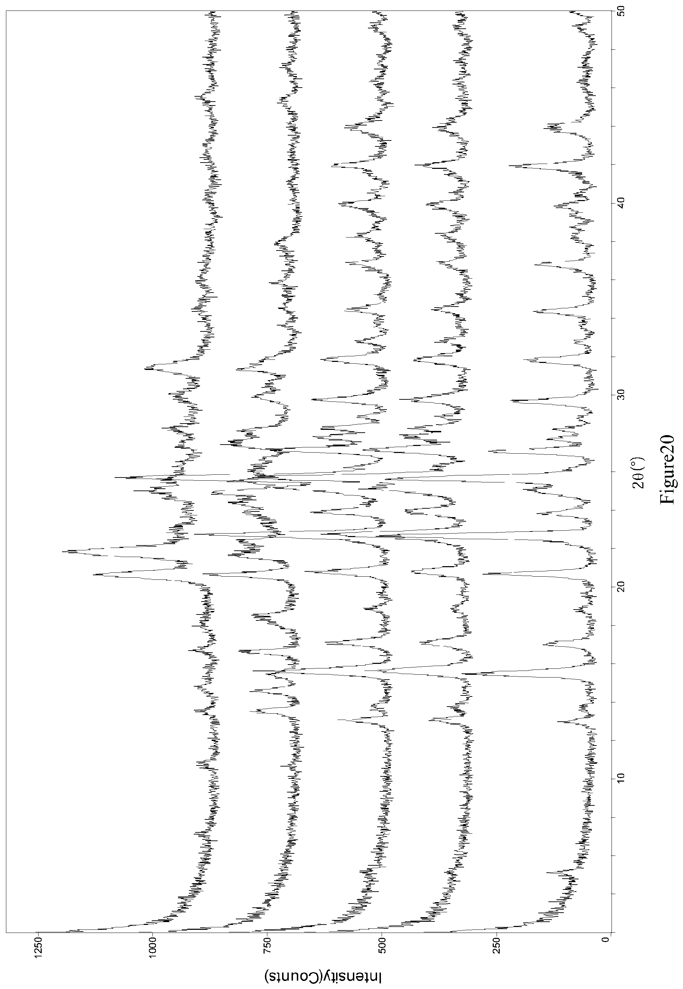

The experimental results are summarized in Table 21 and Figure 20. At 40°C, the obtained sample is crystal form I after triturating the crystal form I of hydrochloride salt of nepicastat in the methyl tert-butyl ether system and tetrahydrofuran system for 3 days, and the obtained sample is crystal form II after triturating the crystal form I of hydrochloride salt of nepicastat in the anhydrous ethanol system or acetonitrile system for 3 days.

**Table 21 Crystal form screening of the crystal form I of hydrochloride salt of nepicastat through the suspension-trituration method (40°C)**

| No. | Solvent | Weight (mg) | Volume (mL) | Temperature (°C) | Time | Experimental phenomenon (After stirring for 3 days) | XRPD |
|---|---|---|---|---|---|---|---|
| 1 | Anhydrous ethanol | 50.07 | 1 | 40 | 3 days | Being an off-white suspension, and after drying, being an off-white powder. | Crystal form II |
| 2 | Methyl tert-butyl ether | 50.47 | 1 | 40 | 3 days | Being an off-white suspension, and after drying, being an off-white powder. | Crystal form I |
| 3 | Tetrahydrofuran | 49.83 | 1 | 40 | 3 days | Being an off-white suspension, and after drying, being an off-white powder. | Crystal form I |
| 4 | Acetonitrile | 50.04 | 1 | 40 | 3 days | Being an off-white suspension, and after drying, being an off-white powder. | Crystal form II |

### 1.4 Solvent evaporation method

About 50 mg of the crystal form I of hydrochloride salt of nepicastat was weighed and placed into a glass bottle, and a good solvent was added to completely dissolve the compound. After filtration using a 0.45 µm nylon filter membrane, the sample bottle was capped with an aluminum foil paper having a small hole, and placed in a fume hood. After natural evaporation of the solvent for about 1-3 weeks, a solid was collected and the XRPD thereof was determined. If the determined XRPD was different from the initial crystal form I, further studies were performed through DSC and TGA to confirm the new crystal form.

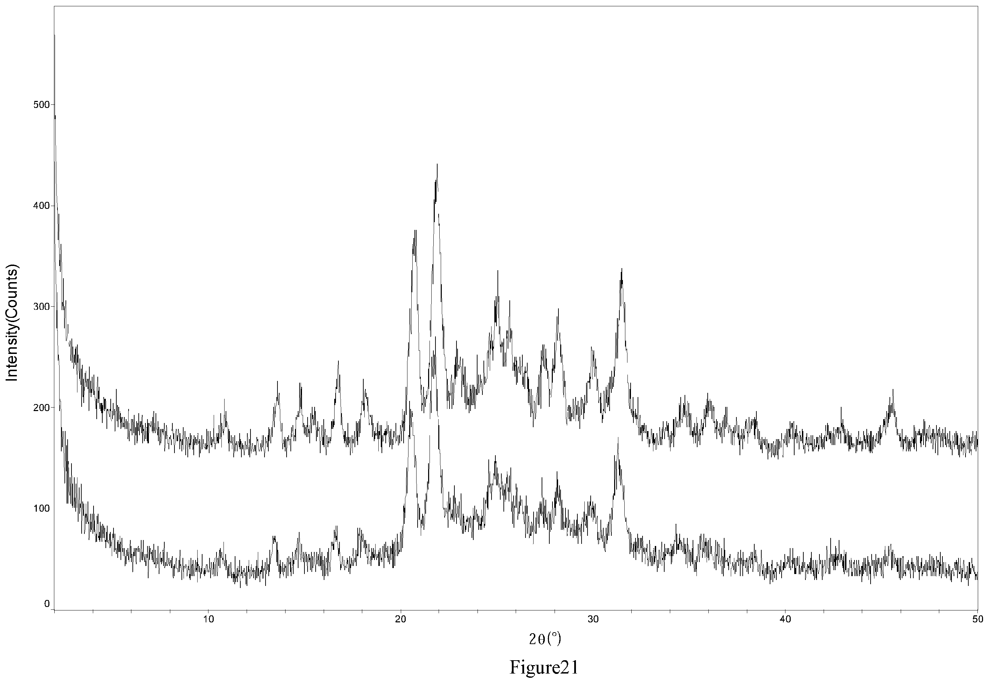

The specific phenomenon, processing procedure, and results are shown in Table 22 and Figure 21. The obtained sample is crystal form II through evaporation in the methanol system.

**Table 22 Crystal form screening of the crystal form I of hydrochloride salt of nepicastat through the solvent evaporation method**

| No. | Solvent | Weight (mg) | Volume (mL) | Temperature (°C) | Time | Experimental phenomenon | XRPD |
|---|---|---|---|---|---|---|---|
| 1 | Methanol | 50.00 | 1.5 | Room temperature | 4 days | Off-white solid | Crystal form II |

### 1.5 Anti-solvent method

About 50 mg of the crystal form I of hydrochloride salt of nepicastat was weighed and placed into a glass vial, and an appropriate volume of a good solvent (such as methanol) was added to completely dissolve it, forming a nearly saturated solution. After filtration using a 0.45 µm nylon filter membrane, different types of anti-solvent were added dropwise to the filtrate, until a large amount of solid was precipitated, wherein the anti-solvent was added at a volume at most equal to 10 times the volume of the good solvent. For the samples with precipitated solid, the centrifugation at 12000 rpm was performed for 10 minutes to collect a wet solid; and for the samples without precipitated solid, they were placed in a fume hood, and a solid was collected by slowly evaporating the solvent. The collected solid was subjected to vacuum drying under reduced pressure at 40°C for about 4 hours, and the XRPD of the dried solid was determined.

The experimental results are summarized in Table 23 and Figure 22. With the anti-solvent method for the crystal form I of hydrochloride salt of nepicastat, there is no significant difference in the XRPD patterns between the sample obtained in the 50% tetrahydrofuran/2-butanone system and the starting material, indicating that the sample is crystal form I; the obtained samples in the methanol/ethyl acetate system, methanol/2-methyltetrahydrofuran system, and methanol/methyl tert-butyl ether system are crystal form III; and the crystallinity of the sample obtained in the 50% isopropanol/1,4-dioxane system is relatively low.

**Table 23 Crystal form screening of the crystal form I of hydrochloride salt of nepicastat through the anti-solvent method**

| No. | Good solvent | Sample amount (mg) | Good solvent Volume (mL) | Anti-solvent | Anti-solvent Volume (mL) | Experimental phenomenon and post-processing | XRPD |
|---|---|---|---|---|---|---|---|
| 1 | Methano 1 | 50.53 | 1.5 | Ethyl acetate | 4.0 | 1. Precipitating a solid after the addition of the anti-solvent, and stirring overnight, followed by centrifuging to separate the wet solid. | Crystal form III |
| | | | | | | 2. Obtaining an off-white solid after vacuum drying under reduced pressure at 40°C for 4 hours. | |
| 2 | Methano 1 | 50.02 | 1.5 | 2-Methyltet rahydrofu ran | 2.0 | 1. Precipitating a solid after the addition of the anti-solvent, and stirring overnight, followed by centrifuging to separate the wet solid. | Crystal form III |
| | | | | | | 2. Obtaining an off-white solid after vacuum drying under reduced pressure at 40°C for 4 hours. | |
| 3 | Methano 1 | 50.26 | 1.5 | Methyl tert-butyl ether | 2.0 | 1. Precipitating a solid after the addition of the anti-solvent, and stirring overnight, followed by centrifuging to separate the wet solid. | Crystal form III |
| | | | | | | 2. Obtaining an off-white solid after vacuum drying under reduced pressure at 40°C for 4 hours. | |
| 4 | 50% Isopropa nol | 50.54 | 2.0 | 1,4-Dioxane | 16.0 | 1. Precipitating a solid after the addition of the anti-solvent, and stirring overnight, followed by centrifuging to separate the wet solid. | Low crystalli ne |
| | | | | | | 2. Obtaining a light yellow solid after vacuum drying under reduced pressure at 40°C for 4 hours. | |
| 5 | 50% Tetrahyd rofuran | 50.48 | 0.5 | 2-Butanone | 4.0 | 1. Precipitating a solid after the addition of the anti-solvent, and stirring overnight, followed by centrifuging to separate the wet solid. | Crystal form I |
| | | | | | | 2. Obtaining an off-white solid after vacuum drying under reduced pressure at 40°C for 4 hours. | |

### 1.6 Preparation method of the crystal form IV:

Isopropanol (194.0 L), (S)-N-[3-(5,7-difluoro-1,2,3,4-tetrahydronaphthalen-2-yl)-2-thioxo-2,3-dihydro-1H-imidazol-4-ylmethyl]formamide (7.80 kg, DBH-7, prepared according to Example 30 in CN1113874C), and concentrated hydrochloric acid (10.2 kg) with a mass concentration of about 36% were added to a reaction kettle. After stirring for 5 hours under reflux, the reaction mixture was cooled down to 20-30°C to precipitate a solid, filtered, and the filter cake was washed with isopropanol (15.6 L) to obtain a wet product. Methanol (293.8 L) and water (22.5 L) were added. The reaction mixture was heated to 65-70°C under stirring until dissolution, and then concentrated under vacuum to about 18.0-20.0 V (based on the weight of the wet product as 1 V). Methanol (220.2 kg/time) was added thereto and azeotroped twice to remove water. The residue (about 7.0-10.0 v) was cooled down to 0-10°C, stirred for another 5-7 hours, filtered, and rinsed with 14.7 L of methanol. The resulting product was dried under vacuum at 40-50°C for 14 hours to obtain (S)-5-aminomethyl-1-(5,7-difluoro-1,2,3,4-tetrahydronaphthalen-2-yl)-1,3-dihydroimidazole-2-thione hydrochloride (5.06 kg, with a yield of 65.0%, HPLC purity of 99.5%), which was determined as crystal form IV by XRPD (Figure 19).

### 1.7 Crystal form I of hydrochloride salt of nepicastat

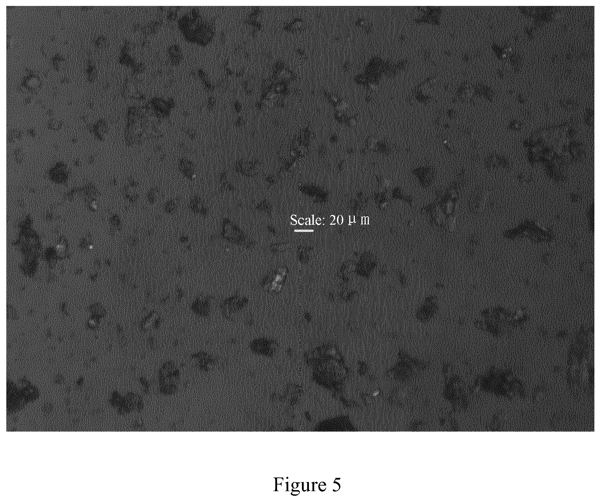

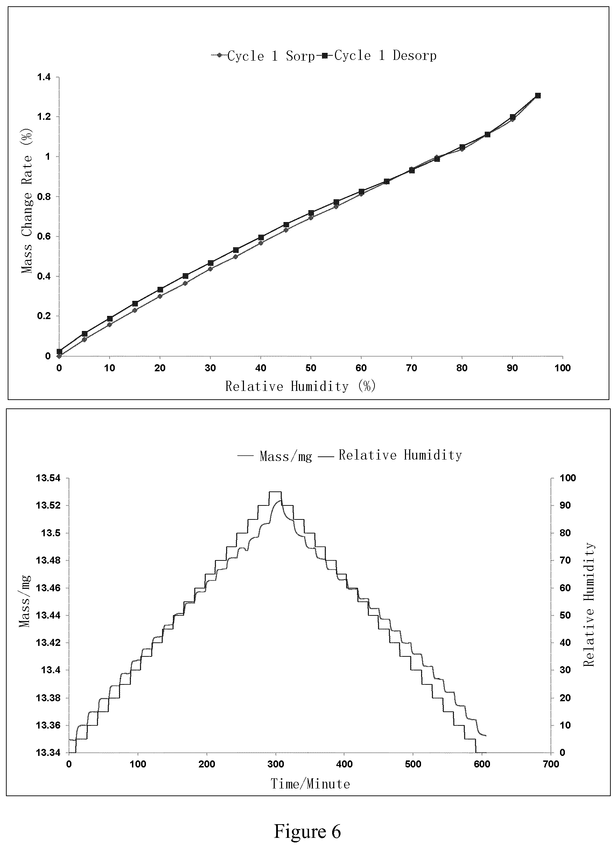

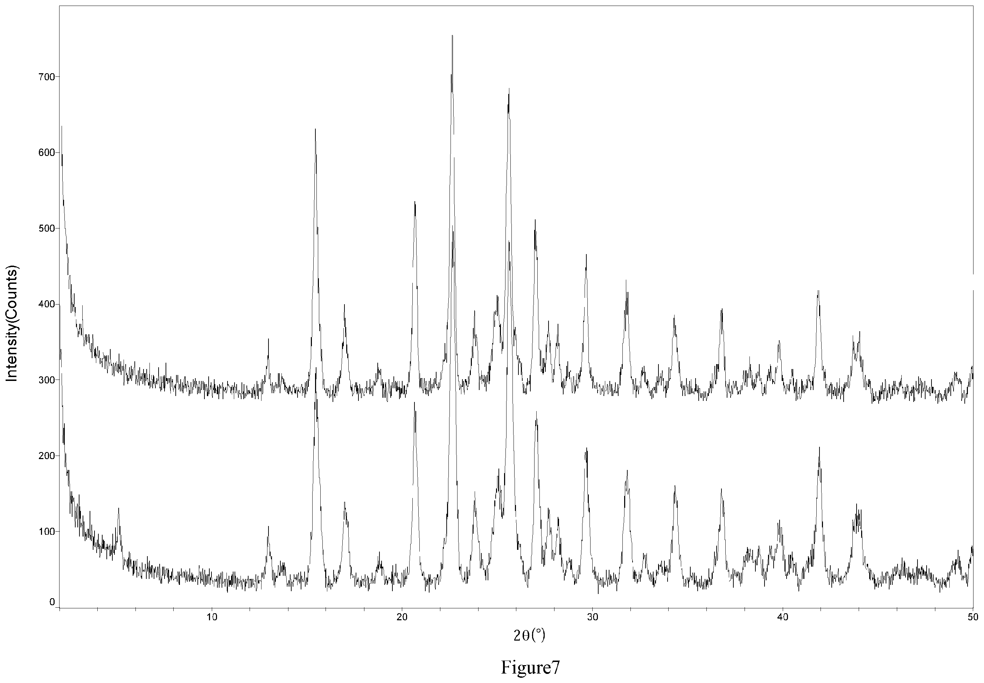

The sample of the crystal form I appeared as an off-white powder. The XRPD result showed that the crystal form I had clear sharp diffraction peaks (Figure 2), and was a crystalline compound. The DSC result (Figure 3) showed four clear endothermic peaks (100.3°C, 198.4°C, 227.8°C, and 288.3°C) and one exothermic peak (208.64°C). The TGA result (Figure 4) showed a weight loss of 5.15% from 30°C to 160°C. The PLM result (Figure 5) showed a birefringence phenomenon under the polarized light microscopy, and an irregular granular crystal habit. The DVS result showed that the weight gain by hygroscopicity of the sample was about 1.036% from 0% RH to 80% RH, indicating slight hygroscopicity of the sample (Figure 6). The crystal form of the sample remained unchanged before and after the DVS test (Figure 7).

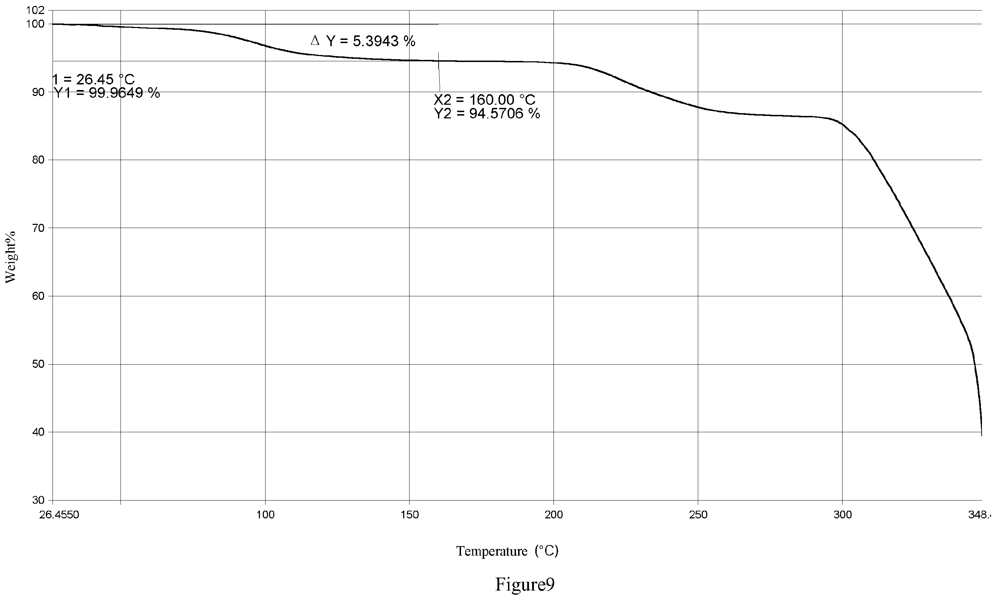

Through the heating experiment (the sample was heated to 170°C by TGA, exposed to air and cooled down to room temperature, the XRPD was determined, and then the sample was heated again to 350°C, it was found that there was the same weight loss of about 5% before 160°C during the second heating), the crystal form I of hydrochloride salt of nepicastat was determined to be a hydrate, and this compound was a monohydrate according to the calculation of the molecular weight (Mw=349.8) of the crystal form I of hydrochloride salt of nepicastat (see Figures 1 and 9).

Stability experiments were conducted on the crystal form I, with the results as shown in Tables 24 and 25 (inner packaging material: double-layer polyethylene bag). The results showed that, under accelerated conditions (40°C/75% RH) and long-term conditions (25°C/60% RH), there was no significant change in the detection results, no new impurities were produced, and the stability was good.

In addition, stability studies were conducted under relatively harsh conditions for the crystal form I (60°C, 60°C/75%RH) and the crystal form II (40°C, 40°C/75%RH, 60°C, 60°C/75%RH), and the results showed that:
After the solid crystal form I was left under 60°C and 60°C/75% RH conditions for 15 days, it had no significant impurity to produce due to degradation, and the crystal form I was more stable than the crystal form II.
After being left at 60°C and 60°C/75% RH for 15 days, the crystal form I was stable (as determined by the XRPD pattern).
Under 40°C and 60°C conditions, the crystal form II was converted into the crystal form III (as determined by the XRPD pattern). However, after being left under 40°C/75% RH and 60°C/75% RH conditions for 15 days, the crystal form did not change.

The crystal form I is more stable than the crystal form II, based on the above chemical and crystal stability studies on the two crystal forms of hydrochloride salt.

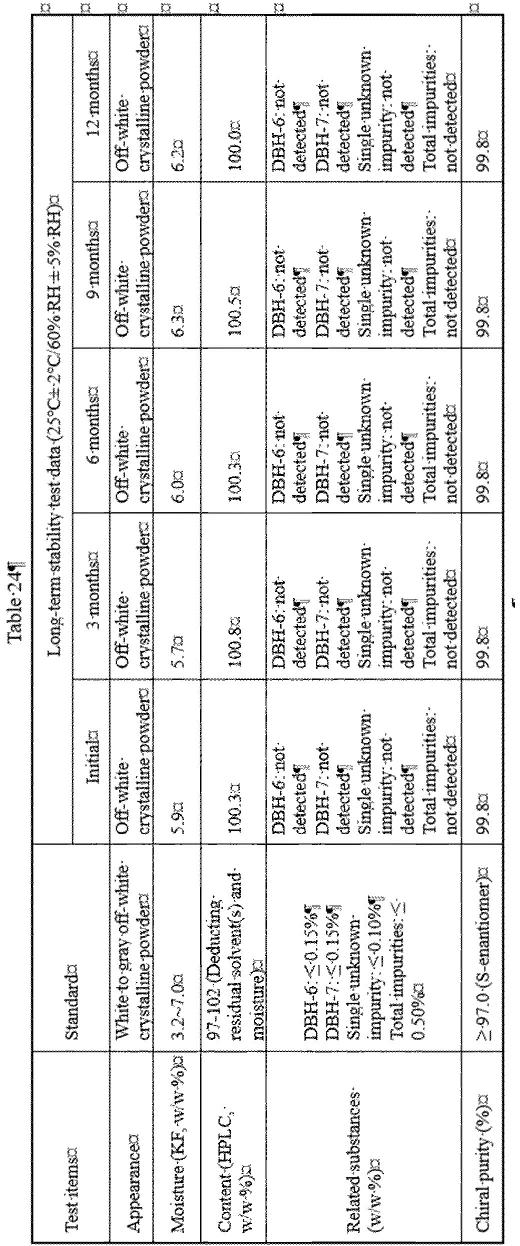

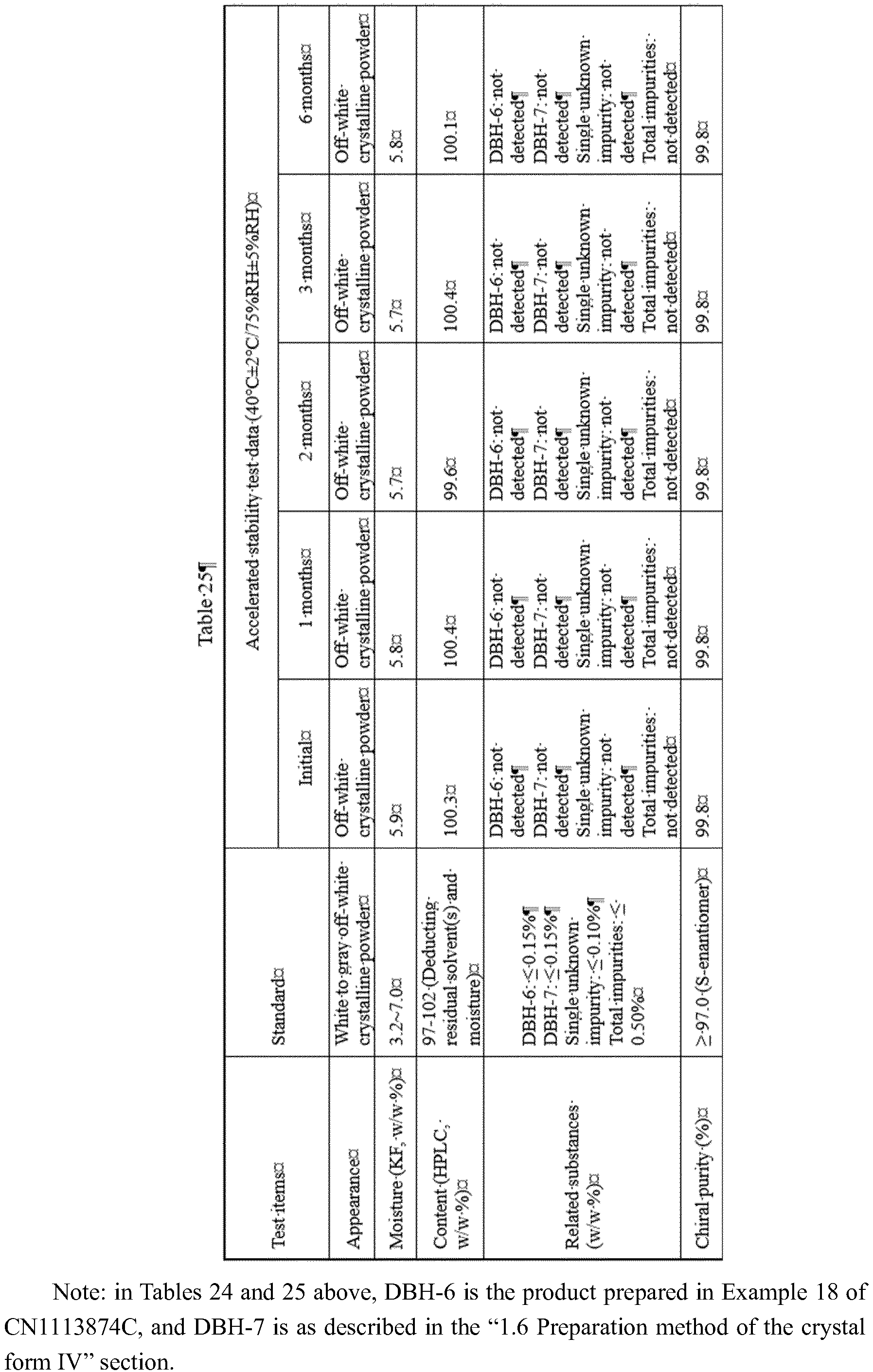

### 1.8 Crystal form II of hydrochloride salt of nepicastat

The crystal form II can be obtained by triturating a suspension of the crystal form I in anhydrous ethanol or acetonitrile at 40°C for 3 days or by evaporation in methanol, and the specific preparation methods thereof are shown in Tables 20 and 21. For the sample of the crystal form II, the characterization results of XRPD, DSC, TGA, PLM, and DVS thereof are shown in Figures 10 to 15.

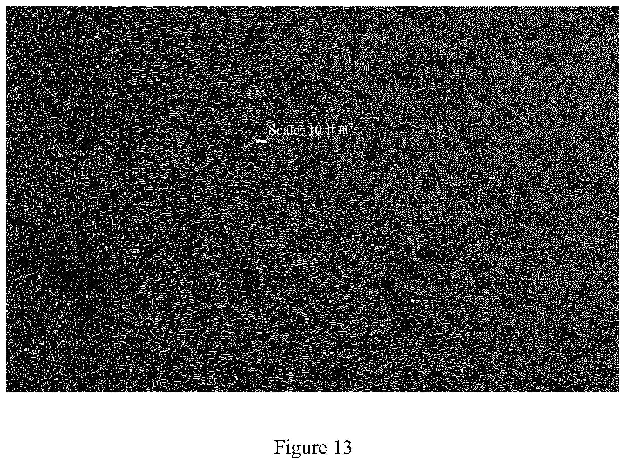

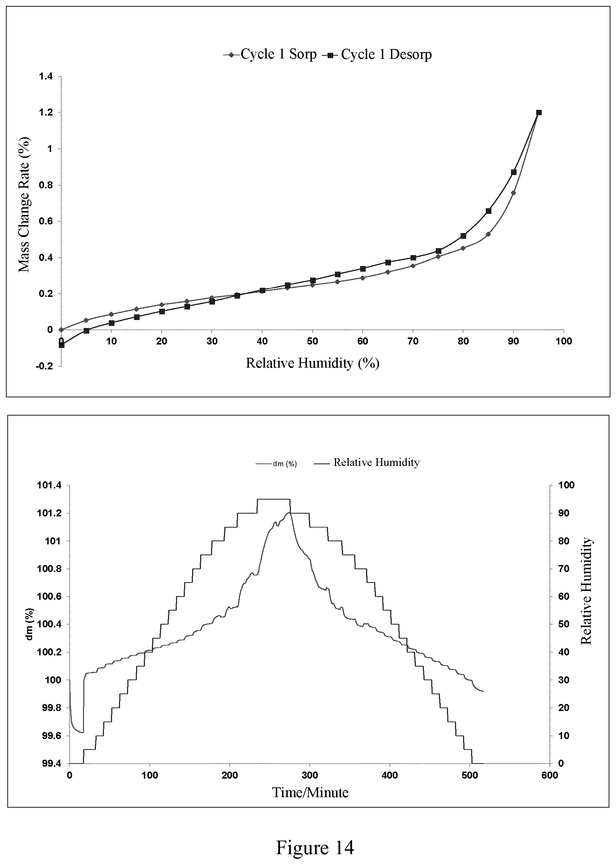

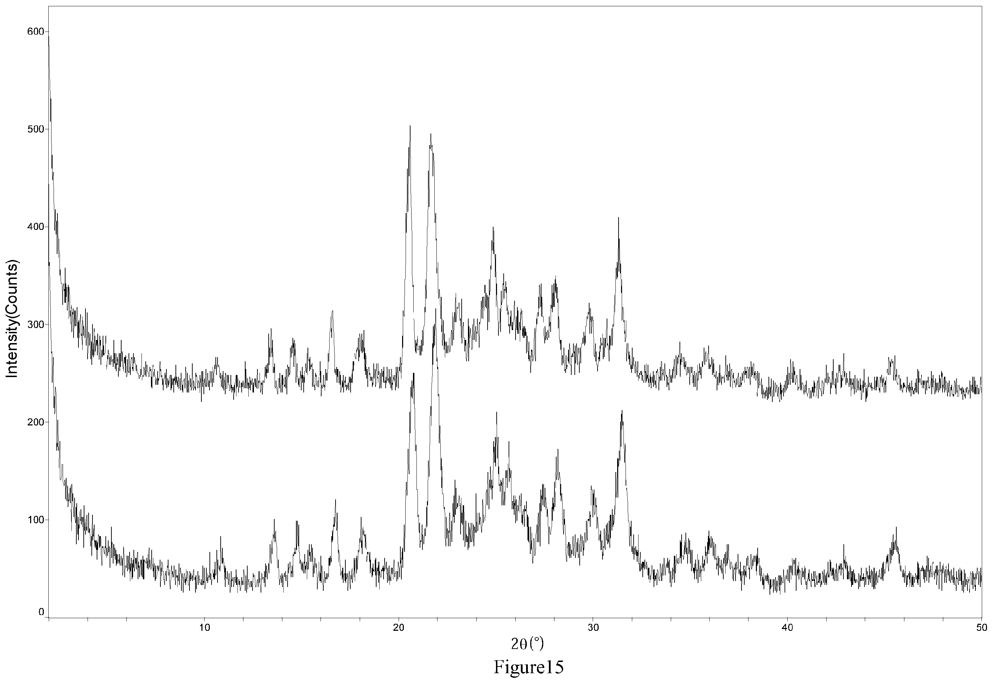

The sample of the crystal form II appeared as an off-white powder. The determination result by X-ray powder diffraction indicated that the sample had multiple clear characteristic diffraction peaks (Figure 10) and was crystalline. The thermal analysis (DSC, Figure 11, and TGA, Figure 12) showed that the sample of the crystal form II had an initial melting point of 247.22°C, melting thereof was accompanied by degradation, and the weight loss thereof from 25.63°C to 120°C was 0.4048%. The PLM result (Figure 13) showed a granular crystal habit. The DVS result (Figure 14) showed that the weight gain by hygroscopicity of the sample was about 0.453% from 0% RH to 80% RH, indicating slight hygroscopicity of the sample. The crystal form of the sample remained unchanged before and after the DVS test (Figure 15).

In addition, the approximate solubility of crystal form II in water was determined to be 7.9 to 10.5 mg/mL, which was similar to the solubility of the crystal form I in water.

### 1.9 Crystal form III of hydrochloride salt of nepicastat

The crystal form III can be obtained by adding an anti-solvent (ethyl acetate, 2-methyltetrahydrofuran, or methyl tert-butyl ether) to the crystal form I in methanol, and the specific preparation methods thereof are shown in Table 22. For the sample of the crystal form III, the characterization results of XRPD, DSC and TGA thereof are shown in Figures 16 to 19.

The sample of the crystal form III appeared as an off-white powder. The determination result by X-ray powder diffraction indicated that the sample had multiple clear characteristic diffraction peaks (Figure 16) and was crystalline. The thermal analysis (DSC, Figure 13, and TGA, Figure 18) showed that the sample of the crystal form III had an initial melting point of 235.39°C, melting thereof was accompanied by degradation, and the weight loss thereof from 30.35°C to 120°C was 2.1661%.

### 1.10 Stable crystal form investigation - competitive experiments by adding crystal seeds

To confirm stability of the crystal forms at room temperature, the following competitive experiments were conducted for the crystal forms I and II:
5 portions of the crystal form II were taken, purified water, 15% ethanol solution, 65% ethanol solution, 90% ethanol solution and 95% ethanol solution were added thereto respectively, and sonicated for 1 minute to obtain 5 suspensions containing the crystal form II. The suspensions were stirred for 24 hours at room temperature, and all systems had solid residues. All systems were centrifuged and the supernatants were taken. Then, the crystal form I (20 mg) and crystal form II (20 mg) were added to each supernatant to obtain a suspension containing the crystal forms I and II. The resulting suspensions were sonicated for 1 minute, stirred for 3 days at room temperature, and sampled to detect the crystal form.

The results (Table 26 and Figure 23) showed that: the solid crystal form obtained after competition for 3 days in purified water at room temperature was converted to crystal form I; the solid crystal form obtained after competition for 3 days in the 15% ethanol solution at room temperature was a mixed crystal of crystal form I and crystal form II; the solids obtained after competition for 3 days in the 65% ethanol solution, 90% ethanol solution and 95% ethanol solution at room temperature were crystal form II.

**Table 26 Results of the competitive experiments for different water activities**

| Solvent system | Percentage of the organic solvent in water* | Value of water activity | Determination of crystal form (room temperature, 3 days) |
|---|---|---|---|
| Ethanol-water | 95 | 0.291 | Crystal form II |
| | 90 | 0.484 | Crystal form II |
| | 65 | 0.750 | Crystal form II |
| | 15 | 0.945 | Crystal form I+ Crystal form II |
| Water | / | 1 | Crystal form I |

| | | | |
|---|---|---|---|
| *The proportions of the mixed solvent are all expressed as a volume percentage. | | | |

The results of the competitive experiments of the crystal forms I and II indicated that: in purified water, the stability of the crystal form II is not as good as that of the crystal form I; in ethanol-water mixed solutions with lower water activity (0.291 - 0.750), the crystal form II could exist stably; in the 15% ethanol solution with water activity of 0.945, a mixed crystal of the crystal forms I and II was obtained.

### Example 2: Crystal forms of other acid addition salts of nepicastat

A free base of nepicastat was prepared according to the method described in paragraph [0190] of the specification in CN106983747A.

### 2.1 Approximate solubility test

In order to select a suitable method for salt form screening, an approximate solubility test was conducted on the compound. The test method was as follows: about 4 mg of the free base of nepicastat was weighed, an appropriate volume of a solvent was added gradually thereto and shaken to observe whether the free base was dissolved, and the volumes of the solvent were recorded, including the volume that enabled complete dissolution and the previous volume of the solvent that was not able to completely dissolve the free base. For poor solubility, the total amount of the solvent added was no more than 4 mL. The approximate solubility thereof was calculated.

According to the results of approximate solubility as shown in Table 27, the free base of nepicastat has higher solubility in dichloromethane: methanol (1:1, V: V), methanol, acetone, and ethanol.

**Table 27 Approximate solubility of the free base of nepicastat**

| Solvent | Solubility (mg/mL) |
|---|---|
| Dichloromethane: methanol (1:1) | 19.7~39.3 |
| Methanol | 6.9~10.3 |
| Acetone | 4.0~5.0 |
| Ethanol | 2.9~3.4 |
| 2-Methyltetrahydrofuran | <1.0 |
| Ethyl acetate | <1.0 |
| Methyl tert-butyl ether | <1.0 |
| N-heptane | <1.0 |
| Dichloromethane | <1.0 |
| Purified water | <1.0 |

### 2.2 Salt form preliminary screening of the free base of nepicastat in the ethanol system

The ethanol system was selected for salt-forming experiments, based on the results of approximate solubility. About 50 mg of the free base of nepicastat (i.e., API in Tables 28 and 29) was weighed and placed into each vial, and 1 mL of anhydrous ethanol was added thereto and stirred at 50°C to form an off-white suspension. Then, the counter ion was slowly added dropwise to the reaction solution at a molar ratio of 1:1.05 or 1:0.55. Finally, after magnetic stirring for 2 hours at 50°C, the reaction solution was cooled naturally down to room temperature and stirred overnight (approximately 20 hours). After stirring overnight, if the sample in the vial was a suspension with a large amount of solid, the solid was collected by centrifugation (12000 rpm, centrifuging for 10 minutes); or, if the sample in the vial was a clear solution or had a small amount of solid, the solvent was concentrated by nitrogen purging or natural evaporation, and the solid was precipitated and collected. The collected solid was subjected to vacuum drying under reduced pressure at 40°C for 4 hours, and the X-ray powder diffraction detection of the dried solid was conducted. Refer to Figure 24 and Table 28 for details. If the XRPD pattern of the dried solids was inconsistent with the XRPD pattern of the starting free base, these dried solids were subjected to further characterization, including DSC, TGA and the like.

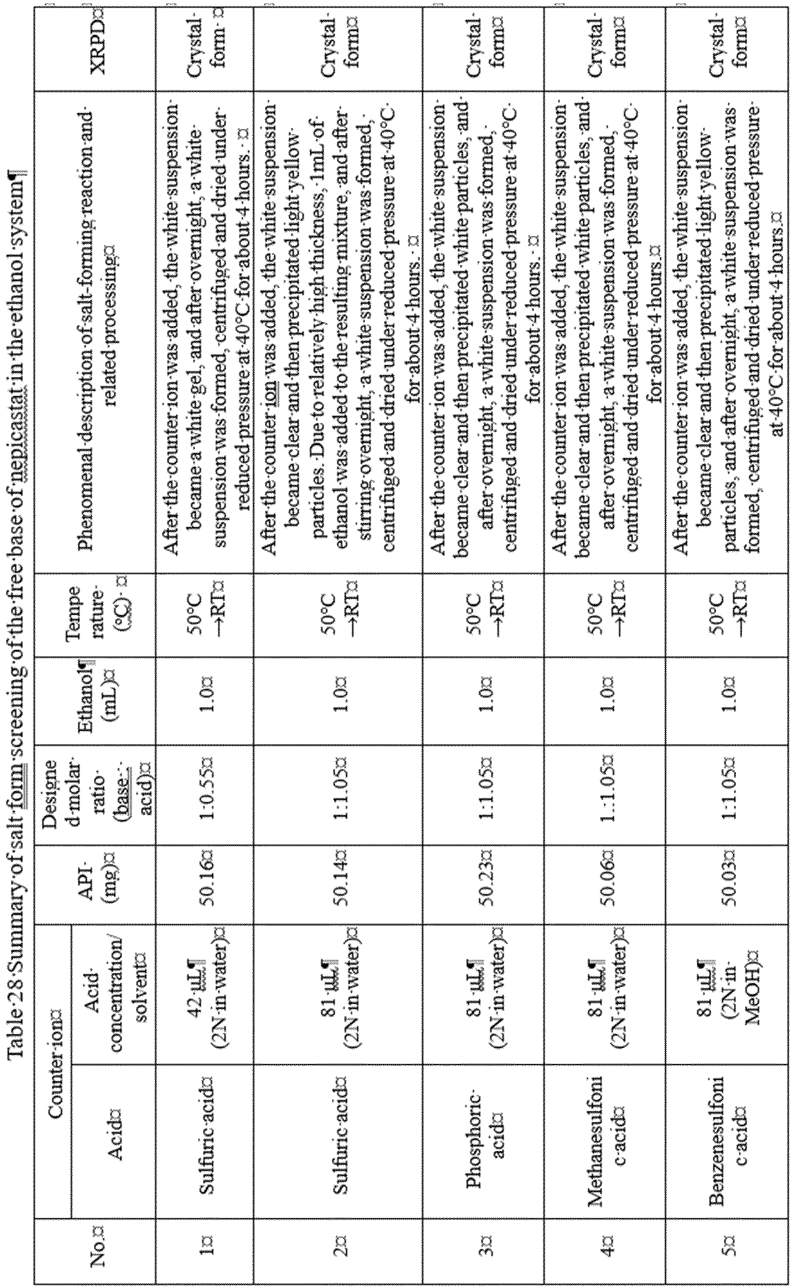

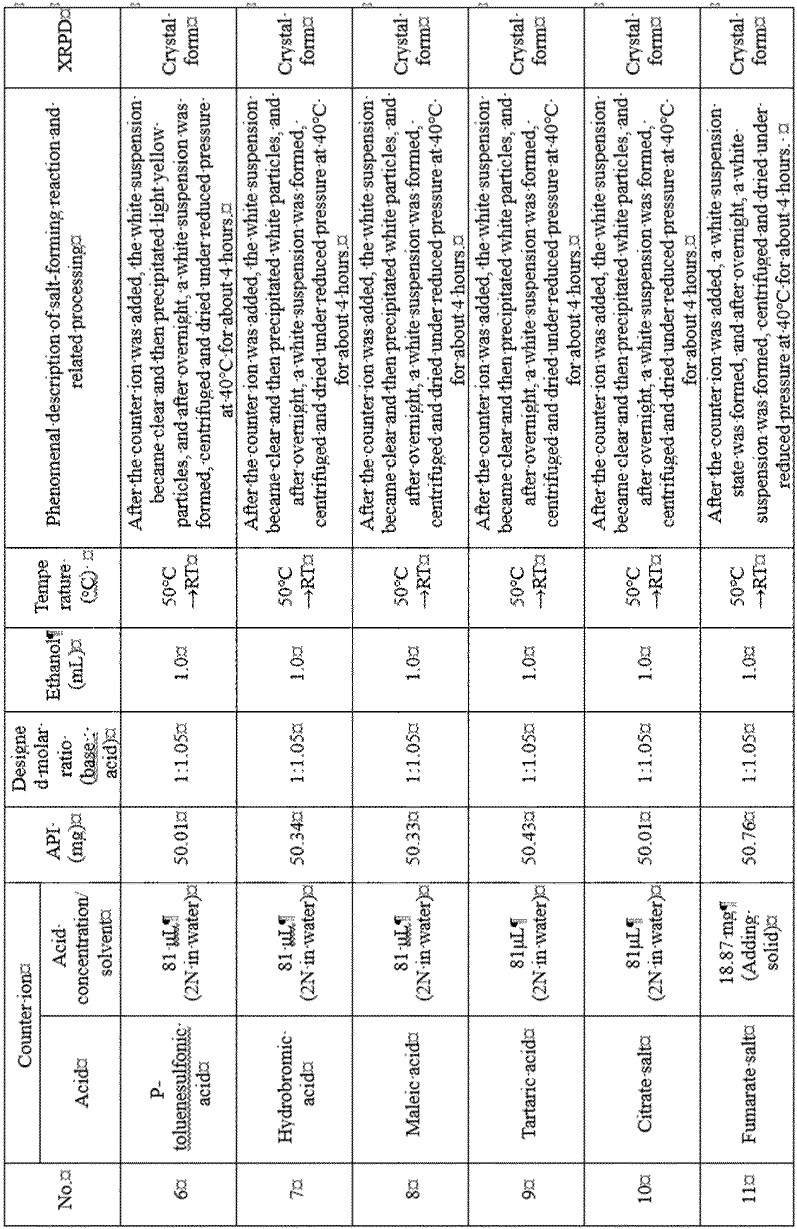

### 2.3 Salt form preliminary screening of the free base of nepicastat in the acetone system

About 50 mg of the free base of nepicastat was weighed and placed into each vial, and 1 mL of acetone solution was added thereto and stirred at 50°C to form a solution state. Then, the counter ion was slowly added dropwise to the reaction solution at a molar ratio of 1:1.05 or 1:0.55. Finally, after stirring for 2 hours at 50°C, the sample was cooled naturally down to room temperature and then stirred magnetically overnight (approximately 20 hours). After stirring overnight, if the sample in the vial was a suspension with a large amount of solid, the solid was collected by centrifugation (12000 rpm, centrifuging for 10 minutes); or, if the sample in the vial was a clear solution or had a small amount of solid, the solvent was concentrated by natural evaporation, then MTBE as the anti-solvent was added, and the solid was precipitated and collected. The collected solid was subjected to vacuum drying under reduced pressure at 40°C for 4 hours, and the X-ray powder diffraction detection of the dried solid was conducted. Refer to Figure 25 and Table 29 for details. If the XRPD pattern of the dried solids was inconsistent with the XRPD pattern of the starting free base, these dried solids were subjected to further characterization, including TGA, DSC and the like.

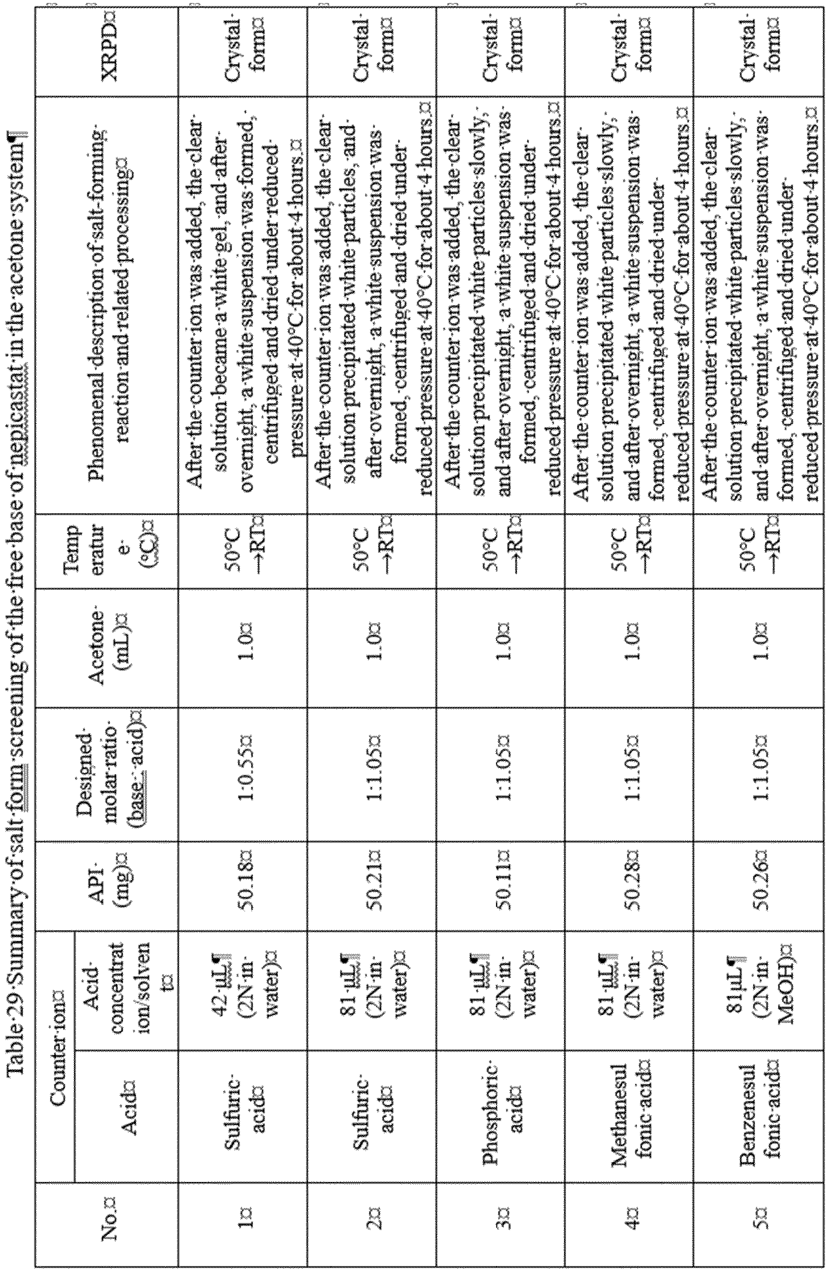

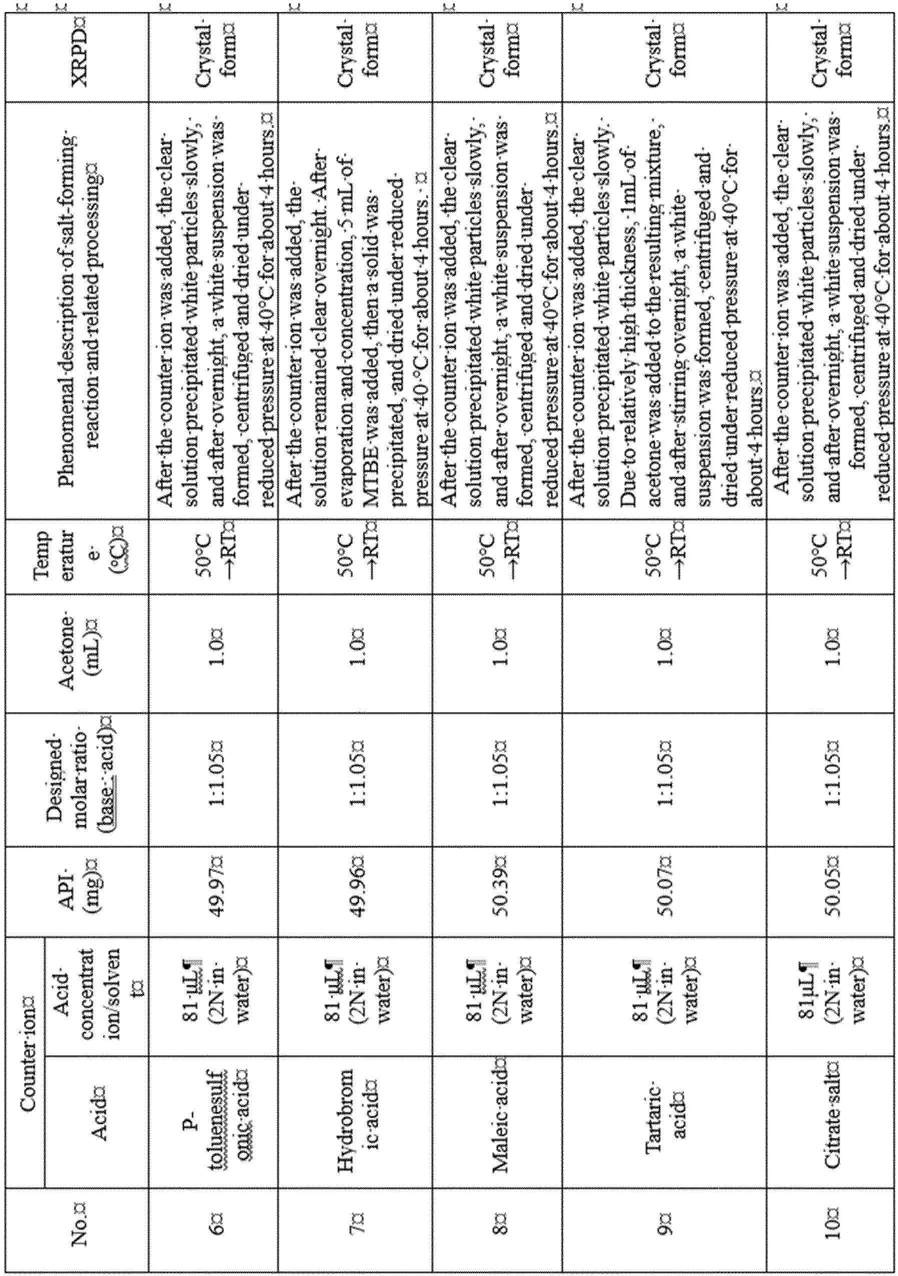

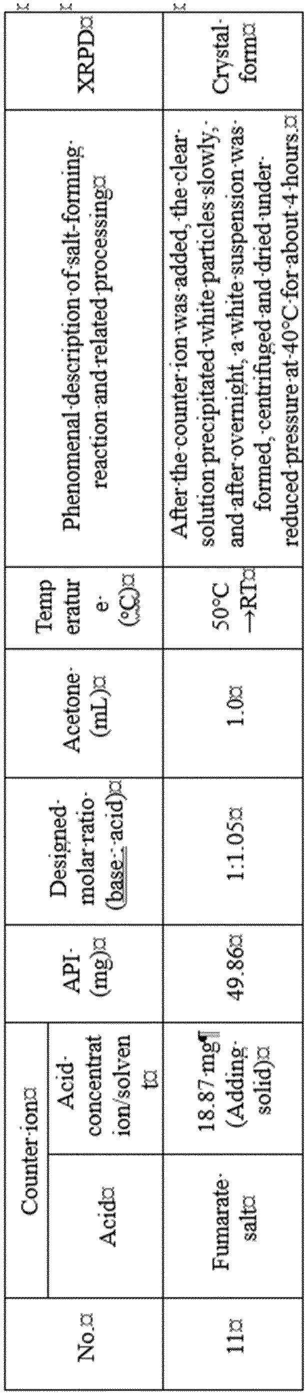

### 2.4 Characterization of salt forms

Sulfate salt (1:0.5), sulfate salt (1:1), phosphate salt, methanesulfonate salt, benzenesulfonate salt, p-toluenesulfonate salt, hydrobromide salt, maleate salt, tartrate salt, citrate salt and fumarate salt of nepicastat obtained through preliminary screening were characterized by XRPD, DSC, TGA and the like.

The XRPD pattern, DSC spectrum and TGA spectrum of the crystal form I of sulfate salt (1:0.5) of nepicastat are as shown in Figures 26, 27 and 28 respectively.

The XRPD pattern, DSC spectrum and TGA spectrum of the crystal form II of sulfate salt (1:0.5) of nepicastat are as shown in Figures 29, 30 and 31 respectively.

The XRPD pattern, DSC spectrum and TGA spectrum of the crystal form III of sulfate salt (1:1) of nepicastat are as shown in Figures 32, 33 and 34 respectively.

The XRPD pattern, DSC spectrum and TGA spectrum of the crystal form I of phosphate salt of nepicastat are as shown in Figures 35, 36 and 37 respectively.

The XRPD pattern, DSC spectrum and TGA spectrum of the crystal form I of methanesulfonate salt of nepicastat are as shown in Figures 38, 39 and 40 respectively.

The XRPD pattern, DSC spectrum and TGA spectrum of the crystal form I of benzenesulfonate salt of nepicastat are as shown in Figures 41, 42 and 43 respectively.

The XRPD pattern, DSC spectrum and TGA spectrum of the crystal form I of p-toluenesulfonate salt of nepicastat are as shown in Figures 44, 45 and 46 respectively.

The XRPD pattern, DSC spectrum and TGA spectrum of the crystal form II of p-toluenesulfonate salt of nepicastat are as shown in Figures 47, 48 and 49 respectively.

The XRPD pattern, DSC spectrum and TGA spectrum of the crystal form I of hydrobromide salt of nepicastat are as shown in Figures 50, 51 and 52 respectively.

The XRPD pattern, DSC spectrum and TGA spectrum of the crystal form II of hydrobromide salt of nepicastat are as shown in Figures 53, 54 and 55 respectively.

The XRPD pattern, DSC spectrum and TGA spectrum of the crystal form I of maleate salt of nepicastat are as shown in Figures 56, 57 and 58 respectively.

The XRPD pattern, DSC spectrum and TGA spectrum of the crystal form I of tartrate salt of nepicastat are as shown in Figures 59, 60 and 61 respectively.

The XRPD pattern, DSC spectrum and TGA spectrum of the crystal form II of tartrate salt of nepicastat are as shown in Figures 62, 63 and 64 respectively.

The XRPD pattern, DSC spectrum and TGA spectrum of the crystal form I of citrate salt of nepicastat are as shown in Figures 65, 66 and 67 respectively.

The XRPD pattern, DSC spectrum and TGA spectrum of the crystal form I of fumarate salt of nepicastat are as shown in Figures 68, 69 and 70 respectively.

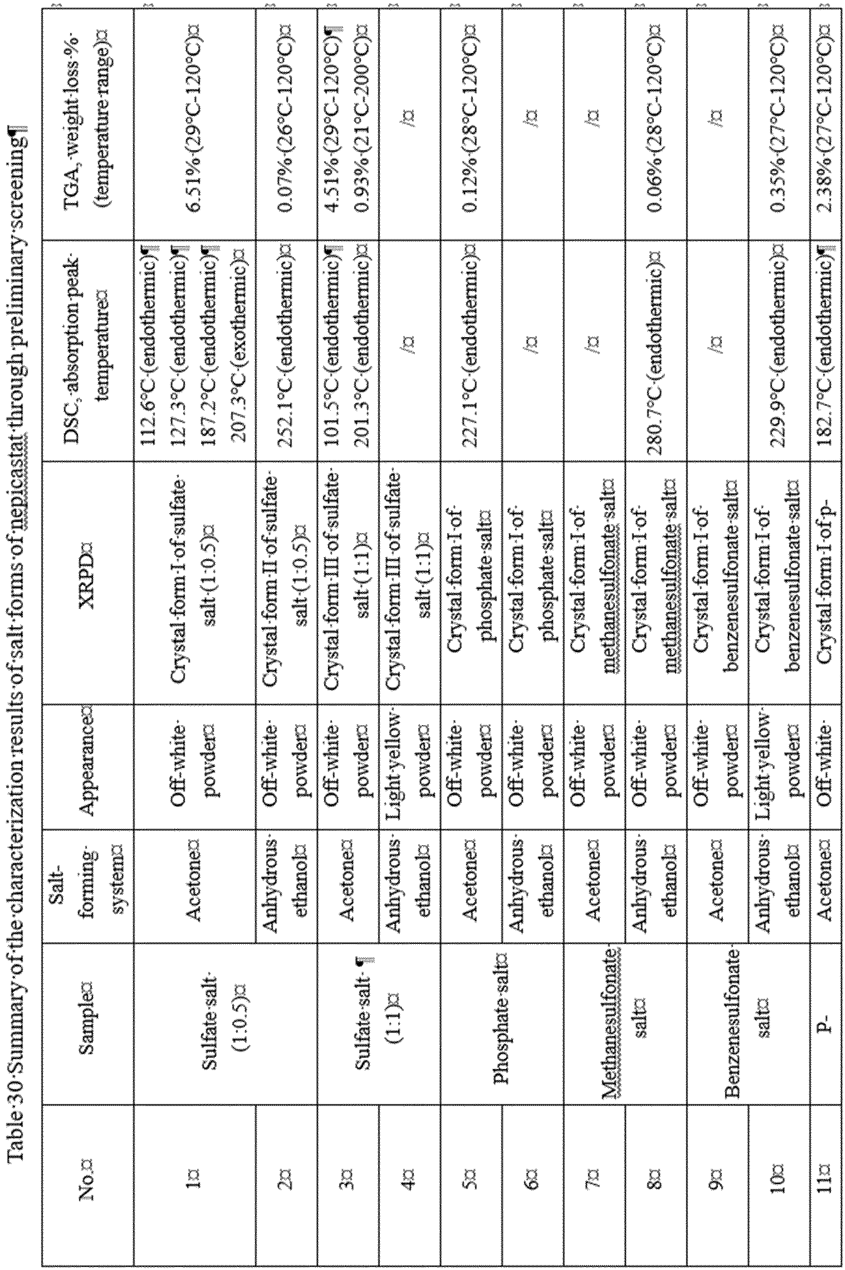

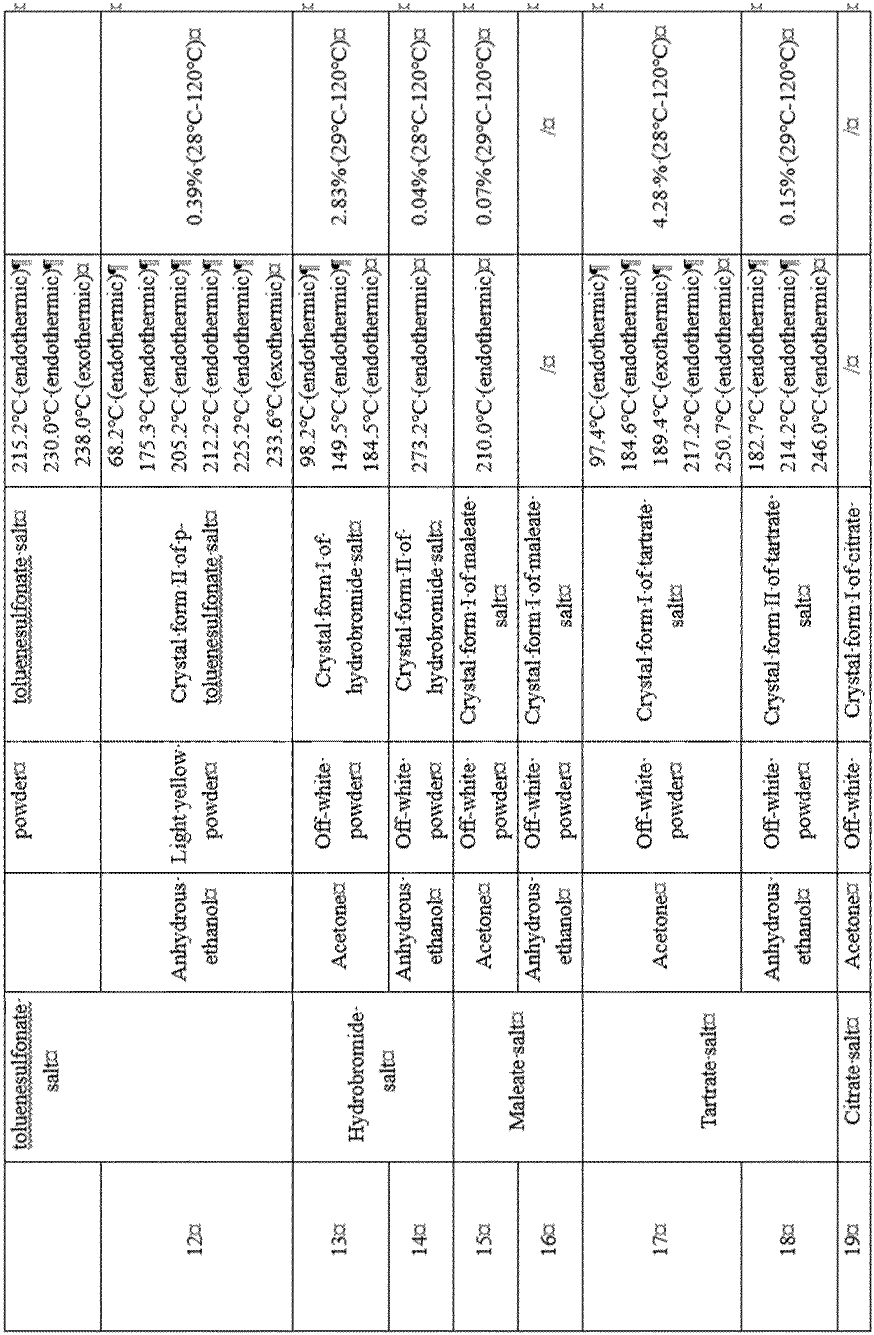

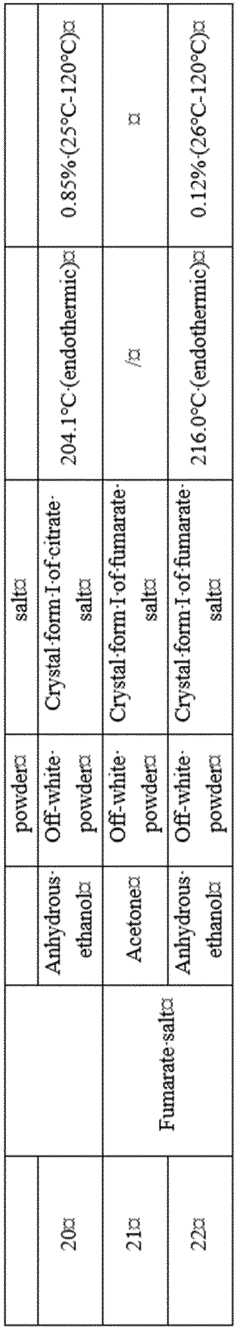

### 2.5 Approximate solubility tests on some of crystal forms of acid addition salts of nepicastat in water

About 4 mg of the compound shown in the following Table was weighed, an appropriate volume of purified water was added gradually thereto at room temperature and shaken by ultrasound to observe whether the compound was dissolved, and the volumes of the solvent were recorded, including the volume that enabled complete dissolution and the previous volume of the solvent that was not able to completely dissolve the compound. The results are shown in Table 31.

**Table 31 Results of the approximate solubility tests on some of crystal forms of acid addition salts of nepicastat in water (at room temperature)**

| No. | Sample | Approximate solubility (mg/mL)* |
|---|---|---|
| 1 | Crystal form II of sulfate salt (1:0.5) | <0.5 |
| 2 | Crystal form III of sulfate salt (1:1) | 0.5~1.0 |
| 3 | Crystal form I of phosphate salt | 1.0~2.0 |
| 4 | Crystal form I of methanesulfonate salt | 1.6~3.2 |
| 5 | Crystal form I of maleate salt | 1.8~3.6 |
| 6 | Crystal form I of citrate salt | <0.5 |
| 7 | Crystal form I of fumarate salt | <0.5 |

| | | |
|---|---|---|
| *: The approximate solubility of a crystal form of an acid addition salt of nepicastat in water is calculated by the salt. | | |

According to the results of the approximate solubility tests of the candidate salt forms in water, the solubility of maleate salt in purified water is slightly higher than that of other candidate salt forms, but still not as good as the solubility of hydrochloride salt of nepicastat in purified water (7.4 mg/mL).

### 2.6 Stability tests on some of crystal forms of acid addition salts of nepicastat

The crystal forms of acid addition salts of nepicastat in Table 31 were taken and subjected to the high-temperature test (60 ± 2°C) and accelerated test (40°C± 2°C/75% RH ± 5% RH) for a total of 4 weeks, and the results showed that:
the crystal form II of sulfate salt (1:0.5) was stable under accelerated conditions, and the impurities increased under high temperature conditions;
the crystal form III of sulfate salt (1:1) was stable under accelerated conditions, and the impurities increased under high temperature conditions;
the crystal form I of phosphate salt was stable under accelerated conditions, and the impurities slightly increased under high temperature conditions;
the crystal form I of methanesulfonate salt was stable under accelerated conditions and high temperature conditions;
the crystal form I of maleate salt was stable under accelerated conditions and high temperature conditions;
the crystal form I of citrate salt was stable under accelerated conditions, and the impurities increased under high temperature conditions;
the crystal form I of fumarate salt was stable under accelerated conditions, and the impurities increased under high temperature conditions.

### Example 3: Bioavailability test

The crystal forms I and II of hydrochloride salt of nepicastat and the crystal forms of other acid addition salts of nepicastat in Table 31 were taken, and administered intravenously and orally to mice respectively, wherein:
the formulation for intravenous (IV) administration was: 5% (v/v) DMSO+10% (v/v) Solutol HS 15+ 85% (v/v) physiological saline;
the formulation for oral (PO) administration was: 0.5% (v/v) MC4000 aqueous solution (MC: methylcellulose).

The specific administration was as follows:
Experiment: three mice were subjected to the test in parallel for each formulation;
Body weight: about 30 g
Administration route: IV; PO
Dose administered: IV - 15 mg/kg; PO - 30 mg/kg;
Before administration: fasting for 12 hours before administration, and allowing mice to drink water;
After administration: allowing mice to drink water, and feeding within 2 hours after administration;
Blood collecting site: the vein at a hind leg
Blood collecting times: IV---0.083, 0.25, 0.5, 1, 2, 4, 6, 8, 24 h;
   PO---0.25, 0.5, 1, 2, 4, 6, 8, 24 h.

Due to very close solubilities of the crystal forms I and II of hydrochloride salt of nepicastat in water, it was found in the experiment that both of them obtained clear solutions upon preparing the administration samples. Therefore, subsequent tests were only conducted on the crystal form I of hydrochloride salt of nepicastat.

The experimental results indicated that the oral bioavailability of the crystal form I of hydrochloride salt was greater than 70%. The bioavailability of each of other acid addition salts was 0.9-1.3 times that of the crystal form I of hydrochloride salt, and was good. The relative bioavailability of each of acid addition salts with respect to the crystal form I of hydrochloride salt is shown in Table 32. T_{1/2} and AUC_{0→t} of each acid addition salt are relatively close. The Tₘₐₓ of other acid addition salts is within the range of 2-4 hours, except for the Tₘₐₓ of the crystal form I of methanesulfonate salt and the crystal form I of fumarate salt less than 2 hours.

**Table 32 Relative bioavailability**

| Compound name | Dosage form | Dose/mpk | Relative bioavailability |
|---|---|---|---|
| Crystal form I of hydrochloride salt | Solution | 30 | 1.0 |
| Crystal form II of sulfate salt (1:0.5) | Suspension | 30 | 1.1 |
| Crystal form III of sulfate salt (1:1) | Suspension | 30 | 0.9 |
| Crystal form I of phosphate salt | Suspension | 30 | 1.3 |
| Crystal form I of methanesulfonate salt | Suspension | 30 | 1.3 |
| Crystal form I of maleate salt | Suspension | 30 | 1.3 |
| Crystal form I of citrate salt | Suspension | 30 | 1.2 |
| Crystal form I of fumarate salt | Suspension | 30 | 1.1 |

## Claims

1. A crystal form of an acid addition salt of nepicastat, wherein the acid addition salt is selected from the group consisting of hydrochloride salt, sulfate salt, phosphate salt, methanesulfonate salt, benzenesulfonate salt, p-toluenesulfonate salt, hydrobromide salt, maleate salt, tartrate salt, citrate salt, and fumarate salt, preferably hydrochloride salt.

2. The crystal form of the acid addition salt of nepicastat according to claim 1, wherein the crystal form is crystal form I of hydrochloride salt of nepicastat, crystal form II of hydrochloride salt of nepicastat, crystal form III of hydrochloride salt of nepicastat or crystal form IV of hydrochloride salt of nepicastat;
specifically,
the crystal form I of hydrochloride salt of nepicastat is a monohydrate, and the X-ray powder diffraction pattern thereof comprises characteristic peaks at diffraction angles (2θ) of 15.48±0.2°, 20.66±0.2°, 22.64±0.2°, 25.60±0.2°, 27.06±0.2°, 29.70±0.2°, 31.84±0.2° and 41.94±0.2°; and preferably, further comprises at least one characteristic peak at diffraction angles (2θ) of 17.04±0.2°, 23.82±0.2°, 25.08±0.2°, 34.34±0.2°, 36.78±0.2°, 43.88±0.2° and 44.12±0.2°; and more preferably, further comprises at least one characteristic peak at diffraction angles (2θ) of 5.12±0.2°, 12.98±0.2°, 27.68±0.2°, 28.20±0.2° and 39.94±0.2°; advantageously, the DSC spectrum of the crystal form I of hydrochloride salt of nepicastat shows 4 endothermic peaks at 130.8°C, 205.5°C, 251.8°C and 296.9°C and one exothermic peak at 210.9°C; the TGA spectrum of the crystal form I of hydrochloride salt of nepicastat shows a weight loss of 5.15% from room temperature to 120°C;
the crystal form II of hydrochloride salt of nepicastat is an anhydride, and the X-ray powder diffraction pattern thereof comprises characteristic peaks at diffraction angles (2θ) of 20.62±0.2°, 21.90±0.2°, 25.04±0.2°, 28.28±0.2°, 30.05±0.2° and 31.46±0.2°; and preferably, further comprises at least one characteristic peak at diffraction angles (2θ) of 10.88±0.2°, 13.60±0.2°, 14.84±0.2°, 17.96±0.2° and 27.32±0.2°; and more preferably, further comprises at least one characteristic peak at diffraction angles (2θ) of 7.12±0.2°, 16.74±0.2°, 18.57±0.2°, 23.01±0.2°, 24.64±0.2°, 34.54±0.2°, 40.17±0.2° and 45.56±0.2°; advantageously, the DSC spectrum of the crystal form II of hydrochloride salt of nepicastat shows one endothermic peak at 266.2°C;
the crystal form III of hydrochloride salt of nepicastat is **characterized by** the X-ray powder diffraction pattern comprising characteristic peaks at diffraction angles (2θ) of 13.58±0.2°, 20.22±0.2°, 22.32±0.2°, 24.54±0.2°, 26.16±0.2°, 30.14±0.2° and 31.26±0.2°; and preferably, further comprising at least one characteristic peak at diffraction angles (2θ) of 6.86±0.2°, 7.62±0.2°, 10.26±0.2°, 11.14±0.2°, 13.03±0.2° and 40.76±0.2°; advantageously, the DSC spectrum of the crystal form III of hydrochloride salt of nepicastat I shows one endothermic peak at 267.1°C;
the crystal form IV of hydrochloride salt of nepicastat is **characterized by** the X-ray powder diffraction pattern comprising characteristic peaks at diffraction angles (2θ) of 13.60±0.2°, 20.70±0.2°, 21.94±0.2°, 24.99±0.2° and 25.64±0.2°; and preferably, further comprising at least one characteristic peak at diffraction angles (2θ) of 15.56±0.2°, 16.78±0.2°, 22.68±0.2°, 26.18±0.2°, 27.32±0.2°, 28.23±0.2° and 31.44±0.2°; and more preferably, further comprising at least one characteristic peak at diffraction angles (2θ) of 10.95±0.2°, 13.06±0.2°, 14.84±0.2°, 17.93±0.2° and 29.95±0.2°, even more preferably, further comprising at least one characteristic peak at diffraction angles (2θ) of 5.23±0.2°, 7.12±0.2°, 23.80±0.2°, 34.52±0.2°, 33.16±0.2° and 38.48±0.2°.

3. The crystal form of the acid addition salt of nepicastat according to claim 1, wherein the crystal form is crystal form I of sulfate salt (1:0.5) of nepicastat, crystal form II of sulfate salt (1:0.5) of nepicastat or crystal form III of sulfate salt (1:1) of nepicastat;
specifically,
the X-ray powder diffraction pattern of the crystal form I of sulfate salt (1:0.5) of nepicastat comprises characteristic peaks at diffraction angles (2θ) of 9.90±0.2°, 14.78±0.2°, 16.72±0.2°, 19.82±0.2°, 22.08±0.2°, 22.40±0.2° and 24.86±0.2°; and preferably, further comprises at least one characteristic peak at diffraction angles (2θ) of 25.56±0.2°, 26.19±0.2°, 28.42±0.2° and 30.68±0.2°; and more preferably, further comprises at least one characteristic peak at diffraction angles (2θ) of 35.16±0.2°, 35.80±0.2° and 40.26±0.2°; advantageously, the DSC spectrum of the crystal form I of sulfate salt (1:0.5) of nepicastat shows 3 endothermic peaks at 112.6°C, 127.3°C and 187.2°C and one exothermic peak at 207.3°C;
the X-ray powder diffraction pattern of the crystal form II of sulfate salt (1:0.5) of nepicastat comprises characteristic peaks at diffraction angles (2θ) of 4.56±0.2°, 8.24±0.2°, 9.02±0.2°, 16.08±0.2°, 16.58±0.2°, 17.90±0.2°, 22.01±0.2° and 25.12±0.2°; and preferably, further comprises at least one characteristic peak at diffraction angles (2θ) of 19.48±0.2°, 23.68±0.2° and 27.14±0.2°; advantageously, the DSC spectrum of the crystal form II of sulfate salt (1:0.5) of nepicastat shows one endothermic peak at 252.1°C;
the X-ray powder diffraction pattern of the crystal form III of sulfate salt (1:1) of nepicastat comprises characteristic peaks at diffraction angles (2θ) of 9.92±0.2°, 16.82±0.2°, 19.84±0.2°, 22.10±0.2°, 22.44±0.2°, 25.18±0.2° and 28.42±0.2°; and preferably, further comprises at least one characteristic peak at diffraction angles (2θ) of 12.40±0.2°, 14.26±0.2°, 24.06±0.2°, 30.68±0.2°, 31.00±0.2° and 35.28±0.2°; advantageously, the DSC spectrum of the crystal form III of sulfate salt (1:1) of nepicastat shows 2 endothermic peaks at 101.5°C and 201.3°C.

4. The crystal form of the acid addition salt of nepicastat according to claim 1, wherein the crystal form is crystal form I of phosphate salt of nepicastat; specifically, the X-ray powder diffraction pattern thereof comprises characteristic peaks at diffraction angles (2θ) of 5.24±0.2°, 10.48±0.2°, 21.14±0.2°, 22.76±0.2°, 23.74±0.2°, 25.54±0.2°, 26.52±0.2° and 29.74±0.2°; and preferably, further comprises at least one characteristic peak at diffraction angles (2θ) of 12.50±0.2°, 16.92±0.2°, 21.92±0.2° and 27.34±0.2°; and more preferably, further comprises at least one characteristic peak at diffraction angles (2θ) of 13.58±0.2°, 15.48±0.2°, 19.40±0.2°, 20.68±0.2°, 34.66±0.2°, 37.62±0.2° and 39.90±0.2°; advantageously, the DSC spectrum of the crystal form I of phosphate salt of nepicastatshows one endothermic peak at 227.1°C.

5. The crystal form of the acid addition salt of nepicastat according to claim 1, wherein the crystal form is crystal form I of methanesulfonate salt of nepicastat; specifically, the X-ray powder diffraction pattern thereof comprises characteristic peaks at diffraction angles (2θ) of 17.52±0.2°, 19.74±0.2°, 20.38±0.2°, 20.10±0.2°, 25.00±0.2° and 27.36±0.2°; and preferably, further comprises at least one characteristic peak at diffraction angles (2θ) of 7.90±0.2°, 13.48±0.2°, 15.06±0.2°, 22.88±0.2° and 23.28±0.2°; and more preferably, further comprises at least one characteristic peak at diffraction angles (2θ) of 28.50±0.2°, 29.08±0.2°, 31.42±0.2°, 32.94±0.2° and 36.36±0.2°; advantageously, the DSC spectrum of the crystal form I of methanesulfonate salt of nepicastat shows one endothermic peak at 280.7°C.

6. The crystal form of the acid addition salt of nepicastat according to claim 1, wherein the crystal form is crystal form I of benzenesulfonate salt of nepicastat; specifically, the X-ray powder diffraction pattern thereof comprises characteristic peaks at diffraction angles (2θ) of 14.00±0.2°, 16.62±0.2°, 17.96±0.2°, 22.70±0.2°, 24.66±0.2° and 26.86±0.2°; and preferably, further comprises at least one characteristic peak at diffraction angles (2θ) of 7.38±0.2°, 8.06±0.2°, 11.84±0.2° and 20.42±0.2°; and more preferably, further comprises at least one characteristic peak at diffraction angles (2θ) of 19.34±0.2°, 21.62±0.2°, 28.50±0.2° and 30.42±0.2°; advantageously, the DSC spectrum of the crystal form I of benzenesulfonate salt of nepicastat shows one endothermic peak at 229.9°C.

7. The crystal form of the acid addition salt of nepicastat according to claim 1, wherein the crystal form is crystal form I of p-toluenesulfonate salt of nepicastat or crystal form II of p-toluenesulfonate salt of nepicastat;
specifically,
the X-ray powder diffraction pattern of the crystal form I of p-toluenesulfonate salt of nepicastat comprises characteristic peaks at diffraction angles (2θ) of 13.40±0.2°, 15.52±0.2°, 19.38±0.2°, 19.78±0.2°, 23.52±0.2° and 28.74±0.2°; advantageously, the DSC spectrum of the crystal form I of p-toluenesulfonate salt of nepicastat shows 3 endothermic peaks at 182.7°C, 215.2°C and 230.0°C and one exothermic peak at 238.0°C;
the X-ray powder diffraction pattern of the crystal form II of p-toluenesulfonate salt of nepicastat comprises characteristic peaks at diffraction angles (2θ) of 4.80±0.2°, 16.62±0.2°, 17.04±0.2°, 17.54±0.2°, 19.34±0.2° and 20.14±0.2°; and preferably, further comprises at least one characteristic peak at diffraction angles (2θ) of 9.44±0.2°, 15.46±0.2° and 21.46±0.2°; and more preferably, further comprises at least one characteristic peak at diffraction angles (2θ) of 24.78±0.2°, 25.10±0.2°, 26.34±0.2° and 29.20±0.2°; advantageously, the DSC spectrum of the crystal form II of p-toluenesulfonate salt of nepicastat shows 5 endothermic peaks at 68.2°C, 175.3°C, 205.2°C, 212.2°C and 225.2°C and one exothermic peak at 233.6°C.

8. The crystal form of the acid addition salt of nepicastat according to claim 1, wherein the crystal form is crystal form I of hydrobromide salt of nepicastat or crystal form II of hydrobromide salt of nepicastat;
specifically,
the X-ray powder diffraction pattern of the crystal form I of hydrobromide salt of nepicastat comprises characteristic peaks at diffraction angles (2θ) of 13.88±0.2°, 14.60±0.2°, 17.74±0.2°, 18.14±0.2°, 22.84±0.2° and 25.76±0.2°; and preferably, further comprises at least one characteristic peak at diffraction angles (2θ) of 20.34±0.2°, 24.14±0.2° and 26.52±0.2°; and more preferably, further comprises at least one characteristic peak at diffraction angles (2θ) of 15.64±0.2°, 16.12±0.2°, 27.14±0.2° and 28.18±0.2°; advantageously, the DSC spectrum of the crystal form I of hydrobromide salt of nepicastat shows 3 endothermic peaks at 98.2°C, 149.5°C and 184.5°C;
the X-ray powder diffraction pattern of the crystal form II of hydrobromide salt of nepicastat comprises characteristic peaks at diffraction angles (2θ) of 16.50±0.2°, 18.34±0.2°, 21.60±0.2°, 22.16±0.2°, 23.96±0.2°, 24.82±0.2°, 29.90±0.2° and 31.18±0.2°; and preferably, further comprises at least one characteristic peak at diffraction angles (2θ) of 20.60±0.2°, 26.02±0.2°, 26.42±0.2° and 27.30±0.2°; and more preferably, further comprises at least one characteristic peak at diffraction angles (2θ) of 7.34±0.2°, 14.74±0.2°, 28.20±0.2°, 34.98±0.2° and 37.16±0.2°; advantageously, the DSC spectrum of the crystal form II of hydrobromide salt of nepicastat shows one endothermic peak at 273.2°C.

9. The crystal form of the acid addition salt of nepicastat according to claim 1, wherein the crystal form is crystal form I of maleate salt of nepicastat; specifically, the X-ray powder diffraction pattern thereof comprises characteristic peaks at diffraction angles (2θ) of 12.81±0.2°, 16.76±0.2°, 23.96±0.2°, 24.58±0.2°, 25.02±0.2°, 25.94±0.2°, 26.34±0.2° and 28.38±0.2°; and preferably, further comprises at least one characteristic peak at diffraction angles (2θ) of 14.98±0.2°, 18.64±0.2°, 19.36±0.2°, 20.56±0.2° and 21.92±0.2°; and more preferably, further comprises at least one characteristic peak at diffraction angles (2θ) of 16.32±0.2°, 27.72±0.2°, 31.50±0.2°, 36.36±0.2° and 39.46±0.2°; advantageously, the DSC spectrum of the crystal form I of maleate salt of nepicastat shows one endothermic peak at 210.0°C.

10. The crystal form of the acid addition salt of nepicastat according to claim 1, wherein the crystal form is crystal form I of tartrate salt of nepicastat or crystal form II of tartrate salt of nepicastat;
specifically,
the X-ray powder diffraction pattern of the crystal form I of tartrate salt of nepicastat comprises characteristic peaks at diffraction angles (2θ) of 14.28±0.2°, 19.08±0.2°, 23.66±0.2°, 24.58±0.2°, 26.34±0.2°, 26.98±0.2°, 28.58±0.2° and 31.34±0.2°; and preferably, further comprises at least one characteristic peak at diffraction angles (2θ) of 14.28±0.2°, 15.92±0.2°, 17.84±0.2°, 21.06±0.2° and 28.00±0.2°; and more preferably, further comprises characteristic peaks at diffraction angles (2θ) of 33.44±0.2°, 37.34±0.2°, 40.34±0.2°, 42.26±0.2° and 46.82±0.2°; advantageously, the DSC spectrum of crystal form I of tartrate salt of nepicastat shows 4 endothermic peaks at 97.4°C, 184.6°C, 217.2°C and 250.7°C and one exothermic peak at 189.4°C;
the X-ray powder diffraction pattern of the crystal form II of tartrate salt of nepicastat comprises characteristic peaks at diffraction angles (2θ) of 17.72±0.2°, 19.22±0.2°, 21.30±0.2°, 23.82±0.2°, 25.00±0.2°, 25.96±0.2°, 26.62±0.2° and 28.48±0.2°; and preferably, further comprises at least one characteristic peak at diffraction angles (2θ) of 14.22±0.2°, 19.72±0.2°, 21.84±0.2° and 22.42±0.2°; and more preferably, further comprises characteristic peaks at diffraction angles (2θ) of 13.00±0.2°, 15.20±0.2°, 31.50±0.2°, 33.60±0.2°, 36.08±0.2° and 37.42±0.2°; advantageously, the DSC spectrum of the crystal form II of tartrate salt of nepicastat shows 3 endothermic peaks at 182.7°C, 214.2°C and 246.0°C.

11. The crystal form of the acid addition salt of nepicastat according to claim 1, wherein the crystal form is crystal form I of citrate salt of nepicastat; specifically, the X-ray powder diffraction pattern thereof comprises characteristic peaks at diffraction angles (2θ) of 12.98±0.2°, 14.30±0.2°, 16.36±0.2°, 17.64±0.2°, 19.38±0.2°, 22.68±0.2°, 25.10±0.2° and 26.34±0.2°; and preferably, further comprises at least one characteristic peak at diffraction angles (2θ) of 22.28±0.2°, 30.26±0.2°, 40.26±0.2° and 40.86±0.2°; advantageously, the DSC spectrum of the crystal form I of citrate salt of nepicastat shows one endothermic peak at 204.1°C.

12. The crystal form of the acid addition salt of nepicastat according to claim 1, wherein the crystal form is crystal form I of fumarate salt of nepicastat; specifically, the X-ray powder diffraction pattern thereof comprises characteristic peaks at diffraction angles (2θ) of 13.58±0.2°, 14.12±0.2°, 15.56±0.2°, 17.18±0.2°, 21.86±0.2°, 23.22±0.2°, 23.98±0.2° and 26.40±0.2°; and preferably, further comprises at least one characteristic peak at diffraction angles (2θ) of 16.20±0.2°, 24.66±0.2°, 25.00±0.2°, 27.92±0.2° and 28.60±0.2°; and more preferably, further comprises characteristic peaks at diffraction angles (2θ) of 10.78±0.2°, 18.22±0.2°, 19.96±0.2°, 30.32±0.2° and 33.06±0.2°; advantageously, the DSC spectrum of the crystal form I of fumarate salt of nepicastat shows one endothermic peak at 216.0°C.

13. A method for preparing the crystal form of hydrochloride salt of nepicasta as defined in claim 2, comprising:
1) suspension-trituration method
mixing hydrochloride salt of nepicastat and an organic solvent, and subjecting the resulting suspension to solid-liquid separation to collect a solid, wherein the organic solvent is one or more selected from C₁-C₄ alcohol, C₄-C₆ ether and C₂-C₆ nitrile;
specifically, mixing hydrochloride salt of nepicastat and an organic solvent under stirring at a temperature ranging from room temperature to 50°C, preferably at 40°C, for 2-4 days, preferably for 3 days, and subjecting the resulting suspension to solid-liquid separation to collect a solid, optionally further drying the solid, wherein the organic solvent is one or more selected from ethanol, methyl tert-butyl ether, tetrahydrofuran and acetonitrile; advantageously, the ratio (W/V) of hydrochloride salt of nepicastat to the organic solvent is 50:1;
2) solvent evaporation method
dissolving hydrochloride salt of nepicastat in a good solvent, evaporating the solvent naturally, and then collecting a solid, wherein the good solvent is one or more of C₁-C₄ alcohol and C₄-C₆ ether or a mixed solvent with water thereof, preferably one or more of methanol, 50% isopropanol and 50% tetrahydrofuran; the ratio (W/V) of hydrochloride salt of nepicastat to the good solvent is 50:1;
3) anti-solvent method
dissolving hydrochloride salt of nepicastat in a good solvent, adding an anti-solvent, and then collecting a solid, wherein the good solvent is one or more of C₁-C₄ alcohol and C₄-C₆ ether or a mixed solvent with water thereof, preferably one or more of methanol, 50% isopropanol and 50% tetrahydrofuran; the anti-solvent is one or more selected from C₃-C₆ ester, C₄-C₆ ether, and C₃-C₆ ketone, preferably one or more of ethyl acetate, 2-methyltetrahydrofuran, methyl tert-butyl ether, 1,4-dioxane and 2-butanone; advantageously, the ratio (W/V) of hydrochloride salt of nepicastat to the good solvent is 50:0.5-2, such as 50:1.5, 50:2, or 50:0.5; and the volume ratio of the good solvent to the anti-solvent is 1:1 to 1:10, such as 1.5:4, 1.5:2, or 1:8;
4) mixing hydrochloride salt of nepicastat, C₁-C₄ alcohol and water, heating the resulting suspension to 45-55°C, stirring until dissolution, cooling down to 30-40°C, performing concentration to remove some solvent, cooling the residue down to 0-10°C, and collecting a solid after solid-liquid separation;
specifically, mixing hydrochloride salt of nepicastat, C₁-C₄ alcohol and water, heating the resulting suspension to 45-55°C, stirring until dissolution, cooling down to 30-40°C, stirring for 1-3 hours, performing concentration to remove some solvent, cooling the residue down to 0-10°C, collecting a solid after solid-liquid separation, and optionally further drying the solid, wherein the C₁-C₄ alcohol is ethanol; and advantageously, the ratio (W/W) of hydrochloride salt of nepicastat to the C₁-C₄ alcohol is 10:1 to 15:1, such as 12:1; the volume ratio of the C₁-C₄ alcohol to water is 10:1 to 20:1, such as 16:1;
5) stirring (S)-N-[3-(5,7-difluoro-1,2,3,4-tetrahydronaphthalen-2-yl)-2-thioxo-2,3-dihydro-1H-imidazol-4-ylmethyl]formamide, C₁-C₄ alcohol and concentrated hydrochloric acid under reflux, cooling down to precipitate a solid, collecting the solid after solid-liquid separation, adding C₁-C₄ alcohol and water, heating until dissolution, performing concentration to remove some solvent, cooling the residue down to 0-10°C, and collecting a solid after solid-liquid separation;
specifically, stirring (S)-N-[3-(5,7-difluoro-1,2,3,4-tetrahydronaphthalen-2-yl)-2-thioxo-2,3-dihydro-1H-imidazol-4-ylmethyl]formamide, C₁-C₄ alcohol and 36% concentrated hydrochloric acid under reflux for 5 hours, cooling down to 20-30°C, precipitating a solid, collecting the solid after solid-liquid separation, adding C₁-C₄ alcohol and water, heating to 65-70°C, stirring until dissolution, performing concentration to remove some solvent, cooling the residue down to 0-10°C, collecting a solid after solid-liquid separation, and optionally further drying the solid, wherein the C₁-C₄ alcohol is isopropanol or methanol.

14. A method for preparing the crystal form of the acid addition salt of nepicasta as defined in any one of claims 1 and 3-12, comprising:
1) mixing nepicastat and C₁-C₄ alcohol, preferably ethanol, under stirring, then adding an acid: if precipitating a large amount of solid, collecting the solid by centrifugation, or, if obtaining a sample as a clear solution or precipitating a small amount of solid, concentrating the solvent by nitrogen purging or natural evaporation, precipitating and collecting the solid; wherein the acid is selected from the group consisting of sulfuric acid, phosphoric acid, methanesulfonic acid, benzenesulfonic acid, p-toluenesulfonic acid, hydrobromic acid, maleic acid, tartaric acid, citric acid, and fumaric acid;
specifically, mixing nepicastat and C₁-C₄ alcohol under stirring at 40-60°C, preferably at 50 °C to prepare a suspension, then adding an acid, cooling down to room temperature and stirring overnight, wherein the ratio (W/V) of nepicastat to the C₁-C₄ alcohol is 50:1, and the molar ratio of nepicastat to the acid is 1:1.05 or 1:0.55;
or, 2) mixing nepicastat and C₃-C₆ ketone, preferably acetone, under stirring, then adding an acid: if precipitating a large amount of solid, collecting the solid by centrifugation, or, if obtaining a sample as a clear solution or precipitating a small amount of solid, concentrating the solvent by nitrogen purging or natural evaporation, then adding an anti-solvent, precipitating and collecting the solid; wherein the acid is selected from the group consisting of sulfuric acid, phosphoric acid, methanesulfonic acid, benzenesulfonic acid, p-toluenesulfonic acid, hydrobromic acid, maleic acid, tartaric acid, citric acid, and fumaric acid, and the anti-solvent is C₄-C₆ ether, preferably methyl tert-butyl ether;
specifically, mixing nepicastat and C₃-C₆ ketone under stirring at 40-60°C, preferably at 50 °C to prepare a solution, then adding an acid, cooling down to room temperature and stirring overnight, wherein the ratio (W/V) of nepicastat to the C₃-C₆ ketone is 50:1, the molar ratio of nepicastat to the acid is 1:1.05 or 1:0.55, and the volume ratio of the C₃-C₆ ketone to the anti-solvent is 1:1 to 1:10, such as 1:5.

15. A pharmaceutical composition, comprising the crystal form of the acid addition salt of nepicasta as defined in any one of claims 1-12 and an auxiliary material, wherein the auxiliary material is preferably a pharmaceutically acceptable carrier, diluent or excipient.

16. Use of the crystal form of the acid addition salt of nepicasta as defined in any one of claims 1-12 or the pharmaceutical composition as defined in claim 15 in the preparation of a medicament for treating autoimmune disease, post-traumatic stress disorder, congestive heart failure, hypertension, cancer or sepsis, wherein, preferably, the cancer is selected from the group consisting of colon cancer, breast cancer, liver cancer, melanoma, lung cancer, prostate cancer, ovarian cancer, pancreatic cancer, cervical cancer, renal cell cancer, bladder cancer cancer, and gastric cancer, and preferably, the autoimmune disease is selected from the group consisting of autoimmune colitis, ophthalmoneuromyelitis, rheumatoid arthritis, scleroderma, psoriasis and uveitis, and further, the autoimmune colitis is Crohn's disease or ulcerative colitis.
